# EUROPEAN PATENT SPECIFICATION

(11) **EP 2 434 015 B1**
(45) Date of publication and mention of the grant of the patent: **20.11.2013**
(21) Application number: 11195654.6
(22) Date of filing: 05.09.2005
(51) Int. Cl.: C12N 15/09, C12N 1/20, C12N 15/67, C12N 15/77

(54) **DNA fragment having promoter function**
DNS-Fragment mit Promotorfunktion
FRAGMENT D'ADN AYANT UNE FONCTION PROMOTRICE

(30) Priority: 09.09.2004 JP 2004263077
(43) Date of publication of application: 28.03.2012
(62) Divisional of application: 05776785.7
(73) Proprietor: RESEARCH INSTITUTE OF INNOVATIVE TECHNOLOGY FOR THE EARTH, Soraku-gun, Kyoto 619-0292 (JP)
(72) Inventor: Yukawa, Hideaki, Kyoto, 6190292 (JP); Inui, Masayuki, Kyoto, 6190292 (JP)
(74) Representative: Kalhammer, Georg

(56) References cited:
- EP-A- 0 285 152
- EP-A- 0 629 699
- DATABASE EMBL [Online] 14 November 2005 (2005-11-14), Han S. O. et al.: "Corynebacterium glutamicum glyceraldehyde 3-phosphate dehydrogenase A (gapA), phosphoglycerate kinase (pgk), triosephosphate isomerase (tpi), and phosphoenolpyruvate carboxylase (ppc) genes, complete cds.", XP002668254, Database accession no. DQ248873
- INUI M ET AL: "Metabolic engineering of corynebacterium glutamicum for fuel ethanol production under oxygen-deprivation conditions", JOURNAL OF MOLECULAR MICROBIOLOGY AND BIOTECHNOLOGY, vol. 8, no. 4, 2005, pages 243-254, XP008082382, ISSN: 1464-1801

### SEQUENCE LISTING

<110> Research Institute of Innovative Technology for the Earth
<120> DNA fragment having promoter function
<130> C01F1814
<160> 595
<170> PatentIn version 3.1
<210> 1
   <211> 575
   <212> DNA
   <213> Corynebacterium glutamicum R
<400> 1
   gtctcctagc gtgacatgtt tcttaagtac tttcaatttc ttttcgacgt gatttgaagt      60
   cactttttct aaactgtgac ccaccaccca accatcaacg ggtgtaacca cacgtcacca     120
   cgttgcattt tgcaaaaccc caggtaaaaa caggttcccc aaaatatgcg ggggtaaaat      180
   gcgatctccc ctcttccgtc aacttgacta tgaggttgca cacctttacg ccacttcccc     240
   acccgacgca gacaacagga acccaaaacg acaccatgaa tttaattccc cggaacttct     300
   tgacagaccg agcagtctag ggtttggttg aaaacgcaat cggttcactt ttaatcctct     360
   ccctggagcc ccggatgatg aggaacgcca aagctttctg aatggaaatt ttaagcgtta     420
   agtgggacga cctcgattac aaaaaggcga ggaaaccccc ggggcagctt tctgccaccc     480
   ggtgatttcg cgaaccttga aacatcgtca gaagattgcc gtgcgtccta gccgggatcc     540
   gcacgttcgg ctcaagcaga aagtctttaa ctcac                                575
<210> 2
   <211> 378
   <212> DNA
   <213> Corynebacterium glutamicum R
<400> 2
   ggccggcccc tgtccttctt aagcttgtct ctggtttcca agcataggca taagcgcagt      60
   tcagtagggg aaatgaccaa gaaacaagtt ttattcacac actgggggtg attccagtca     120
   ctaagtttag ctaaggtgtc ctgagttgct ttttgggtag cttaagtagc cttgacctgc     180
   tgttatgttt tttgcggtct ggtataaatt gtgccgattt aaggattttg tgggggtgga     240
   ttgaaattag ttggcccgat cccactactt ttcgcctgga gtgcttgtag gttgatagaa     300
   agtaaactaa agtaaacatc aggttaacag ccgggggttc aagtattaac tccctcggaa    360
   acagaaagga acacgaca                                                   378
<210> 3
   <211> 151
   <212> DNA
   <213> Corynebacterium glutamicum R
<400> 3
   aatcaccaac cctagtagcc ggtgcgccga tttgataaaa aactaagcgt ggcctgcggg     60
   aatcggtact tttaggatag ggcaacctaa aataaataag cttaggctaa gagtcggtga    120
   caatttatca agcagtgcta taataggggt c                                   151
<210> 4
   <211> 119
   <212> DNA
   <213> Corynebacterium glutamicum R
<400> 4
   gggaaagccc tcctcttggg aatcattttc aatagagtca acgcaagtgt acacttctta     60
   atagaaatcg ttatcaataa ggtcaagttt ttttgacctt cgctttttag gagcacccc     119
<210> 5
   <211> 283
   <212> DNA
   <213> Corynebacterium glutamicum R
<400> 5
   gttttaacct ccgcttctaa caaacggcta acccttttgg gttagccgtt tgttgaggtt    60
   aaggggtttt aaaagagggc ttttcgacga gtgctgatgg gcattcttat ttcccacaat    120
   ggcaaggggc ttcgtcgaaa agcgcgcttc gtgcccctga aaaatagttt tcccgcgcgg    180
   gaggcgcctg atcaaaccgc gcggttattt ctacctaatc ctgttcagtg ccatattccc    240
   gctcaacata cttgaaagcc tctaaagcct gtttaaatcc ttc                     283
<210> 6
   <211> 913
   <212> DNA
   <213> Corynebacterium glutamicum R
<400> 6
   tttctttttc ttcataatca aattggccgc tttccactca tgtttttgag cgggaggcgg 60
   ctttttgatg tccagggttg tttctggggt tttatttgag gttttgggtt cgaaacgggc 120
   tgtgggagtg catttcgatg cctcgaaagg gggatgcgag ggcggggttc ttgcgcaacc 180
   aaaccacagt gcccacggaa ctcgaaaatg tgtttcgtgg gcactgtggt ttgttttatc 240
   gccctccata ccggccacag atgcccaagt tttggatttg aggggttcgt gggcacgcct 300
   gtctggtgcc catcgaagcg gggtaagttt cgagctcgcg cctaaaaact ttgccctctc 360
   agaatcgctt ctaagcgccg ttcatggctg tgtccttaca agcgcacatt cagaaaattg 420
   aggcccttaa aacgccatac agcacctctc tcgaaatgcg caaccacggc aaaacaagca 480
   atcgacaata aaaaagtaaa acttatttag aatcccacta aaattcccaa aaccccgcaa 540
   caaccagcaa atttacataa accgcatgac tgattaaata ggtgttaaat attaaaaact 600
   agcgagccct gaattaatct gttaaggtcc tacatggact tcagttagtt gaggctgaag 660
   tttttgacat gtatgtctcg tccgagttcg ctgccatcca ggcggtgcac cgaggtggat 720
   tcatggtgaa tccttaatcg agtgaggccc cagaccaggg aagcctgccg cattaagtga 780
   gttcctgatg gcttcgggtg gaggatgtag aactttttgt acatggtctg tggcacgcat 840
   tgtttcgttt gccctgccat gttggtgttt taagtgaagg ccgccttttg cggaatgggt 900
   ataggaggca ttc 913
<210> 7
   <211> 158
   <212> DNA
   <213> Corynebacterium glutamicum R
<400> 7
   aacagttcat ttttctaaag gcagtgactc cgatgtgggt cgctgccttt agtcatttta 60
   agtcactcag cccaggcaat cctatctttg gaaagactta ccgacataga ctgacctgca 120
   aagttgttct agaatagcca tatcgttgac atagagat 158
<210> 8
   <211> 91
   <212> DNA
   <213> Corynebacterium glutamicum R
<400> 8
   gtccgcttca gttgtggtgg ctccgaatct gatgaacaat gatcattcct agttcattta 60
   catctttatc aaagagagcc accacctact a 91
<210> 9
   <211> 678
   <212> DNA
   <213> Corynebacterium glutamicum R
<400> 9
   tgatttttgg aaaaagtatc gcattagcga tgtctaaatc ttgactgaaa ccatcatatt     60
   cgctgtggat aagctgtgta atcagctgat attgcgctgt gttcctgtga attagctgat    120
   ttagtacttt tcgggggtgc tcattcttac caaagtgtca agttgtgggt agggtcactt    180
   gaataataat tgcaccgcac aggtgataca tgcttacctc ctcaagtagc ccgaggttaa    240
   gtctatttta ggtgaacaaa tttcagtttc aggtagaaaa ctttcgacct gcttcagagt    300
   ttctattagg aaatctgaca ccacttgatt aaataaccta cccccgaatt gggggagggg    360
   ttattttttg ctgtgaacgt agttttggtg catatgacct gcgtttataa agaaatgtaa    420
   acgtgatcag atcgatataa aagaaacagt ttgtactcag gtttgaagca ttttctccga    480
   ttcgcctggc aagaatctca attgtcgctt acagtttttc tcaacgacag gctgctaagc    540
   tgctagttcg gtggcctagt gagtggcgtt tacttgaatg aaaagtaatc ccatgtcgtg    600
   atcagccaat ttgggttgtt tccatagcaa tccaaaggtt tcgtctttcg atacctattc    660
   aaggagaccc tcgcctct                                                  678
<210> 10
   <211> 75
   <212> DNA
   <213> Corynebacterium glutamicum R
<400> 10
   atccgacacg gccctttttt agacaacaca acaaaagggc caccgggaaa ctttttagaa      60
   aggtgtgttt caccc                                                      75
<210> 11
   <211> 295
   <212> DNA
   <213> Corynebacterium glutamicum R
<400> 11
   gagcacgcca cacaaaagat cccggcatct gaattgacgt cagataccgg gatttttcat 60
   gcctgctaca tgcccactac aaagccccag aaagccccag aaagccccca ccccacatac 120
   ccccactctc aagtagacgt cgagattttt catttcaccg caaacatagt tctagatcag 180
   accatttaaa aaatcacacc ctcacctgga attttgcaga aataggacaa gaatcaaaat 240
   agtgggagat ccccatgttt cgcgaagtcc cattgttggg gttaggctta taccc 295
<210> 12
   <211> 242
   <212> DNA
   <213> Corynebacterium glutamicum R
<400> 12
   ggttacgcgg aaaggctttc cgtcaagccc gccctatggc tgtgataagt actaagataa 60
   cagctctgac ctgcgcccgc atgtttttca acggggtttt ccgtatttaa ttaggcaaca 120
   tatttgtgtc gtaattcact ttatgcaggt aaacgaattt gtgcttatat caacattcgt 180
   aattcggcaa aattaattaa actgaaaaag gggattaatt gcccccactt gaggagaaat 240
   tg 242
<210> 13
   <211> 278
   <212> DNA
   <213> Corynebacterium glutamicum R
<400> 13
   gatctacagt gtacgtaagc gtgtattttt gcactttgga ggaaataatg aacccccatt 60
   gatgcaggtc aatgggggtt aattgtgccc tggagaggaa tcgaacctcc gacacccgct 120
   ttaggagagc ggtgctctat ccactgagct accaaggcgt accgaactag attacaacac 180
   gcggggctgg gaagaaaaag agggggttgt ttttgcttcg ataagctgtt tccacaggta 240
   aggtgggagt taatacttcc cgaccttaag gagtggaa 278
<210> 14
   <211> 899
   <212> DNA
   <213> Corynebacterium glutamicum R
<400> 14
   cagcgccgta gtacgtgata attctgacgc gtcagacggt gaaacaatgg aagaagaact 60
   tggggcgcct gcgccgagaa aaacgcgagc tgaaatgagc aaacgggatt ctcaagactg 120
   cttcggcttt tttcgcagcg gaactcattc gcgcccgaga tccgtggacg tcggtcggag 180
   aagtcgaact ggccaccttg cggtgggtgc attggtggaa caccaagcgg cttcatgaag 240
   cattggacta cgccatccta caagtggtag aaaccaagtg ctatctcacc cagcccatca 300
   acacaggggc gtaaaagaag cggaactaaa cccaggacac ttcaggttca ggccgctgac 360
   ttggtagctt atggtgcgta gcaatggcat ttgcaggcgt atcctgagaa atggtcacaa 420
   agaaattcgc cagtaaagga tgcgattcgc gcatccttag gactaaaacc acatggatac 480
   ctgagtccga caatggcgtt atttgggtgg attaacgcaa aagcccctga ccattggcca 540
   ggggcttgtg agatgcgaga ccccgtggac gactaagaaa ccgtataggg cctcgcagta 600
   cttacggtaa caataaaggg tgttgttgca aagttggggc agtaggaaga gcgacgtgaa 660
   ataatcacag ccaggggtat cttctccggt cgtcattttc cccatgacat cattctgtgg 720
   gcagtgcggt ggtactgccc caactttgca acagcacccc cggtttcaac ctcgaagaag 780
   tccgagttga tgcagcatct gtgctcgatg atgggggaga gctgtgggaa aaatatcaac 840
   gcacacctaa ttcagcgacg tttgtatctt ttagtggcga gccgcaaggt caccttcat 899
<210> 15
   <211> 253
   <212> DNA
   <213> Corynebacterium glutamicum R
<400> 15
   gtgcgaagaa ctcctttgtt gaataaattc ttgagatagt ttgtccccac aggctagtcg 60
   tcgcttcttg tgattgcgag gagtctggcg atttcgttag ggtttcgtct tgtgtgctgc 120
   ctcacacccc caattggggc cgggtattta gcgcgctcgc cgagggtttc ggccttttaa 180
   ctagcatggc gtatagatcc atgcggtcga ccttgcgcct gaccgtccac tttttaggga 240
   ctaggagtac agc 253
<210> 16
   <211> 176
   <212> DNA
   <213> Corynebacterium glutamicum R
<400> 16
   tttgcttgaa tttaggattt cccttgcgaa gttgagttgg taatttcaac ttcgcaaggg 60
   aattttgaac ttcaaatgaa atagagcaag tgctgtaatt gttgatatag ttggttggag 120
   accagatagt ttcttgagcc atatcaatgt gtctgaacat tctttaagga ggaatc 176
<210> 17
   <211> 251
   <212> DNA
   <213> Corynebacterium glutamicum R
<400> 17
   cttttcgatt tccaccaaac ccgcacgtag atcgagttcc cgtttcatga aattggtgga 60
   cgaaaaccag gtttggagtt tagacttggc gaaattcacg aaaactattt attgccgggc 120
   tacctggatg aacatcggtg taattttagg gattattttc gagggtcccc acgttgtact 180
   gagcagttgc caatacggtt gccagttcga aatttaaaaa atggtggctt agagctataa 240
   aaggaggaaa a 251
<210> 18
   <211> 100
   <212> DNA
   <213> Corynebacterium glutamicum R
<400> 18
   caagcactgc tgcgggagcc actatcactt tgttaagtgc tgcgatattt tttgccacct 60
   tattgacaaa gagtgccatt agtgggttaa acttcaccgc 100
<210> 19
   <211> 221
   <212> DNA
   <213> Corynebacterium glutamicum R
<400> 19
   ctaaaacgat ttatagcgaa atttaggcga tttttaaacg atacgaaaat cgcctacttt 60
   ggcattttat tttcatgaac acgcaggtca agggttagtt gcagaaattt tccgaataac 120
   aggctattgt gtctatcagg aatacagtta atacatcttg aaaagcccat gggccatccg 180
   aattcccagg atcggcccgc tcactccaag ggggtcaggc a 221
<210> 20
   <211> 312
   <212> DNA
   <213> Corynebacterium glutamicum R
<400> 20
   aatcgatcaa ttcctttcgg gtaacgagaa aacgtgaact agaaacggag tcaaagtaaa     60
   tatcaaaggt aacaccatcg gtaaatccaa actgacaact ataaatggtg cccgatatca    120
   ggaaaaattg cttgcacacc gcgccgattc cccatgatgc cctaacatct tgcaggtaag    180
   gggtacatat tggggcaatt cgggggtgat tttgcagtat cgtcaagatc acccaaaact    240
   ggtggctgtt ctcttttaag cgggatagca tgggttctta gaggaccccc tacaaggatt    300
   gaggattgtt ta                                                        312
<210> 21
   <211> 417
   <212> DNA
   <213> Corynebacterium glutamicum R
<400> 21
   ctaacaggcc acagatctta gctttgacca gctgatttgt ggctaatcgc ccggtctgtg     60
   tagagtattc atctgtgcgc aggacagtgt gacaaacact gaatagtgca tggctttaag    120
   gccctgtggc gcagttggtt agcgcgccgc cctgtcacgg cggaggtcgc gggttcaagt    180
   cccgtcaggg tcgctggagt tggtctatac tgtagagcag cccctggcca gatagctcag    240
   tcggtagagc gttcgcctga aaagtgaaag gtcgccggtt cgatcccggc tctggccacc    300
   atctacaccc tgttttattt cctaggaaat aaaacagggt gtctttgtgt ttaaaaggta    360
   taaaggaaga gtagttccgg ttaattccac cggggtgaga taccgaggag aacgcac       417
<210> 22
   <211> 100
   <212> DNA
   <213> Corynebacterium glutamicum R
<400> 22
   tgaatctgtg gcggcaccgg cgcgtgttgc cctggagagg atgctgtctg ttgtcccagc     60
   ggctcctgtt actcctagct cctcgaagga tgcgtaattt                          100
<210> 23
   <211> 81
   <212> DNA
   <213> Corynebacterium glutamicum R
<400> 23
   caggtcaagt gcgtaattga gggctagact ggttagtacc ggatattctt ttttctttag     60
   tttgtaggag tggagataac t                                               81
<210> 24
   <211> 276
   <212> DNA
   <213> Corynebacterium glutamicum R
<400> 24
   acttttgccg tacttacggt gcttaattat agctagtgtt acagatgtta cttttgtgac     60
   gtgggaaaat gtccgaaatg ttatgaagtt taaaccttaa ctttagcttt agtaaatggg    120
   ggataaagct taagttgagg gttgccgggg tccacttact caaccacaat cgcctgtgta    180
   gtcattcaag gaattttgaa gcgagctcga ggccgttggg gaagacgaac ctcgtcttac    240
   ttcctttagt aactgcacat atctacttgt atatgt                              276
<210> 25
   <211> 138
   <212> DNA
   <213> Corynebacterium glutamicum R
<400> 25
   caccgcgaat tatagactga actggtactt gggaagcttt ttatagtcat atgcgttgag     60
   atacgtggac gacaaagcac cagttggttg ccttcccagt ccagcccaca tccgatttct    120
   aaattaggag catatctt                                                  138
<210> 26
   <211> 215
   <212> DNA
   <213> Corynebacterium glutamicum R
<400> 26
   accagcactc attggagaaa taagaacgcc ggaaatgagc actgccaggg ctttagttaa     60
   ggagcttcgc attgttagag catacctacc accatgttgg aggcgctagg caacaagaga    120
   gttttatgct tatcgacgcc accctccacc cacccttctg cccacgcagg tgctaccgtg    180
   aggctgaagt ggaaaatcta ctaggagggt attgc                               215
<210> 27
   <211> 84
   <212> DNA
   <213> Corynebacterium glutamicum R
<400> 27
   aaccgccaaa cttttgatag cgtagcaatg gccctttcgg tggatggggc catgtttgca 60
   caatgcttga agaagggtag ggca 84
<210> 28
   <211> 76
   <212> DNA
   <213> Corynebacterium glutamicum R
<400> 28
   aaatgctctg ctgctggctt tagctgcggg gctgttggga attctccaaa accttctaaa 60
   ccgttcaggt aggaaa 76
<210> 29
   <211> 216
   <212> DNA
   <213> Corynebacterium glutamicum R

<400> 29
   ttagttcaca tcgctaacgt gggcgatcga tgctcacggt gatgtgtcat cccaatagcc 60
   cggggtgtgc ctcggcgcac cccgggctat tttgtgtctt taatcaatac aattgaatac 120
   cggtgccagc gccacacaat gtgtggcaat ctgggacagt gcatcacatt gcaccagaag 180
   aattttttaa acaatcaaat ctccaaggag tacggc 216
<210> 30
   <211> 382
   <212> DNA
   <213> Corynebacterium glutamicum R
<400> 30
   gtgtttgaag ttgcctttcg tgagcccttt tatggaaaca agggtgtgaa aatcaagtag 60
   ttaaaggtgt ttcaagtcca ggttgtttaa ctctcctaga ccgcttggtc tgtaaacgta 120
   gcagcgaaat gcgacaatgc gaagactttt gcttaattaa attcaaactc catcaaaaaa 180
   ctagacagat cagtctatta tattcacggt gaacctaacc taatatcccc aggttaattc 240
   atttaaacgg gcgtaaggtg actccattgc tttcagtctc atgaatgtaa tggttggtct 300
   agacagagcg gtacgtctaa gtttgcggat agatcaaacc gagtgacatg tacttcacta 360
   gctctttaag gattaactcc cc 382
<210> 31
   <211> 156
   <212> DNA
   <213> Corynebacterium glutamicum R
<400> 31
   agcacagcct taaagcacaa gcactgtaaa agtgcggttt tgatgcgccc tgcaggccat 60
   cgaaattaat cgcccagcca aacacaggtt ttgttgggcg tttttttatc tcatgcacgc 120
   caacaccctc aatgtgaaag agtgtttaaa gtagtt 156
<210> 32
   <211> 488
   <212> DNA
   <213> Corynebacterium glutamicum R
<400> 32
   tcgctcgtct cataaaaacg accgagccta ttgggattac cattgaagcc agtgtgagtt 60
   gcatcacact ggcttcaaat ctgagacttt actttgtgga ttcacggggg tgtagtgcaa 120
   ttcataatta gccccattcg ggggagcaga tcgcggcgcg aacgatttca ggttcgttcc 180
   ctgcaaaaac tatttagcgc aagtgttgga aatgcccccg tctggggtca atgtctattt 240
   ttgaatgtgt ttgtatgatt ttgaatccgc tgcaaaatct ttgtttcccc gctaaagttg 300
   gggacaggtt gacacggagt tgactcgacg aattatccaa tgtgagtagg tttggtgcgt 360
   gagttggaaa atttcgccat actcgccctt gggttctgtc agctcaagaa ttcttgagtg 420
   accgatgctc tgattgacct aactgcttga cacattgcat ttcctacaat ctttagagga 480
   gacacaac 488
<210> 33
   <211> 197
   <212> DNA
   <213> Corynebacterium glutamicum R
<400> 33
   aagtcgcttg aaccggcatg aaaatctcgt gctggttttg ggctcacaag gccatatagg 60
   aactttgtaa ttagttgcag gttccaattt tgggtcaatg tagcgtaata ttgttcaagg 120
   cccatgtgcg ggctgtggag gacgtgcatt cacgttctgg tcaaatgaaa aacggtgaaa 180
   gggattgaac gcagcag 197
<210> 34
   <211> 100
   <212> DNA
   <213> Corynebacterium glutamicum R
<400> 34
   gcttgcggga acaccgcacc gcccacccaa actgttcaga ttccaaagat aaattctgac 60
   gctcattcca gcccaccgtt tagaagaaaa gaccccaatc 100
<210> 35
   <211> 138
   <212> DNA
   <213> Corynebacterium glutamicum R
<400> 35
   tcgaagttcg gtggtttgtg ggggcgtttc tgcatttaaa ggtgatagat ttgggcaaaa 60
   atggacagct tggtctatca ttgcggaaaa gacctgttta tacaggtgtc ggaaatgagt 120
   atcaggagct gatctacc 138
<210> 36
   <211> 82
   <212> DNA
   <213> Corynebacterium glutamicum R
<400> 36
   gcccgcgtac cggctacccc gaggcacgta atgagtaggg cagttctagc cataatcagc 60
   agaaaaggtg gagtgattcg cc 82
<210> 37
   <211> 65
   <212> DNA
   <213> Corynebacterium glutamicum R
<400> 37
   aatatgacct ttgctggttg gctacttttg ggtcaaagtt ttgtgaaacg aagtaagctt 60
   aagtt 65
<210> 38
   <211> 76
   <212> DNA
   <213> Corynebacterium glutamicum R
<400> 38
   tacataccga tacccaagaa tgcctcaaag ttccgtattg tgggaggttg aagcagatat 60
   ttaaggagag gttcaa 76
<210> 39
   <211> 100
   <212> DNA
   <213> Corynebacterium glutamicum R
<400> 39
   tccagcaatt gcaggatgaa gcccgccgag gcaccacagt actagcggtt tcgcatgatc 60
   cgttgctgcg tgcggctgcc gatgaagtgg tggaggtcaa 100
<210> 40
   <211> 100
   <212> DNA
   <213> Corynebacterium glutamicum R
<400> 40
   tcataacgcc catcataact acatcgagcg aaatgccaac cacatgtccc atgcttttac 60
   taatgtgggg tcttagaaga aagcgaccaa ttaaggagag 100
<210> 41
   <211> 100
   <212> DNA
   <213> Corynebacterium glutamicum R
<400> 41
   tgctggccta ttgtggcgac tgagggcctt tgaaggttcg acaaactgta taaggcctta 60
   aatcttgaga atctattttg aggaagcaag aggaagtgtc 100
<210> 42
   <211> 100
   <212> DNA
   <213> Corynebacterium glutamicum R
<400> 42
   agatcgccac ctccaccctt accgaacgtg gcgcaacccg cgccgatgac cgcctcagcg 60
   aatccgcaat ggaagaccgc aagaaggaag gctacttctg 100
<210> 43
   <211> 328
   <212> DNA
   <213> Corynebacterium glutamicum R
<400> 43
   ctcacggtcg cagtcgtagc cggagcttat agaatctcgg ttcggatgct gaatagatcg      60
   tttgaaggtg aagaatacgg agtgggggga attaatccgc cacctccgat tggaagcagt     120
   gcacgaagac ctccgggatc ctcgcctcca gaacctgacc attttggcta ctggcatgca     180
   ccacggcatg gcttccgggc tttgtcatag ggtccaaact agtgctaatg taataggtag     240
   cactctagat ggcgcacagt gactcaattc actgtttctc acactacgga tcgttcggca     300
   cgtacctgcc gatggaggag attctgca                                        328
<210> 44
   <211> 242
   <212> DNA
   <213> Corynebacterium glutamicum R
<400> 44
   gtcggatgct ctcctccacg tcgaatgaaa aaataaccgt cccttttagg acatacccaa      60
   ccaatcctag cccaactaac gatgttgctt ccgctgaccc cgtcattttg cggtcttcgc     120
   gatgtaaact agttctaata actatttttt catttccctc ataaaaggtt tatatagaag     180
   gtaaaatagc aagcgtgcta gatccattcc agacctagcc taaggacgga aggacttccc     240
   cc                                                                    242
<210> 45
   <211> 292
   <212> DNA
   <213> Corynebacterium glutamicum R
<400> 45
   tgacacccca ccggatgtgg ctgcaaatga ccggttgccg ggcacttgcg taaagaggtg      60
   ggaacactac cctcacggcg gaagaaaccc acgaagtcca cataaccagg cactgcgaca     120
   gtggtgaacc tcggcatctg tgccaacggc ttctgacatg tgtgtcaacg cctcctgaca     180
   ctgacagaga cacttctgat cccactattt ctactgtctc actaagtcct aatctacttt     240
   ttgatacact tgtctcaaga agttattaat cctattgaga cagcgggacg ac             292
<210> 46
   <211> 100
   <212> DNA
   <213> Corynebacterium glutamicum R
<400> 46
   caattggatg cgaaacctgc acccttcccg ttgctgaagg acaagaccct agggccggcc 60
   gttgggctgg aaacgccaag acagaatgcg gacttcactc 100
<210> 47
   <211> 125
   <212> DNA
   <213> Corynebacterium glutamicum R
<400> 47
   tcacttcctg ttgtggctgc ctgttgcagc caaatacggc caacggctcc ttggaaattt 60
   aaggggtcgt tggccttatt attttaggga aagcccatcc ttgccttgcc aagggaagcc 120
   tgtac 125
<210> 48
   <211> 59
   <212> DNA
   <213> Corynebacterium glutamicum R
<400> 48
   gatacaacgt tcccggcgcg cacaactttg tcgcgccggg ctttggagag gagtttgtg 59
<210> 49
   <211> 126
   <212> DNA
   <213> Corynebacterium glutamicum R
<400> 49
   ccagtcaagc ctaagagctt gaaacgcccc aatgtggggg tgttaagaac tccacaaaag 60
   cgcttgggaa ctttttgtgg aagcagtccg ttgaacctct tgaaccgcga atttaggagg 120
   ccagtt 126
<210> 50
   <211> 100
   <212> DNA
   <213> Corynebacterium glutamicum R
<400> 50
   gtcggttcgg aaacaatgga aattatccga aactctcctg taccgaccat catttatccc 60
   ggtctttagg ctctccaaca gcagaggact agactgaagt 100
<210> 51
   <211> 339
   <212> DNA
   <213> Corynebacterium glutamicum R
<400> 51
   ttcaaaactc ccgcctcaat tgtttgccca catcttaact tttagccacg acacgttcga      60
   acaccaatac cgttcacatg ttcgactaag tttctatgtt cgatttatag caccccgttc     120
   gaacgttgtc tagagaagat ttagaaatct cgaacactcg taccatttcc gcaggaaaac     180
   ctgtatggtt ggaaaataga aatactgaca gaggttcgaa tagctcagca ttcagcttca     240
   gaagttcaca ctggatgaac cctcatcaag agtgaacatg gaagcgaaca gagtttttag     300
   tgctttaacg ggcacacaat gagaggaagg gaacggaag                             339
<210> 52
   <211> 131
   <212> DNA
   <213> Corynebacterium glutamicum R
<400> 52
   gatcaacctt tcgtatgaat tttctgtata aaacccatgt tagatgtttt attcagggat      60
   tttagttgat atgtccagta tctcgctgaa aacgctggtt gtcttgtaga aaaaggcgta     120
   acgtcatata c                                                           131
<210> 53
   <211> 956
   <212> DNA
   <213> Corynebacterium glutamicum R
<400> 53
   gagctttcat ctctcgtgat gtgatgggca actaatctcc gggctaacgg tgttggctaa      60
   ttttggctag agggtatttc ctgcaatttt ctaacccggc aacctgcaga tttggccgta     120
   ttgcatggtt gtggttatag tattcctcgt tgaacagcgc gccatgcgag ttgcttggag     180
   cgtaagtttt aaacattggc ctatggtgta attggcaaca caacggtttc tggtaccgtc     240
   attctaggtt cgagtcctgg taggccagct gcgatcagct ggataaacaa ttgatcgtgt     300
   cctaatgccc cgttcgtcta gcggcctagg acgccggcct ctcacgccgg taacacgggt     360
   tcaaatcccg tacggggtac tccgcatcat gtatttgcgg taagttttta aatgttttac    420
   aaggtttaca atcttgaaaa acgattggcc tatggtgtaa ttggcaacac aacggtttct    480
   ggtaccgtca ttctaggttc gagtcctggt aggccagctg cgatcagctg gataaacaat    540
   tgatcgtgtc ctaatgcccc gttcgtctag cggcctagga cgccggcctc tcacgccggt    600
   aacacgggtt caaatcccgt acggggtact cttaaaagca tccacccttc atcaattgag    660
   ggttggatgc ttttgtgttt gctaacacat ctgacatcac cccctcccag gacaaatgta    720
   cgggctcatg tagcagggga ttttaaagac gcgggggtga aagatctcca gcggttgcgg    780
   agttaaatcg caaaagcctt taccgagatt cggtctcggg ttaaagtcag gttcatacag    840
   agaagttcat agccgatcac caacaagttg ttgctctaac cagcacaaat gcaactttgg    900
   aggcttcgga aagggcgctc tggagtcaat ggaaccgacc agtaaaagga gagtgt        956
<210> 54
   <211> 275
   <212> DNA
   <213> Corynebacterium glutamicum R
<400> 54
   agtgatttct cctcatggat gaaggtggac actcggccgc gaaaataaaa aatgcccgcg     60
   accgactcga atgtcttggt cgagtcttcg caggcagcgc acaaaagtgc actttagaag    120
   tctagatcgc gtcgataacg gttgagcact aaaagtactt gaccctcgcc gagacctaaa    180
   tatgctagca cgatggccaa acctaatcca aaaaccggca gtatactggt tggggtcaca    240
   ttagccaaaa ggcgctttga ggaagtggaa ctccc                               275
<210> 55
   <211> 382
   <212> DNA
   <213> Corynebacterium glutamicum R
<400> 55
   ggggagttaa tccttaaaga gctagtgaag tacatgtcac tcggtttgat ctatccgcaa      60
   acttagacgt accgctctgt ctagaccaac cattacattc atgagactga aagcaatgga     120
   gtcaccttac gcccgtttaa atgaattaac ctggggatat taggttaggt tcaccgtgaa     180
   tataatagac tgatctgtct agttttttga tggagtttga atttaattaa gcaaaagtct     240
   tcgcattgtc gcatttcgct gctacgttta cagaccaagc ggtctaggag agttaaacaa     300
   cctggacttg aaacaccttt aactacttga ttttcacacc cttgtttcca taaaagggct     360
   cacgaaaggc aacttcaaac ac                                              382
<210> 56
   <211> 297
   <212> DNA
   <213> Corynebacterium glutamicum R
<400> 56
   ctgtgtccat ttcgccgtcc atgccgcctc cactctcatg gtgtgctcgg aagcgggcgt      60
   gaggttaacc ctaatagggc ccgcgggtgt tcttgtaaat tgcccaggga ccggtatctt     120
   cccccggttc gccccctgca gggagcaccg acctgggaag aacaacaccc ggtgaaggcg     180
   ccgtggccgg gaactttcct cccgcctgtt ccgactagtg tcttggcacc tcccgcaggc     240
   aggtgtctta gtcgctcaca tccggttgaa gaaccggccc agtccaggag gactttc        297
<210> 57
   <211> 1000
   <212> DNA
   <213> Corynebacterium glutamicum R
<400> 57
   accacgtttc tgaatctgga gcgatgagca ttggtcaaat attcgaggtt ttctttgctg 60
   tcctgcgaaa taacatccgt gtgatttgga aagtgggttt tcactagggg ttttacctgt 120
   gcttttgtgg cattgacttg gggatttgtg ttcgagggag cgttcggtct aaagttattc 180
   cagcaccgca cagaacggaa caagcaggaa caagttctaa gtagtgcatg cggatgtagc 240
   gcagttggta gcgcatcacc ttgccaaggt gagggtcgcg agttcgagtc tcgtcatccg 300
   ctctcatttc accgggccaa tcgatgaaat gtcccgcgcg tttagctcag cgggagagcg 360
   cttccctgac acggaagagg tcactggttc aatcccagta tcgcgcacca agaggacgaa 420
   caacctcttt aatcaatgtt catgcggatg tagcgcagtt ggtagcgcat caccttgcca 480
   aggtgagggt cgcgagttcg agtctcgtca tccgctcaat ttaaaacacc tatgggttaa 540
   cgctcatagg tgtttttgcg tgcttcccaa aaagcgtttt aaggggtcga agtgctcaag 600
   atgggtcgat atatggattg aggtcattca ggcggctgag cgcgatttta ggggccctca 660
   cttttggggc cggtggggca gttgtgaatc ctgaaagctt ccagggcaag gatccaccac 720
   aaaccaggct ggactctaga aatcggtcct aaagttgcac acgcaggcag ataaagcgga 780
   aatatccacc acagccagca ggaaatccgc ttttcgtggt gaatattcgc acattttttc 840
   tattcatgta tgggtgcata tggtaatgcc ttcccgccct tgagttctac cccggaatct 900
   gctcaaggca cccccagaat gggaacgtag tctagatcac acgacttcga aaagtggctc 960
   caagctggat agactctatt tacctactgg taacctccgc 1000
<210> 58
   <211> 100
   <212> DNA
   <213> Corynebacterium glutamicum R
<400> 58
   atcgattgtg ttgccgtaac ctggggctac ggcagcaaaa ctgaatggga cgctgcccgc 60
   tacaccgtga gcaccgcaga agaattagaa aggatcatcc 100
<210> 59
   <211> 100
   <212> DNA
   <213> Corynebacterium glutamicum R
<400> 59
   gcatatgctg aagcagaaac taattcaggt tttgatcccc gcgctaactg ggcgggccag 60
   aaccgctaga cgttctcttt gagaaaggag gtgacgaaac 100
<210> 60
   <211> 246
   <212> DNA
   <213> Corynebacterium glutamicum R
<400> 60
   aacaggaatg ttcctttcaa aaattgagga agccttatga cctacaaccc tactcagctg     60
   ccgattattc cgggtttgtg accagctacc cgataaatag gtcggctgaa aaatttcgtt    120
   gcaatatcaa caaaaaggcc tatcattggg aagtgtcgca ccaagtactt ttgcgaagcg    180
   ccatctgacg gattttcaaa agatgtatat gctcggtgcg gaaacctacg aaaggatttt    240
   ttaccc                                                               246
<210> 61
   <211> 145
   <212> DNA
   <213> Corynebacterium glutamicum R
<400> 61
   actaccccct tttgatgtaa cacataactc aatcttgcga tattagataa tgagtcagaa     60
   aatgaaggta gcactacttg ctggattaat tttcaaggct ttctttcaga aattaatcca    120
   tagccgatat ttaaggtgag aacac                                          145
<210> 62
   <211> 167
   <212> DNA
   <213> Corynebacterium glutamicum R
<400> 62
   aactctcagt tagaccatta caagccaccg gagccacaag ttggaccggt ggctttgtcg     60
   tctattcggg gagtggctgt ttatggagct ctttgacgca ggtcaaggaa gttcagtgag    120
   cgcaaccata acgttaaaag taaggaaaag agagaaagga agaaatc                  167
<210> 63
   <211> 87
   <212> DNA
   <213> Corynebacterium glutamicum R
<400> 63
   actgcgtgag gttgtggcct gtcacacata atcagcctag ggtgggactt taaggaaaca     60
   gtgcacaaat aaatctcaag gagcccc                                         87
<210> 64
   <211> 100
   <212> DNA
   <213> Corynebacterium glutamicum R
<400> 64
   gctactgccc tcaccattcc acgggcattc cgtgcggggt agaagcttaa aagatttttg 60
   cttttcgacg cctccctcca cctcggatta cgcttggggc 100
<210> 65
   <211> 100
   <212> DNA
   <213> Corynebacterium glutamicum R
<400> 65
   acaccgtggg aaagattgca tcaaccggtg tcgatgtcat ttccgttgga gcgcttaccc 60
   attctgtgca tgcacttgac ctaggactcg atattttcta 100
<210> 66
   <211> 119
   <212> DNA
   <213> Corynebacterium glutamicum R
<400> 66
   ggggtgctcc taaaaagcga aggtcaaaaa aacttgacct tattgataac gatttctatt 60
   aagaagtgta cacttgcgtt gactctattg aaaatgattc ccaagaggag ggctttccc 119
<210> 67
   <211> 1000
   <212> DNA
   <213> Corynebacterium glutamicum R
<400> 67
   cgctggttcg gctgcgccaa ccaaaccaac tatttgaaag gagtcaatta tgtctaagcc 60
   aaatgaattt gttccaccgg acgcttcatt aatggagctc acggtagtca actggacgtt 120
   gagcaaaatg aaagaaaaaa tcctgactgg agttccgtca ggtcaggtta ttgaccaagc 180
   actcaaagag gtattggata gttcattaag gaagtagctc aaatagatgt tgtccaagtt 240
   ctccaagttg tgaggctgcc cagttcgctg gtgcagtagt cattgtgctt gtcgtgaatg 300
   gccatacaaa cttgtctttt ataaaggtag cccaacgatt tttttctgtg gcatttgggg 360
   cagacgaagc catgaaaagt actgtcattc tgaattgcat cgcagcttca tgaagttcaa 420
   attcacctaa cttgtcaaag ttttcaatgc accactcgag atgattacgg actcgagcta 480
   aataagcttt aagttcaaag gggtagtctt gagcaggctt atttaagata tctgagtcaa 540
   gcatgtctct gagggaatct aggccaccct cgttaagttt ggggacgtac aagtctaaga 600
   aagcggacag cgaaagcaga ctattgagtg cgtgttcgtc gacaccccca ttagctcccg 660
   agcttcctga ccaaggcaca tctaagccaa ataccgctct gttccaactt tctatgtacg 720
   aagtccaagc agttatctgt tgtccgttgg cctccatttt gttgagaaga tcaatgcagt 780
   tctgcaaatt cgttacagca aaacgcatat ccgaccatcc cacctcgcta gggaggttac 840
   gtgtggtttt aagcgaaccg gtcttccgcc attcgctgag ctgagtatgg agtaattctg 900
   ctgggtttgc catagttctt atcaaaccac atacatgtaa gttgcccgcc acctggatca 960
   gaggtggcgg gcttttctga tttcaagaaa ggaaatcatt 1000
<210> 68
   <211> 241
   <212> DNA
   <213> Corynebacterium glutamicum R
<400> 68
   gtcttcggag gaaacccaat cccaaccgca accaccctct gtactgccca tactgcgcgg 60
   gagaagttct tttccccgat gagcaaacag aattcgcgtg gttgtgtgcg gattgcacca 120
   gagtttttga agtgaaatat cacggccagg acgatccagt gcacaggcca gcaccagcaa 180
   agtccacatc gcaagcatta aaagaatctc tcgaaagaca caaaagaggt gagtcgcaac 240
   a 241
<210> 69
   <211> 89
   <212> DNA
   <213> Corynebacterium glutamicum R
<400> 69
   tctgggaaga aaccgctaat ctgggaagaa accgctaatc gacgctgcag gtggcggtca 60
   gatccacggg agaaggagag gacacgcgg 89
<210> 70
   <211> 100
   <212> DNA
   <213> Corynebacterium glutamicum R
<400> 70
   attgattggg taatcaaaga cgcaggcatt gaggatctgg caaccggtga gatcaccgtg 60
   ttgatcaacc ccttcaggtt ccttcattct gggtggcccc 100
<210> 71
   <211> 232
   <212> DNA
   <213> Corynebacterium glutamicum R
<400> 71
   gaaagttttc cacactaaaa tagtgtgatt ctgtccgaat ctgttgtttt agttttgaaa 60
   ctgcgggatc atggaaagta gtgaaaagtg aattttagtt ctgtgctttc tctgcccttt 120
   aagtgaacct tttgttggat cttgcattaa aaaaatgaaa acctcgtcgg gaatgcaact 180
   tgggatcacg tctcgggcaa gaaacgtcct taaaaaaggg gagtgattgt ga 232
<210> 72
   <211> 75
   <212> DNA
   <213> Corynebacterium glutamicum R
<400> 72
   gcatggagaa tttacttgca ggcgcgttat attgcaagtg gtgttattat gaaagtgagt 60
   tttaggaggt caacc 75
<210> 73
   <211> 67
   <212> DNA
   <213> Corynebacterium glutamicum R
<400> 73
   cataataagc ctaaagcttt cccatattta ttagcctctt agagttctca ggagaaaacg 60
   aaatccc 67
<210> 74
   <211> 110
   <212> DNA
   <213> Corynebacterium glutamicum R
<400> 74
   tgttaactct tccttttttc ctatgcatta ttttgggcat gcacttgtca tatcttcaca 60
   gacgtatggt tttgtaacaa cctcccccaa aatccctaaa gtttcccccg 110
<210> 75
   <211> 376
   <212> DNA
   <213> Corynebacterium glutamicum R
<400> 75
   aatcactgct tctgcgggaa tacttgtgcc agagacattg ttgcgttgtt cgtaggctcg 60
   ttgcctacaa gcttggctgc agtatttcct tggacgtccc ctgcctgttg aggcgacgtc 120
   tttaccgcac caggagcact ttgtagtctg ctggtttggt cttaatttct gcacctttaa 180
   caccttagtc gattgcctcc catacaacga ttcgggggtt ttggctatac ttataggtct 240
   gatcacattt caagggcagc tgttgtgact ggcagcgtgt cgttttattt gcacactagc 300
   aggtaaactg tgtgagacga attcttttgc ggcaacttgg aggccgcatt ctagtgggca 360
   aaggactgat acctag 376
<210> 76
   <211> 92
   <212> DNA
   <213> Corynebacterium glutamicum R
<400> 76
   cacaattgca cattcgtctc atcgattccg atgagattgc cacccccaaa atggggcgcc 60
   tatttttttg acggccaccc gcggtagcgc tt 92
<210> 77
   <211> 103
   <212> DNA
   <213> Corynebacterium glutamicum R
<400> 77
   ttgtttggat tcctttccga ttatgtcttg atcgcccatt ctgtcacatc cgggttgtct 60
   aaacacccgc gaagatttcc tgtgatgtgc cacactggtt ctc 103
<210> 78
   <211> 146
   <212> DNA
   <213> Corynebacterium glutamicum R
<400> 78
   tcacatcaca ctgggattac cccgtgtagg ggtgaaaacc cgaatgatga ataaaattcc     60
   gggtgcagtg accgtaggtg aggtaaacgc ggttagagtc gaatgagagt ttgatacttt    120
   ctttcgactt ttagattgga ttttca                                         146
<210> 79
   <211> 143
   <212> DNA
   <213> Corynebacterium glutamicum R
<400> 79
   tcttggatat aaaaggggcc cctattggca atgtggttat gaccagaagt agtataagaa     60
   gtgaagttga tcgcgtcatc gtagtacgag taattccact cacacatcaa tgcggtgacc    120
   acaattggga ggagaagtag cac                                            143
<210> 80
   <211> 82
   <212> DNA
   <213> Corynebacterium glutamicum R
<400> 80
   accagttgct gtgaaggcaa ggtgtcggct tagatgccgg cgcctagcct acatccagct     60
   aagaccccct ttaggacacc tc                                              82
<210> 81
   <211> 212
   <212> DNA
   <213> Corynebacterium glutamicum R
<400> 81
   accagaagtg gtgacgtgag ttgggtgttc cgcagaaatc tatgcaggaa tcggccgaac     60
   tgaagtagtt tacaccccaa aatcgattat tcaaatatgt gttcgaataa tggattttga    120
   tgtccacccc tgtccatata gtgagggcta acaaaagaaa cgaaggtttc taagtcttca    180
   aagctagagc ggattgaaag gagaggaaga ca                                  212
<210> 82
   <211> 169
   <212> DNA
   <213> Corynebacterium glutamicum R
<400> 82
   attttgatcc ctatcatcga tatttcacaa aatgagcaag atagcgatat ttttatggcc      60
   tttatttatc taggtactct cctagttctc attgggtgca tggctttgtg cgaccaccgt    120
   tggaagctag cgttcttccg ccgtccctta cgagcaattg tttcggtag                169
<210> 83
   <211> 84
   <212> DNA
   <213> Corynebacterium glutamicum R
<400> 83
   gtaggctggc gggcaggtgc ttgaaatact ctgattagtt ccaagcaaat tagcacaact     60
   tcacacttta tttaggagca tgtt                                            84
<210> 84
   <211> 341
   <212> DNA
   <213> Corynebacterium glutamicum R
<400> 84
   ggaggaccag accaaaccag cgtgcccacg gttggccatt tcaggatccg tgggcacgct     60
   ggtttggttt atcaacctcg aaaccggcca cagctgccca cgaaactcga tttcgcgatc    120
   cgtgggcact ctcgtttggt ttcctcggaa agctaccctc ggacggtcag aaaatgccct    180
   ctcagaatcg cgtttaagag cctctcaccc aataatccgc acaaataccc gttccgcgat    240
   cccaaccgct taaacgggcg tatattgact tccctgcaat acagcccaaa tcgaccaaac    300
   tagttgaccg aaaactagcc agccgaaaga ttctcggcca c                        341
<210> 85
   <211> 844
   <212> DNA
   <213> Corynebacterium glutamicum R
<400> 85
   ctccgtgcgg gtgccatgga cagatttcgc tctagggcgt ccgcagtgcg ctcatgatta 60
   ggtaccctcg tggaactctg ctcaacatgt ggcggactga tcgcaagagg atgccatgag 120
   cggataccaa gacggagtct cggggagtat accgcccgat gccgtgaact ttctggagtt 180
   cgttgctttt ccggaggaac gtcggattcc caaggaatgg ttggaagaac aagaagagct 240
   gctggcaaca gatccatcat caatcacgca acgcgcgttg actgtcatgc ccaggtgaga 300
   tgctcatatt caacttggtg gagagcaaag aagactaccc gctagggttt gcgagatggg 360
   gttccctgag gatgccttcg acgtcgggct gataatcgcg gaataccttg agctggcaaa 420
   agagcttgcg ccgctggaag cccagatttt aagggctatc cattcgattc gatcgtcatg 480
   gcggtcgagc gaacttggct gaagcaaagc aggacggcac ggcctcatct aggggctgta 540
   aaacgtgatg ctgtgggaga atgagtcatg gactcgtaga acatccacga gcacccagga 600
   cttgagctag ccgaaccggt agatggtgta gcgctggccg tcgccgatgc tttacgctcc 660
   accccgctag caccggcgct cgtggagttt ttgaaactca gtgatggtgc acgcttaggt 720
   gaggttgagg tcttttcctc agagcacatc gtggaggaaa ctaatttggc taagcactct 780
   tggcagctgc ctgtctcatt ggttattggc agctccggac cggagcgagc gcttgtcatg 840
   ctgg 844
<210> 86
   <211> 69
   <212> DNA
   <213> Corynebacterium glutamicum R
<400> 86
   ccagcagtgg ggagcgcaca ccaacaatca cgctcaccgc acaccaccac tcaagaaaga 60
   agatcaaaa 69
<210> 87
   <211> 100
   <212> DNA
   <213> Corynebacterium glutamicum R
<400> 87
   cgacagcgcg tgatgatgcc accacagcgc tcaatacgat gctgggaatc ggagtggatc 60
   atacccgcgc accagagcac cgcgtagagg agtagctgtc 100
<210> 88
   <211> 59
   <212> DNA
   <213> Corynebacterium glutamicum R
<400> 88
   atctttttag attaaaatca tgcgccccgc cagaacttgg cggggcgtaa atctatttt 59
<210> 89
   <211> 176
   <212> DNA
   <213> Corynebacterium glutamicum R
<400> 89
   ggagcagaag actcccgcta catcctgagc gccggtgtgc catgccgcgg gtcaatctcc 60
   ctccaggctg acgcggtttc aggaccccac ctcccacctg acgttcgtcg gacatccagg 120
   ggcgaccacc ctagggacaa ttgcagacta atcagtcccc actgtatagt cagtgc 176
<210> 90
   <211> 246
   <212> DNA
   <213> Corynebacterium glutamicum R
<400> 90
   gggtaaaaaa tcctttcgta ggtttccgca ccgagcatat acatcttttg aaaatccgtc 60
   agatggcgct tcgcaaaagt acttggtgcg acacttccca atgataggcc tttttgttga 120
   tattgcaacg aaatttttca gccgacctat ttatcgggta gctggtcaca aacccggaat 180
   aatcggcagc tgagtagggt tgtaggtcat aaggcttcct caatttttga aaggaacatt 240
   cctgtt 246
<210> 91
   <211> 100
   <212> DNA
   <213> Corynebacterium glutamicum R
<400> 91
   atggaacatt cgacgcggaa ggttatgagt tccgcgatgt ccccgccact gccccagcaa 60
   ccctttaagg ctttacccct aatacttaag gagatagaac 100
<210> 92
   <211> 116
   <212> DNA
   <213> Corynebacterium glutamicum R
<400> 92
   gatcgcgact gattcaaagt ggccggtgaa ctactcgcgg tagagcaacg tgagacgatt 60
   tccggctaga gcgtgtttga taattgaggg tagcgcggca tgggatcaga gatccc 116
<210> 93
   <211> 100
   <212> DNA
   <213> Corynebacterium glutamicum R
<400> 93
   accatcgtgg tttctccaga cggcaatgta gtggacacct tcccgcagcc tttcgaaacc 60
   atcgatgacc tcgaaaccgc tgtggcaggg gcgctgcaga 100
<210> 94
   <211> 131
   <212> DNA
   <213> Corynebacterium glutamicum R
<400> 94
   gtatatgacg ttacgccttt ttctacaaga caaccagcgt tttcagcgag atactggaca 60
   tatcaactaa aatccctgaa taaaacatct aacatgggtt ttatacagaa aattcatacg 120
   aaaggttgat c 131
<210> 95
   <211> 100
   <212> DNA
   <213> Corynebacterium glutamicum R
<400> 95
   aacccctaac tactgacctc gcaccacttg ttgcagcccg ttaccacgct gcattgagcg 60
   cactgctggc acaaatttaa aaccgctaag gaattcagct 100
<210> 96
   <211> 412
   <212> DNA
   <213> Corynebacterium glutamicum R
<400> 96
   agctgattat attaacaagg aaagtgactc tgatcaggga atactgaaac tgttgagcct 60
   atacccctgg atgctctcac aggttcgaat cccgttagct ccgcatcgtt tgtggaattt 120
   cactaccctg gtgaacgcac cacatttatt ggttgcagga tgcttccgga tttccggggt 180
   gtttccgatt gaactgatgt ggaccgcatt gtggcacagc ccgacgggct aattgggttc 240
   gagtcccata ctcctttctg acatagagtc gccgtctact ggattcttct ggtagaagcc 300
   tgcggttcgg cccaatcctg ttctgcttac aagttcaggg gacgcaccga aaccccgctg 360
   ctgctagacc cagtggcggc gactcgctag agacgttttc gtaggtgatt gc 412
<210> 97
   <211> 690
   <212> DNA
   <213> Corynebacterium glutamicum R
<400> 97
   ggctctgctg ggcttagttg ggcgctcaac ttagttaaaa ccagacacac gtgcccgcgg      60
   ttcgggtttt ccgagttggt gggcactctg gtttggttta tcgctttcga aaccggtccc     120
   agttgcccaa gtgtttcgat ttgaggattc gtgggcacgc ctgtctggtt ggccagaaaa     180
   actggcctta gacccaaaaa accagactcg ctgtatggct gttagacgca cttccgtgac     240
   ccactttagg ggaacgttcc ttcgaaaaac tcgacccttt aaacaggctt ctagagtgtt     300
   gatgctttaa ggggccgtaa atgtagggtt ctttggatag gagctaaggc cagggctcac     360
   ttttgctttg atcgggaggc tccaagccag caaaccagac acacgtgccc gcggttcggg     420
   ttttctgagt tggtgggcac tctggtttgg tttatcgctt tcgaaaccgg tcccagttgc     480
   ccaagtgttt cgatttgagg attcgtgggc acgcctgtct ggttggccag aaaaactggc     540
   cttagaccca aaaaaccaga ctcgctgtat ggctgttaga cgcgctcccg tgacccactt     600
   taggagaacg ttccttcgaa aaactcgacc ctttaaacgg gcttctagag ccttggaacc     660
   atttggactt cttcggagtc taaccagagt                                      690
<210> 98
   <211> 100
   <212> DNA
   <213> Corynebacterium glutamicum R

<400> 98
   ccgtgatgat cgtcgtgacg atcgccgcga tgaccgcgga gacgacctgg atgtacccag      60
   cttcctccag taattaagaa ggagaataga cttatccact                           100
<210> 99
   <211> 62
   <212> DNA
   <213> Corynebacterium glutamicum R
<400> 99
   tccctaggta aacgcactgg gtagtatttg tttaaccatc cacctcaagg agtaaaacgc      60
   ac                                                                     62
<210> 100
   <211> 179
   <212> DNA
   <213> Corynebacterium glutamicum R
<400> 100
   ctggtagggg ggggatatcg gccgcaccac tctttgcatc tagcgtgatt gttttctgaa 60
   gtcctgcgcc caaggcgccc gtttgcgatg cgtgacgttc ggagctgatg cacatatttc 120
   ctggcccatc ctccgggggc gggcaaggat tgttcagtgt ctgctgtata gttaagtgc 179
<210> 101
   <211> 82
   <212> DNA
   <213> Corynebacterium glutamicum R
<400> 101
   ttcacaccca aggctagccc tgcgcccctt ctatatctag ctgaccagta ttaataccaa 60
   taatagaaca tattatcgaa ca 82
<210> 102
   <211> 87
   <212> DNA
   <213> Corynebacterium glutamicum R
<400> 102
   aaacttctcg ctaaaggctt ctcctagaag cttctccatg ggttaacaca acccatatcc 60
   ctcccactac atatataagg actgaaa 87
<210> 103
   <211> 1000
   <212> DNA
   <213> Corynebacterium glutamicum R
<400> 103
   gcgcctgacc agtgattttg agtttcttca agttcacgct aatctactta agacttcaac 60
   ggagcacagc ctggctgttt ttgaagaagt agatgccccc ttagctcagt cggtagagcg 120
   tttccatggt aaggaaaagg tcaacagttc gattctgtta gggggctctt tttgcatttc 180
   tggaccattt tggaatcatt tctggatctc ttcaggccac aaatgcacac ctggcagacg 240
   cactcaaggc gtgagttttt agtttcaggc cacatgcatc gcagtggggg tgcaatttaa 300
   tccaagaaaa cctttagctt cctgcagatt tgtgttgcgg tagcggttcg ggttaaagtt 360
   cttaaggctt caacggagcg cagcggtagt tatacggact gcatttcaaa gagcttgacg 420
   cccccttagc tcagtcggta gagcgtttcc atggtaagga aaaggtcaac agttcgattc 480
   tgttaggggg ctctgttgct tttattctcg taagagatgt tgagcaatgt ggcggtgtag 540
   ctcagtggta gagcaagcga ctcataatcg ctgtgtcgcg agttcaattc tcgccatcgc 600
   taccgcggat gaaaccgctt ctaactgcaa attttttgag gttagaagcg gtgttttcgc 660
   aaattgacag gctgatggta acattgcctg tgctccatag gggcgtagct caattggcag 720
   agcaacggtc tccaaaaccg taggttgcag gttcgattcc tgtcgcccct gcaagatgaa 780
   cacccttgat ctggttaaac aataaaacca gatcaagggt gttttttgtt tcatcaaggg 840
   atctttcacc tgtagcaggt atgtcctaat aaatattgcg agggttcgcg ggattaatgt 900
   actctcgaag gttgaacaca gggctgcgat tgtgctggat caaatgtctg cacgaaaaat 960
   tgttatcgcc cctggatgag tagtgattta gaggagtgct 1000
<210> 104
   <211> 100
   <212> DNA
   <213> Corynebacterium glutamicum R
<400> 104
   acctcgccag aaaaccactg tgcaggcgct ggattaccta acggaaattc aatactcatc 60
   gtgcattcct tttctcatat cccatgcggg gcttataacc 100
<210> 105
   <211> 107
   <212> DNA
   <213> Corynebacterium glutamicum R
<400> 105
   gcgaccgaga ctagtggcgc tttgcctgtg ttgcttaggc ggcgttgaaa atgaattgcg 60
   aatgaaaagt tcgggaattg tctaatccgt actaagctgt ctacaca 107
<210> 106
   <211> 112
   <212> DNA
   <213> Corynebacterium glutamicum R
<400> 106
   gactaggggc taattctact tgtctttgcc gaaagtccaa gtgaaagcag cccacgcggt 60
   gccaggtagt ctggaggtga actaatcact atagggaggg cgacgaaaaa ca 112
<210> 107
   <211> 478
   <212> DNA
   <213> Corynebacterium glutamicum R
<400> 107
   gtctcacgag cttagtgttt tctggttttg gggttgggtt ctagttgggt tctagtgctg 60
   gataggctcc tctggcaggc ttgctgccat agaggactaa taggggaaag gatgtccgaa 120
   aatccaccac aaattcctgg cgagttacag tttgtggtgg atccttgcgt cccagggtcg 180
   gtttaatgca aaaatcagtt cgattaactg gttggacctc acggatgtgg tgagaatttg 240
   atcagcaacc tttcaggttt cactcctgcc ccactagtcg caaaatggac accctccaga 300
   tttgccccta agcactcaaa tcaaccccgc ccacacttga gaacattccg gaaatctaac 360
   cctttaaatc gcctcccacc ctcccgaaat tagaaatccc cttttaatgt gatatcactt 420
   tctgttaaac tgatatcaca ttctttttca gcaccccaga cttaaaagga gcaccacc 478
<210> 108
   <211> 374
   <212> DNA
   <213> Corynebacterium glutamicum R
<400> 108
   acaccaagaa ttgggcagaa ggtcattaac tttctgccca gttttttgct tttcgacgcc 60
   tacctccagc gcaccttgca ggttgctgcg gatcgctgaa cggctgcccg cgaagcgacg 120
   ggcaatctca atactgttta agaatcgaga aatcggttga gaagagtgct acgtttcgaa 180
   atgaactggg gtttgtttct gtggatcttt ttcattcagc ttgttgtgat gggattattc 240
   tcggtcagaa aacgcactaa gaataagaga ttgcgcggat agggcggttg cggtgaggtg 300
   ataaacgagg gcaggcacga gacctggtac atacggattc gaccaagaaa acgtaaaata 360
   tctcaggagc actc 374
<210> 109
   <211> 100
   <212> DNA
   <213> Corynebacterium glutamicum R
<400> 109
   agaacaaagt actgcccata ctcatgaact ttgccgaacc cccaaccccg ctggccgtcg 60
   atggcctaga aaaaatcatc gactttgtgg aaacccaccc 100
<210> 110
   <211> 100
   <212> DNA
   <213> Corynebacterium glutamicum R
<400> 110
   tggatgagcc tgagtacctg aaaaagatcc atgccaagaa tgcgacagcg gaaaagcctg 60
   cacctggcac cgatacgaac caagaggagg agaagtagcc 100
<210> 111
   <211> 341
   <212> DNA
   <213> Corynebacterium glutamicum R
<400> 111
   gtgttctcca gttccccttt gcgcgttgtg gattactctg gagccacacc aaacaacaac      60
   gccaacgtgc gtgccacccc cagttagcaa cacacatcga ttttttagta attaagaaac     120
   caagtctgtc aactgagaaa cacttttaaa gctcagtcgg cgtcgcacag ggcgcgacca     180
   atacaggggc agtctacctg tgtcttgact gagatcccaa cggaaatccg aattgcactg     240
   tatatattgg gggctattgc gcacgcttgc caagtgttgc tgcagctcaa caggttgtag     300
   caggtaggtg aaagatttat ggaaaatagt aaattccccg g                         341
<210> 112
   <211> 200
   <212> DNA
   <213> Corynebacterium glutamicum R
<400> 112
   tcttgccatt ctatttgctc tagctgcgca aacggcgtac tgtttattag gcgtgtcgtt      60
   gcgtcatgta tcggtgtgtt catgtaggaa agcacattga gcctgaacgt gagatcaaaa     120
   ccccgtctat acagggcatt tgaaagatac tgcatcctgt ccattatcta atttcctatc     180
   catttcggag caatttacat                                                 200
<210> 113
   <211> 100
   <212> DNA
   <213> Corynebacterium glutamicum R
<400> 113
   ctaatcagcg tttccgcatg gtcatcgctg atgcggtggc acagttgaag catccgcaag      60
   gttataagcc ccgcatggga tatgagaaaa ggaatgcacg                           100
<210> 114
   <211> 414
   <212> DNA
   <213> Corynebacterium glutamicum R
<400> 114
   tcgctagatg ggcgtgaaaa acttcccagt acctttcagt agaaggtgct ggggagtttt 60
   ttatttaagt aagcccaatc ggttgtgatc tagttcggtg ttctatgctg ctgcgatctc 120
   ctggcagatc tgaggatcgc gataaaactc atattttttc atcatggcaa aggcgcatta 180
   atccgacgac gagccaatgc gttgttttgc ggcacctgat ctaagtccaa ttctttttca 240
   ctttgggtta gttgccgctt cacggccgtt gccggttcag gtggaagtca ttgaatcagg 300
   cttctccaaa tgggcagtag acaactactt ggcgggtctt aaatcagctg tgaaggattc 360
   tgcataagct gggaaccaca cgagaatcag aacgcgaaac gaaggtaaaa gccc 414
<210> 115
   <211> 100
   <212> DNA
   <213> Corynebacterium glutamicum R
<400> 115
   cactcgttgg tctcctccgg atctgagtcg ttcaaataag atgccacgcc atcgcggatc 60
   atcgcgaagt tagagtcatc cgagggcttg ccaccaagtt 100
<210> 116
   <211> 100
   <212> DNA
   <213> Corynebacterium glutamicum R
<400> 116
   tagatatgaa aataagcccc caccaccata aaggttgtgg ggaaattaat tttgtgccag 60
   gacgaatatt aaagtaccag aactgacata aggtagcgtc 100
<210> 117
   <211> 100
   <212> DNA
   <213> Corynebacterium glutamicum R
<400> 117
   cactgatgac ctggatcagg ccgtcaaatt catcgtcgat gcacacgctg gattggacgt 60
   agcgcgtcgc cacaattaag cagtggctac attaggtgtt 100
<210> 118
   <211> 464
   <212> DNA
   <213> Corynebacterium glutamicum R
<400> 118
   cgagtacaaa caccgccagt atctgaacct caacagggaa cccggccata cctttaaggt 60
   gcggccgtgt tccttgtttc tgtggcgggt aggaaacggg gcaccttaaa ccgaacgggc 120
   atgtggtcgt ggagacaact tctgacggtg caaaagcggt gcggaaacgg caatgatgac 180
   gagcggactg tgcaggcatt acaggagtac gtcggtccga aaaagagacg cgaccagagc 240
   agccggaaaa atgcttcttt agggtatacc catgtgaaac gctcgatcgg ggcttatttt 300
   ccctgttcca catggtcaat ctgggttttt tggactacct gttgctcagg gtctaagaat 360
   gccaagacag gtttagcagt gcttcgttag gaatacatac gtcctgctca cccctcattg 420
   tgcagttatg ggcggagaaa acagcaaacg tgcagaatgg gcat 464
<210> 119
   <211> 1000
   <212> DNA
   <213> Corynebacterium glutamicum R
<400> 119
   gagatctcgg tgcggtcgta caaggaaaga cagtcacaac tgaatatgga tactttgctc 60
   ctggatcaag cacaaaccca ttcagtgtgg aacatactcc tggaggctcc tccagcggtt 120
   ccgcagcggc ggttggggcc ggcacgattc aatgtgcgct aggtactcaa actgcgggtt 180
   cactcactcg accagcatcg ttttctggag ccgcaggctt ggtcatgaca caaggtagta 240
   cttctctgaa gggagtacac ggtatgagtg gctccttaga ttccttggga atcatgactc 300
   gaaacgttga agatctagat tatgtgtacc gacacttctc tggaagagct ggagaacaat 360
   cagttgatcc tgaaaatcta agtatcttta tttgggatgg ttccgggttg ctcaaccttg 420
   atccagcaat gtcagatctc cttcgagccg tgaaaaaaat atttactgac agagaaatcc 480
   gaacacatcg attcatctgg gacgatcata ttgcgtcttt agtagatgac cacaagacca 540
   tcatgagcta cgaagctgct cgttcacttg gcgcgatgtt aaaggacaag agaaaacaac 600
   tcagtcctca gttacagcga ctacttactg aaggcgatgc agcaactgat cagatgtact 660
   cagaagcagt attccgtaga gacgcttcct accaagtgtt tttgaaaatt atgagtaggg 720
   attcagtaat tatcgggcct gccgcacacg ggcaagcact aaggttagaa gaggggactg 780
   ggtcccccga attaagtcga ccatggcagt tactgggact tccagttgtg actgttccag 840
   gagcctcaac ctcaactgga atgccattgg gaatacaact tatcggcaac aaacataatg 900
   aattgacctt gttgcgtcta ggaaaagtac tggaaccgct gctgcgcgag ctcccgtcat 960
   tttcaaacac ccagacacca tcaacactta aggaaatgaa 1000
<210> 120
   <211> 343
   <212> DNA
   <213> Corynebacterium glutamicum R
<400> 120
   agcgtaccgg aagtctattc tgtagcaggg ttcgtgagac agttcgttgg aatagcttgt 60
   ggaacagttt tctcttggga ctatgcattt tcaattggtt tcattaaggg tgtcccactt 120
   cctaggaagt gggacacctt gatcggtacg tgggtcgccg ctggcctggg ccggaagctg 180
   ccggcgatag ccctcacctt taaatcacgt cgggaacacc gccaactagg cagctgcttc 240
   cttcttgcgc agtgaaggcc tcagcctgaa agtgcagacg acctctggca acgacgccac 300
   cccatgttga agttagttca gcaacagctg tatagttcag cac 343
<210> 121
   <211> 322
   <212> DNA
   <213> Corynebacterium glutamicum R
<400> 121
   acttagaagc aggcattaac actgccacct ttgcaaaatt aaccaccccc tgatggggtg 60
   gttttttcat gagttgaaaa aagtgtcttg attcactttg tgatgacggt taccatagcc 120
   atcgtgacta aaaacattga ccttaagcga gtagccaagg ctacgtaccc tactgcggga 180
   tagatggact ggctccccgc actagggaag tagtcgttaa tcaacaccaa gaagcttaag 240
   agtcggtcct ccacctaaca ggatccttca cacccctctt ttcaatatct agaaaagacc 300
   gatcctcccc cacatctttt ta 322
<210> 122
   <211> 100
   <212> DNA
   <213> Corynebacterium glutamicum R
<400> 122
   tgccggatga cctgcgcgcc ctaacccaca gcaatgttga agcagggccg gcggaggaac 60
   gcgtcggcaa gctattcgcc ctttaaaaga aagcactgtc 100
<210> 123
   <211> 218
   <212> DNA
   <213> Corynebacterium glutamicum R
<400> 123
   tgaccccgcg agaaatattc attgaataga catcgtttta cctgcagata ctctcggtct 60
   ataaataatg aacagaactg tctattttta gaattgcttt ttgtgtaaac tcaagtcaca 120
   gaggctgctt caagtaaatg tttcgtaatt gtttacagcg tttactcgag cggacaacca 180
   acaaaaacag cacttcaacg attggagcac accccaca 218
<210> 124
   <211> 69
   <212> DNA
   <213> Corynebacterium glutamicum R
<400> 124
   attagtcaat tagttaaatg aggcggaagt gtagaacttc cgcctcattc cagacattaa 60
   ggagttcaa 69
<210> 125
   <211> 100
   <212> DNA
   <213> Corynebacterium glutamicum R
<400> 125
   ttttcctccg cactgcaccc cgccaacgcg gacgcaccag caatcaagcc ggcaccccct 60
   aggaaacccc tacgggaaac catgtcgctc cttatatata 100
<210> 126
   <211> 78
   <212> DNA
   <213> Corynebacterium glutamicum R
<400> 126
   tagctggtct ttacatttga cagaaacctc cgacaaaacc ccaatagttg acacggaaac 60
   caattcattc tagcttta 78
<210> 127
   <211> 100
   <212> DNA
   <213> Corynebacterium glutamicum R
<400> 127
   tcccggcgcg caccattgct gctggtgatg aggattatgg cgattcggga gctgaaggcc 60
   acgagggcca ctaaaccggc cactatatat aaggagcgac 100
<210> 128
   <211> 57
   <212> DNA
   <213> Corynebacterium glutamicum R
<400> 128
   ggcactataa tagacctagt atctatagat tgatagaaaa taatttagga agtttcc 57
<210> 129
   <211> 719
   <212> DNA
   <213> Corynebacterium glutamicum R
<400> 129
   gcgaaaaata ctacgcgcgt gttacccgat ttttagtaaa tccctagcca ctgacactgg 60
   attgttatag cccggaggtg tagagcaagg cagattagct ccctgggtgg gcggctccgg 120
   aggggagtct gcccggcttc gcccgctgtg gtgcaaacag ggcgggtttc agctcgggat 180
   ggtcgcttcg acgcgcgagc ttgccttgtt gtgcgcgcgg cgggtcaatc aaggcaggct 240
   tcggcctggg atagcccttc ccgtcgagga acccgccctg ttttgcccgg gcgggaatcc 300
   acgcgcgcga aaccgggcag attagcctcc cgaatggctg cattcgccga caagttcgcc 360
   ttgttttgca cggagcgggg acagccctct cgaattcagg cgggttagct tcacagacgg 420
   ccggtttcgc cgatgagtcc gccccgttgt gcgcgcggcg ggtcaatcaa ggcaggcttt 480
   ggcccgggac agcccctcct gctgaggaac tcgccctgtt ttacacacgc ggcgaacgaa 540
   acacggcgaa ctagcccgcc aaatggcacc cccggctggg aaattcgcct tgttttgcac 600
   caacagggaa cctggagcgc agtttcgggg tctaagtagg tatgggagaa aaacggcgga 660
   tgtggacggc gaaggaactg atcgggatgg ggtattcgcg gcggaggatc aacagtttg 719
<210> 130
   <211> 100
   <212> DNA
   <213> Corynebacterium glutamicum R
<400> 130
   gatacaactc cttgatggag tgaataaatt cccgcgcctg ctcctgatct tgcacacgcg 60
   tgatataggt caaaaatcgc gagcgcttga tctctagttc 100
<210> 131
   <211> 492
   <212> DNA
   <213> Corynebacterium glutamicum R
<400> 131
   gcggaaccgg ggatgtttcc cagccaggac tgaatcgttg gatgtttatg cgtgccgtag 60
   ttgtcacaga tgacatggac atcgaggtgc tgttgcaaac aggaatttct gcttactcgc 120
   tatcggttag cgtgcaccgc aactggtgcc ggatccgggg ttttcgtcaa aaacgcgagt 180
   ttgcaacagc accaaagaaa gtattgacgc cgccgtcaag gaagctcaac aggaccctgc 240
   ggccgcgagg cggctctttc gtttcctgac tcattatcag cgcgacgacg aaaaatatgt 300
   gcaagaggtc cgtgataact tcgaggaact tggtatcccc ctagaacact tgtcacataa 360
   gttattttca cgccctctgc gatacgtagt taaaataacg tgttaagtga caaacttttc 420
   cgagacgacc tcggacatca aattttcaga agcgtgtcac gtaaacgatc gtataagtga 480
   tggggaaaca cg 492
<210> 132
   <211> 100
   <212> DNA
   <213> Corynebacterium glutamicum R
<400> 132
   gatcagcgcg aatgaacgtc aagaacctat ccgactccgc aaagaaaacg ctgaactcaa 60
   actcgataac gagttgcttt aagaaaagca gcagccttgc 100
<210> 133
   <211> 100
   <212> DNA
   <213> Corynebacterium glutamicum R
<400> 133
   tctcggcgct aatctggttt gttggtgata tccgagccaa gggaactccg agctcaccca 60
   ttaccactga tccacaacac gaccatcttg agaggacagc 100
<210> 134
   <211> 187
   <212> DNA
   <213> Corynebacterium glutamicum R
<400> 134
   cggttggtga acaggacttc tgacacttgt cgtcttgtgc acgcgggtag tgaccagata 60
   gtggtcagaa ggcgcgtgca cagccctgct caccacatcg tgtttgtgca atcgccttct 120
   gaccctgaca acgatatttt ttcgatgcgt gtcacgtaaa tgatcgtata agtgatggga 180
   gaacact 187
<210> 135
   <211> 81
   <212> DNA
   <213> Corynebacterium glutamicum R
<400> 135
   gcaccgcaac gctgcggcac aacaacaccg caacgctgcg gtactaccgc aaaactgccc 60
   atttttactc aaaggagaac c 81
<210> 136
   <211> 100
   <212> DNA
   <213> Corynebacterium glutamicum R
<400> 136
   gaccgcatgc aacaccgcag cagattatat gagctcctgc gatacgaaga ctacaacgtc 60
   tttgaccagc acattttcac ctacagaaaa ggagaaaaca 100
<210> 137
   <211> 100
   <212> DNA
   <213> Corynebacterium glutamicum R
<400> 137
   gtttcggtca tgacagatga tccaacgcca caaagtggac tagcggtaga tccactttca 60
   gccacttgca ttagaccact tttttgagga cgatgaagcc 100
<210> 138
   <211> 59
   <212> DNA
   <213> Corynebacterium glutamicum R
<400> 138
   aataggaagt tagccgcgtt gaatcgcgga ttttatcgtt gtgaggagat ggaatcaat 59
<210> 139
   <211> 100
   <212> DNA
   <213> Corynebacterium glutamicum R
<400> 139
   ccactcgtcc tcgacatact tctcctggca ctaaacgcag gggttgacac atctgggtag 60
   actatcgaag tacattttgt gtcattgagg aggatcaacg 100
<210> 140
   <211> 100
   <212> DNA
   <213> Corynebacterium glutamicum R
<400> 140
   ggataagttt gtttctgagc gtattggtct tgatgatgtt gaagaggctt tcaacaccat     60
   gaaggctggc gacgtgctgc gttctgtggt ggagatctaa                          100
<210> 141
   <211> 278
   <212> DNA
   <213> Corynebacterium glutamicum R
<400> 141
   ttccactcct taaggtcggg aagtattaac tcccacctta cctgtggaaa cagcttatcg     60
   aagcaaaaac aaccccctct ttttcttccc agccccgcgt gttgtaatct agttcggtac    120
   gccttggtag ctcagtggat agagcaccgc tctcctaaag cgggtgtcgg aggttcgatt    180
   cctctccagg gcacaattaa cccccattga cctgcatcaa tgggggttca ttatttcctc    240
   caaagtgcaa aaatacacgc ttacgtacac tgtagatc                           278
<210> 142
   <211> 103
   <212> DNA
   <213> Corynebacterium glutamicum R
<400> 142
   ttttttacag cagtagggga ctatgctggg acgtccaagg aaaccgtccc ttttttgcca     60
   agaagtggct ggttcgatca tcccaaactt tcaaagagga ttt                     103
<210> 143
   <211> 75
   <212> DNA
   <213> Corynebacterium glutamicum R
<400> 143
   atgggttact cagcggttgt gtattcgagg cctgggtgta tgaagtgcag ggctacggag     60
   aaggcgttgg tgaag                                                      75
<210> 144
   <211> 460
   <212> DNA
   <213> Corynebacterium glutamicum R
<400> 144
   ggatcgtaac tgtaacgaat ggtcggtaca gttacaactc ttttgttggt gttttaggcc 60
   acggcgctgt gtggcgattt aaggcgtcgg aaatcgtagg ggactgtcag tgtgggtcgg 120
   gtcctttgag gcgcttagag gctattctgt gaggtcactt tttatggggt cggggtctaa 180
   atttggccag ttttcgaggc gaccagacag gcgtgcccac gatgtttaaa taggcgttcc 240
   gtgggcatct gtgtttggtt tcgacgggct gaaaccaaac cagactgccc agcaacgacg 300
   gaaatcccaa aagtgggcat ccctgtttgg taccgagtac ccacccgggc ctgaaactcc 360
   ctggcaggcg ggcgaagcgt ggcaacaact cgaattgaag agcacaattg aagtcgcacc 420
   aagttaggca acacaatagc cataacgttg aggagttcag 460
<210> 145
   <211> 57
   <212> DNA
   <213> Corynebacterium glutamicum R
<400> 145
   ctcaagtttc caggtaaact gggaacaaat tttagggaaa gggagttgaa cctaacg 57
<210> 146
   <211> 100
   <212> DNA
   <213> Corynebacterium glutamicum R
<400> 146
   cttggattcc aaccctgaag tacaactatg tccgtattgt tccgaatgaa atcactggcc 60
   gtgagttcac cctcggcgag gagcctgagc gctactagct 100
<210> 147
   <211> 100
   <212> DNA
   <213> Corynebacterium glutamicum R
<400> 147
   ggaactgctg gcaggcaacg tccgcgatgg cgatgctgtg cttgtcgacg tcgccgacgg 60
   cggtcagaag ctcgacgttt ccaaggcggt ctaacggctt 100
<210> 148
   <211> 100
   <212> DNA
   <213> Corynebacterium glutamicum R
<400> 148
   ttggcgacct attcacgaaa gccaagacat gatagcgttt gttattgact tgttattttc 60
   cagttttcag acaactactg cacttcggag gtgaaccacg 100
<210> 149
   <211> 100
   <212> DNA
   <213> Corynebacterium glutamicum R
<400> 149
   tgaaggcggc actatcaaag ccgtgctccg agttgataaa accaaccagt aacccacaac 60
   cactgaaccg actacgcatc aacgaaagca gctaagaaca 100
<210> 150
   <211> 61
   <212> DNA
   <213> Corynebacterium glutamicum R

<400> 150
   gtggcgcact cctatcacgt tccggtatca gtcttgcatc atttgtgtcg tttaaaagta 60
   t 61
<210> 151
   <211> 171
   <212> DNA
   <213> Corynebacterium glutamicum R
<400> 151
   gtctacatat cctagtcaat gatgcttcaa cgttctgcaa tgagatgaat gctcacttga 60
   tttctaggga agtacatata gaatcgttaa cgcccaagca acgaaaaact tggcgcaacc 120
   acttccattt cgaaggggag tagttccaca caaaggcgat ctgactcgtt t 171
<210> 152
   <211> 82
   <212> DNA
   <213> Corynebacterium glutamicum R
<400> 152
   aaaacgccag ggaattttcc gcgcccgctt ccttgtttga ataaacgagg atgcgggctt 60
   atttacaatt gcagttttac aa 82
<210> 153
   <211> 67
   <212> DNA
   <213> Corynebacterium glutamicum R
<400> 153
   tagaaaactc caagaaagtt aaaactgaaa tggtcgtgct agtggtgggc acagaattaa 60
   atcgctg 67
<210> 154
   <211> 73
   <212> DNA
   <213> Corynebacterium glutamicum R
<400> 154
   aagcgcacag gtttttgcat ggctagactt tgaatgagta agcacgaaca catttcaaag      60
   gaagggtttc acc                                                         73
<210> 155
   <211> 472
   <212> DNA
   <213> Corynebacterium glutamicum R
<400> 155
   attcccccta atcatttaca acacttatgg gctatgggtg gaagtggcat tatgcaactc      60
   tcttttggcc cagaagcttc taaacgagtg tttgataacc aatcagctac gtcacggttg     120
   ctggggtctg acactatccc cgccaggtat aaacactccg attcaacttc atccaaagat     180
   tttcaaatta attccatctt gcatagataa tgaacagtcg gtggcattgt gtgtaaagag     240
   ttttcatcca acccaaccta atttcggttc cgcgcgaggt aaatgtaatg cgggtggtat     300
   ctaaataaag ttgcattggg gctgggtgga ttgtggttga gggggcgtcg ataagcaaaa     360
   aagcttgcac cgggcatgta gcttgacggg gcgcctgcgc gctagatgcc tggaaaatag     420
   catctcttat acacctgaaa gagggcagcg ctaacaggaa atagtaataa tt             472
<210> 156
   <211> 100
   <212> DNA
   <213> Corynebacterium glutamicum R
<400> 156
   tttggtttgg aggggccgtc agatccattt gaggcgcaaa aactcattaa aagtgatgat      60
   agggagcacc tgctgaaaat ggcaggaatg taaaaaacaa                           100
<210> 157
   <211> 100
   <212> DNA
   <213> Corynebacterium glutamicum R
<400> 157
   aatcggattc atgctgtgtg gtgtgatcag tttgctggct gcggtcgcat ggatcttcgg      60
   ccgggagacg ctgccaacgg cgaaagtcga gcaggtataa                           100
<210> 158
   <211> 100
   <212> DNA
   <213> Corynebacterium glutamicum R
<400> 158
<210> 159
   <211> 216
   <212> DNA
   <213> Corynebacterium glutamicum R
<400> 159
<210> 160
   <211> 100
   <212> DNA
   <213> Corynebacterium glutamicum R
<400> 160
<210> 161
   <211> 100
   <212> DNA
   <213> Corynebacterium glutamicum R
<400> 161
<210> 162
   <211> 272
   <212> DNA
   <213> Corynebacterium glutamicum R
<400> 162
<210> 163
   <211> 382
   <212> DNA
   <213> Corynebacterium glutamicum R
<400> 163
   aaccctagtt ttaaagcagc agtcgaggca ccccctcgac tgctttcgca tgcccaattc 60
   tagccttaaa aaccatcaac taccagcata aatactaaaa attggccacg tttttaccta 120
   agggaattcc ctatatagca ccaccccaca gatgcataac atgacatatc acacacccta 180
   ataaatttta aggtgccgat caaaggagag tgccatgcta aagaagccct tgatgggtct 240
   ccctgcttta ctcacttttt cattgttatt aactgggtgc gctgcatcca caaacgacaa 300
   cactgacagc gctgaaagca acgtgacaat ttcggtcgct agtggattcg ctcggaacca 360
   ttcaaataat gatggattct gg 382
<210> 164
   <211> 100
   <212> DNA
   <213> Corynebacterium glutamicum R
<400> 164
   cgaaactcga aacacccaac ggctgctcgt cgaaaagcga atcttcgagc tagaagcaca 60
   agcacgttgg ctcgaccgaa ttgaagcatt ggacaaataa 100
<210> 165
   <211> 100
   <212> DNA
   <213> Corynebacterium glutamicum R
<400> 165
   acgccatcgc agcctgccct cccggccgca tcgaagtcct cgccaactac accgcattcc 60
   gagacctcaa gaaggctctg gagaaaggga ccgaacaata 100
<210> 166
   <211> 261
   <212> DNA
   <213> Corynebacterium glutamicum R
<400> 166
   ggggttttcg cctttccatg cttcatgggt taatttctac ggatttaatc taattaaata     60
   aatcccagga tccaagcaat ttggccttaa tatgatggca atcactaatt ggaaaaatgt    120
   ttaaacgggc attaaaaacg gattcaaccg ggtttaatgc cgttttatcg cgatttaata    180
   caggtctacc cccactaccc ccattcagtt cagggaatcc ccggatttaa aacaactaaa    240
   aaccctctag aatgagacat t                                              261
<210> 167
   <211> 398
   <212> DNA
   <213> Corynebacterium glutamicum R
<400> 167
   gttcatcttt ctcaacaatc tataaatatt cgcaacaact taggggcagt cggtgggaac     60
   ttacgcaaag tactttcaag cggtaccgac tcgaatccaa ttcttaaagc ttgcttaagg    120
   gaattgtaat ctaactctta ttatttggcg tcgctgtcca aaagcttaga aaaaggtgtt    180
   ttcattaaat tagattttct atccgtttag aatagcctgt cacaagcgct gaaccaatta    240
   agaggacagc gtgttggctg ccttaattaa atacacgtag aagtaccgat gtgattgaat    300
   tcgctaatct ttaagtggcc aaattcgccc cccatacagg gtttctttga aattgaagtt    360
   tatggggcaa atgaaaatat cctgggtagc atgggctt                           398
<210> 168
   <211> 52
   <212> DNA
   <213> Corynebacterium glutamicum R
<400> 168
   tcgaaataga agtctccccc ttttcaaatc ccctcctcgg aaagcaggaa cc 52
<210> 169
   <211> 246
   <212> DNA
   <213> Corynebacterium glutamicum R
<400> 169
   ttctcgagcc tatcaaggaa atcttatggg ggagatattt tccgaaagaa aatttaattg     60
   gtaagaaccc atgattgggg acttgcattt gtttcgccgt tcacttaagg ttaactctcg    120
   gttaacaaaa agagttctca aggtgaactc aagtgatcgg tggaacatcc actaacgggg    180
   ttgccaaaag ccttcgcgga ctctcccgaa ggaacacttc ttgcgagaag gaaaggaaga    240
   agcact                                                               246
<210> 170
   <211> 100
   <212> DNA
   <213> Corynebacterium glutamicum R
<400> 170
   ttcggtccgc tctggcaaaa atggctggct gccacctcgg cgcagcagct aaagggctgg 60
   gcttaaattg cttgtcgacg cctagtgcca caatggagac 100
<210> 171
   <211> 100
   <212> DNA
   <213> Corynebacterium glutamicum R
<400> 171
   atatgtctcc tgtgttggat gatgcggtgg ataatccgag gtatgcggag ttttatcagg 60
   cgatgcgcac ggaacggttt tgatcattta aggttcattc 100
<210> 172
   <211> 149
   <212> DNA
   <213> Corynebacterium glutamicum R
<400> 172
   gatggcagag aaatacgcac gtcattgata ctggccagtc aactgcggaa aggtgctgtt 60
   gcaaactcgc gtttctgatg aaaacgaccc agtcggccac ctgtgttgcg ctgcacgcta 120
   tcaaatagtt agtaaacaga aaatcctct 149
<210> 173
   <211> 51
   <212> DNA
   <213> Corynebacterium glutamicum R
<400> 173
   gccggaggtt ggcgtcgaaa agcaaaatgc ttttcgacgc ttccctatac t 51
<210> 174
   <211> 100
   <212> DNA
   <213> Corynebacterium glutamicum R
<400> 174
   tggggccttc tgcgccagtc tttacagcgt tttctgaagc ggctttgtcg ttgaaatcca 60
   tgatcccaaa ctacctcaaa gcgcttgtag gctaagactt 100
<210> 175
   <211> 91
   <212> DNA
   <213> Corynebacterium glutamicum R
<400> 175
   ctccatatcg agaacttaat cgagcaacac ccctgaatag tgaatcaaat cggaatttat 60
   ttattctgag ctggtcatca catctatact c 91
<210> 176
   <211> 100
   <212> DNA
   <213> Corynebacterium glutamicum R
<400> 176
   gagcatatgg tgcgcgcggc cgatatgctg atcaattcca accccgatcc gcacgcttaa 60
   cttctgccaa aaagtcgctt tggccataag ctaagcgatt 100
<210> 177
   <211> 100
   <212> DNA
   <213> Corynebacterium glutamicum R
<400> 177
   tggaggtgga cggcagccaa cggcagaaga cgttgctcaa gagatctgtc tggcagtagc 60
   tacctccatt aagaactttg tcgaccaggg taggccgttc 100
<210> 178
   <211> 100
   <212> DNA
   <213> Corynebacterium glutamicum R
<400> 178
   ggcagaaccg tgtactgctc aatcacattg tgaccaagct cggtgatgat ccacaggcga 60
   tcgtcgatgc cgctgtcaag gctgatcgtg aggggaaata 100
<210> 179
   <211> 100
   <212> DNA
   <213> Corynebacterium glutamicum R
<400> 179
   ccttcgatcc ttaaagccga ggaaacaaga gcaggtagaa tctgaaacgg ttgtcgagca 60
   cacgtatcaa acatcggcag cgaattaaga aggtgaacag 100
<210> 180
   <211> 100
   <212> DNA
   <213> Corynebacterium glutamicum R
<400> 180
   gccaactcgc cggaggtggt gtcgcggaca tcatctaaaa tttcgtgcag gtactcgggt 60
   atcggcatcg tcaacataga atcaagacta gtgatttctt 100
<210> 181
   <211> 96
   <212> DNA
   <213> Corynebacterium glutamicum R
<400> 181
   gcaatgactc cgaaaccttc aagaacgtgt ggcactaaca attgcggact atccttggga 60
   actgttttag attttattca gggtagggag attgtt 96
<210> 182
   <211> 100
   <212> DNA
   <213> Corynebacterium glutamicum R
<400> 182
   tgcgcaccta cggcgccgaa ttcccgctgg tcctgcttaa agatggacag gcactgctta 60
   tcgacgacca cggcgtccac ctaatttagg atggttcccc 100
<210> 183
   <211> 88
   <212> DNA
   <213> Corynebacterium glutamicum R
<400> 183
   atagtggaaa atcagcggtg ctacgattgg aattagcttt tgaatgtcag catattggac 60
   gcacggtgga aaacttcgag gagtaatc 88
<210> 184
   <211> 144
   <212> DNA
   <213> Corynebacterium glutamicum R
<400> 184
   tgttctcctt taaatcgaaa ttattgcatc atgcaatttt gttatgaaca caaactacca 60
   tgtttattgc atgatgcaac acccctgcta ggatataaat tactctatga gtccaaacgt 120
   ttttaaaggg agcgaattac cata 144
<210> 185
   <211> 122
   <212> DNA
   <213> Corynebacterium glutamicum R
<400> 185
   accttaaatt catctcctac aaccttttgt aggtaagaat ttaacaagag ccagttatct 60
   tctcttaaaa tgaggaggta actggcttct ttatgcttaa gaggtgttag cataagtgaa 120
   ac 122
<210> 186
   <211> 131
   <212> DNA
   <213> Corynebacterium glutamicum R
<400> 186
   cagaccttat ccccgagggg tgggcaggca ccctcatcct cgtcatggtc accctaattt 60
   taggcccatg ggcgatcttc tcaaaacaaa acgcagaaaa atggtgggca ctcggacact 120
   tcaccaaatg g 131
<210> 187
   <211> 100
   <212> DNA
   <213> Corynebacterium glutamicum R
<400> 187
   agaaggtggt gcccaccagt ccgttagcgc agtggtgggc aatgaggtgg gagtggttta 60
   aacgcaccgg cctgcaggtg accggggcgg tgttggaggg 100
<210> 188
   <211> 321
   <212> DNA
   <213> Corynebacterium glutamicum R
<400> 188
   ttttcgaagc tcgggataaa tctgcgaatc ggaaccactc cacacaaagc ccaccgaagc 60
   cccaaatcgc tgggaggcgt catacccagt caatggtccg gaacttagta gcgcaagtaa 120
   tgcagatcgt agagtcacgt actcattgtc ccatatctac ccccgccccg gagcatgaat 180
   acccgcagct cagaggccct aggtgacatt atctcacatt taccccttga cagtgatttg 240
   aaacacaagc aaaatatgac ccacgccata aactaactaa gagtttaggt atttgattac 300
   atagttcttt aggagttcac c 321
<210> 189
   <211> 100
   <212> DNA
   <213> Corynebacterium glutamicum R
<400> 189
   ctccgatgaa ctccctggta aaagagctag gagaaccgta gtgattgtga ggcatgtgag 60
   gtaattttgc cccgctgtct tctgaccaga atgatgttag 100
<210> 190
   <211> 58
   <212> DNA
   <213> Corynebacterium glutamicum R
<400> 190
   gggtatacga tgggagggga aaccccgcct gatcccccga tcccacagga gcgatccg 58
<210> 191
   <211> 100
   <212> DNA
   <213> Corynebacterium glutamicum R
<400> 191
   cttcggtggt cagtgcttgg tgcaccttgc cgacgggctg attgatcgta atggtgtttt 60
   ctgtacgcgt tgccatgagg ataagactac cgttagtgag 100
<210> 192
   <211> 170
   <212> DNA
   <213> Corynebacterium glutamicum R
<400> 192
   gtgctcacac tagcgccgta ggccggcttg cgctcggcaa gtgttttgct tatcgatgtc 60
   tccccacata acaattccaa ctcgaagcac caacgattca agccttatca gtttcgtaca 120
   ggaaaatagt gcaaaaatgg ggtttaagct tcgtggagag gattgtcatc 170
<210> 193
   <211> 228
   <212> DNA
   <213> Corynebacterium glutamicum R
<400> 193
   tttacttacg acgctgcgta gttaaaaagc gtgactcccc acattctagg gagtcacgct 60
   tttttgattc tgcccgcatt cttggacgtg tgatgtacgg caggtgaatt ccgctttgct 120
   aggctgaaca ctaggcacgg ggtgccaacc ggatggaaaa attccggggg ctgagaaaac 180
   acccgttgaa cctgctctag ctcgtactag cgaagggatg gccttaac 228
<210> 194
   <211> 100
   <212> DNA
   <213> Corynebacterium glutamicum R
<400> 194
   tcattgtcta cgccaccctc ggtctgctgt ctgaagcgct gatcagagct tgggaacgtc 60
   acaccttccg ctaccgaaac gcataagaaa gttgctcgcc 100
<210> 195
   <211> 149
   <212> DNA
   <213> Corynebacterium glutamicum R
<400> 195
   agggcactct aaaaccggaa gagccattaa aacccctgtg tccactttcc ggggtcaggc 60
   ccaaaagaat actttaaaag gataagattt atatgacagc ctgattcaca ttgtaaaagc 120
   cctattctcc gtgaaggatg acgattgct 149
<210> 196
   <211> 105
   <212> DNA
   <213> Corynebacterium glutamicum R
<400> 196
   ggcggcagat tacctttccc agcatcatgc tggtttcagg gtgtccacca aacaggggtc 60
   aggtccctgg atcaagtttc aattgattct ggggttgtaa gtgca 105
<210> 197
   <211> 216
   <212> DNA
   <213> Corynebacterium glutamicum R
<400> 197
   gtgaacctcc aagagagtga aaagaagttg gtattgccac cgcgatgtcg ttttcgtttt 60
   tgctttgaag cggtgtgtga catttgcgag caattccgca gtgtcggtgg cgtatgacca 120
   acagtgcccg atcaatgaag tgaatcacat taggggaatt cctagggttt gctcgggggt 180
   aggtgttcgc atgatgtaaa ttgacaggct gtttgt 216
<210> 198
   <211> 84
   <212> DNA
   <213> Corynebacterium glutamicum R
<400> 198
   gcccacattc tagatcgccg aagaaagcag cgggacgtct ctatatacta aagggcacta 60
   aagcaacgca gttgaaggga cacc 84
<210> 199
   <211> 130
   <212> DNA
   <213> Corynebacterium glutamicum R
<400> 199
   gggtttgcgt cgaaaagcaa gcacgcctgg tgcctgattt gagcggtttt acctatggcg 60
   ctttggcgcc gtcaaactgt cccagcgatt tcattattat tttcgtgcat tcaccgttat 120
   agttataggc 130
<210> 200
   <211> 100
   <212> DNA
   <213> Corynebacterium glutamicum R
<400> 200
   gcggtgtggc ccggtgctgc gatcgctttg acggtccttg gttttaatct tttcggtgat 60
   ggtttacgcg atgccatcga tccaaagcgg gaggtcggcc 100
<210> 201
   <211> 533
   <212> DNA
   <213> Corynebacterium glutamicum R
<400> 201
   ggttgcccat gataatcggt tctgttgtac ctttcagtgc tccaactgct tgatttacca 60
   tcaactattt aacctccact ctaaatttat cgcccgaggt tacttgatta tatttgtaga 120
   aactacttag gcttcctcct tggctgttgt tttctactcg ggggacccac tggcctctat 180
   tacttgagcc accttcctca gaagaagcaa acgggattca tcgcatgaag ggcttggcat 240
   tccaccacgt cgccaacttt catatgcaag acgtgttgca gctttttata tcaatttagt 300
   ggttgatgaa gtctctaagt ggccttttac ttttaatatc caggttgcaa aatcaataat 360
   tgcccatcag gttttgatgc ttcaacccag cttaagacct gtggctgcca ggaatggctc 420
   ctaaaaccaa tgcctgagcc tttccctgag ctgtaatgaa aggacattgg tatggggttg 480
   cctctgacat cgtttgagct ctccatcctt ttcatttcgc tattctggta tcc 533
<210> 202
   <211> 100
   <212> DNA
   <213> Corynebacterium glutamicum R
<400> 202
   tggtccgcca ccaactggtt ggacacattg tggatgcata cgaagactat gaagaacgcg 60
   aggcccgcga attgaaacgc aaacgccagg agacacggcc 100
<210> 203
   <211> 228
   <212> DNA
   <213> Corynebacterium glutamicum R
<400> 203
   caggtcagct aacctttcac aagacgaatc ccgcgcgtaa gacctcactc gcgggacagg 60
   tacgcccaca agcatagacc gccgataccc atatataaaa gtatgggttc acttccatcg 120
   tttcgcgcgc atgagtttac tcacgtgccc acgtctttta gccacccatt gaagtgaaaa 180
   aataaccccg atcacactaa tggagtagct aaggtgcaca atggattc 228
<210> 204
   <211> 100
   <212> DNA
   <213> Corynebacterium glutamicum R
<400> 204
   atggatcgat atttaagacg ccagcgcggg tggcgtcgat gccgaattga atcgcagaat 60
   ccgggcggat cagaacttgg gcagagttgg ctaaagcgat 100
<210> 205
   <211> 153
   <212> DNA
   <213> Corynebacterium glutamicum R
<400> 205
   acatacagtc cccgtgatgt gaccatacac accacgggga ctgtggcgta ggtcttacaa 60
   aattctccaa aaggagttat gatagtacca atacgttttt gtggcagcct cctgcattcg 120
   gcagtcgaga cgccaccaaa gaaagggtaa gac 153
<210> 206
   <211> 100
   <212> DNA
   <213> Corynebacterium glutamicum R
<400> 206
   gttgcgggag tgtccaccaa aaatattgcc tgcatagggg tctgaaaaac tcatagctca 60
   ccaccctagt caaagcactg cactacactt ccctaacact 100
<210> 207
   <211> 100
   <212> DNA
   <213> Corynebacterium glutamicum R
<400> 207
   caggttggaa ccctgactgg ttcatgttct tcctcggcgg caccctactt ctggctgttt 60
   tgctcaatca ccgattcgag cgtttcaaca aggagcgatc 100
<210> 208
   <211> 100
   <212> DNA
   <213> Corynebacterium glutamicum R
<400> 208
   caccaccgac tccgacgact tcgacgccga ctcctttacc accgaagtca tccggattac 60
   cggctactcc cgccacgaag tcaacaacgg ccttaacgcc 100
<210> 209
   <211> 280
   <212> DNA
   <213> Corynebacterium glutamicum R
<400> 209
   ccaccgcaac caagcgtcga aaagcaaaat cttttcggcg ctttttggtg actcgtcaac 60
   aagggggagc aaaatcagcc attgccagga aaaggttgac ctctatcggg gttagccttt 120
   ctaaagttaa gctgtgagcg ggaacttgag aatcaacttc aacgacaacc tttaagaagc 180
   tcttattggt tcttcgtttt gtatcgataa atgcaatcga tttcctggct taataaggct 240
   gttcctgtaa acctgcaatg gaagaggaag gggacctagc 280
<210> 210
   <211> 100
   <212> DNA
   <213> Corynebacterium glutamicum R
<400> 210
   ggtcgcagaa gcactacatg gcatggtgcc gggcctcaaa aacacaggta gctcggtcaa 60
   cgacgattct cgtcggaacg tggaaggaca gtagaaaaca 100
<210> 211
   <211> 269
   <212> DNA
   <213> Corynebacterium glutamicum R
<400> 211
   tgtccgacaa gatctggaaa gtcggcatta tcggttgcgg tgcaatcagc cgaaaccaca 60
   tcgaagcagt tcaagcaatc cccggcgcgg aagtcagcgc agtctgtgat gtggatggtg 120
   tgaaagcatc ggaaaccgca gcgaaatatg gaatttcttc cagcttcacg tctgtcgatg 180
   agatcctcgc ctccggggtg gacatcgtcg cagtatgcac cccacatcca acccacgaaa 240
   cagtggtcct cgccgctgct gccgccgga 269
<210> 212
   <211> 92
   <212> DNA
   <213> Corynebacterium glutamicum R
<400> 212
   ctaagtatga acgcgaatcc gactttggtc gtactgcaag aatcgaccag agcccgatta      60
   aaaaatgccc ccgcgcaacg aaactaataa tc                                    92
<210> 213
   <211> 165
   <212> DNA
   <213> Corynebacterium glutamicum R
<400> 213
   ctaatttcac caaactcgtg agcgaggtac cgttccaaag ccatcatgag atcattttct      60
   cctagcctga tccccatgac tttttcttct ccatcgaatt tgtggtatgg gcgagacagt     120
   gccatatgag aagaccaagg ggatgaagaa gataaccccc caatc                     165
<210> 214
   <211> 100
   <212> DNA
   <213> Corynebacterium glutamicum R
<400> 214
   tcgcggacat catctaaaat ttcgtgcagg tactcgggta tcggcatcgt caacatagaa      60
   tcaagactag tgatttcttt tgcgctatat actcttgtgc                           100
<210> 215
   <211> 100
   <212> DNA
   <213> Corynebacterium glutamicum R

<400> 215
   acatggagct catccgcagc ggtccaccag cagaaatggt gggcatcggc acgcctctac      60
   cgttccccac ctcacaaccg gacattcact aggacacact                           100
<210> 216
   <211> 91
   <212> DNA
   <213> Corynebacterium glutamicum R
<400> 216
   ggaacatcct tggcggagtt atgcaaacgt tttcataaag gggttattcc attctacgtc      60
   gatctttgta gaaggtggtt attatggctt c                                     91
<210> 217
   <211> 100
   <212> DNA
   <213> Corynebacterium glutamicum R
<400> 217
   acgcgttgct ggacgtcacg ctcgcggtgg atgagcacgc tccctgtctc gactcgggct 60
   gtgacgtgcc cggttgtgtc accgggcagg agggcatgtg 100
<210> 218
   <211> 100
   <212> DNA
   <213> Corynebacterium glutamicum R
<400> 218
   gccggtgcag gcacgtgggc tggggcgaaa gacgccggcg cgctgctgaa aattttcgca 60
   accatctcca cattccacta ctaaaggttt aaataggatc 100
<210> 219
   <211> 137
   <212> DNA
   <213> Corynebacterium glutamicum R
<400> 219
   ttagagattt acttgcttga accgccttcc catctttgaa ttcattcaag gtggtaaggc 60
   ggtttttgct ctttaaatac agttttaaag gtagatttgg gagagaagat ttcccttaag 120
   aaaggttttt atcaacc 137
<210> 220
   <211> 100
   <212> DNA
   <213> Corynebacterium glutamicum R
<400> 220
   ccgagaagct ggagaaggcc aacaagcgtg gcctctacac ctccgcgtcc ttccacagcc 60
   ccggcgccat cactggcgac cactaaaaga ggagacttcg 100
<210> 221
   <211> 464
   <212> DNA
   <213> Corynebacterium glutamicum R
<400> 221
   taggtgcgct cttcccttct acaaattttt ttgcttttcg acgccacaat gcagcgcgat      60
   ttcagatcgc cagcttaccc caaccgaagc ccttcagaag acccgcccca gcctatcaat     120
   tttggtcaag tcgcctagct ttagacctag ctttagagcg caatccccca ccccaaaacg     180
   tgatcttagt cacttatttt ccattacgcc ccacagggtt cagtgtcgga ctaacgccac     240
   aagaccacct ccatcagccc tcaaattcaa ccatttattg ccctaaatta tcgccactag     300
   cacgtcattt agagaaaacc tttaatgtga aacaggtctc aaactgggcc attctcaagc     360
   cttcaaaaag gtgggaaact tagccaatcc aaagcccaaa aatgcgggtt atgctgcgct     420
   aacctatgct gacagccttg cggaatttgt gtacgttagg ggcc                      464
<210> 222
   <211> 188
   <212> DNA
   <213> Corynebacterium glutamicum R
<400> 222
   cgccgcggcg cccggaaagc cctcagcggc ggggaaaccc gattcgatgg aggcctggaa      60
   ttcatcaaaa agcccgggcc cagagggctc gggcttcccc ggcgcaacgt cggttgcggt     120
   gttaagttct ggacatgcag aagcctttcg aggatctgtc ccgggagttt gccgcgtcca     180
   tcgccgag                                                              188
<210> 223
   <211> 97
   <212> DNA
   <213> Corynebacterium glutamicum R
<400> 223
   cggcggcaac aaagccgtag cggttcacgg caccgcccca gagtgttggc tgctggtcga      60
   atggcccgcc ggagaagacg aacccaccga ttactgg                               97
<210> 224
   <211> 100
   <212> DNA
   <213> Corynebacterium glutamicum R
<400> 224
   agcgtcgcag gaatcggcgc gatcttcctc cttccgatcg gcatcatcat gtgcctgatc      60
   gccggattca accgcttcta cgcagccctt aaggtctaaa                          100
<210> 225
   <211> 110
   <212> DNA
   <213> Corynebacterium glutamicum R
<400> 225
   tatttttgtc ttgagttcgc ggttttgcac tctttccgac attttttcta ccctttccac 60
   acttatgaat cacatcactt ttagtgtggt gtatgacata agctaaagcc 110
<210> 226
   <211> 72
   <212> DNA
   <213> Corynebacterium glutamicum R
<400> 226
   agaaaccttt aaggactagc tcgaaaaaca gccaactata gttaagtaat actgaactat 60
   tttggaggtg tc 72
<210> 227
   <211> 100
   <212> DNA
   <213> Corynebacterium glutamicum R
<400> 227
   acgggaagga ggaacacagt gggtcgatat cccccgtaac cgttactgac tgaccttgtc 60
   tctcgacccc taactcacac cacctttgaa aggaacgctc 100
<210> 228
   <211> 350
   <212> DNA
   <213> Corynebacterium glutamicum R
<400> 228
   ttctcgggga aaaggaataa aatggcttgt ggtcagactc acaggggctt ctccaagtca 60
   gtggatttat gaggtcccag tgggtacaca ccgggtgtcc tacaacgatc aattgtcaca 120
   gattcgactg gcatgctgta ccatctgctt taagcatttt ggtgtttcac tgttgttaac 180
   agtgtttcac cgtggagcac tacctaaaga tcatagtcag catcttgggg tgaatgtgac 240
   acggtacgct atagtgtcag acaacaacca ggaaactggt cgttgcagag tttttgcaaa 300
   attggacatc ctttaacgga ccgcacagag aggcggggaa ggaggtcacg 350
<210> 229
   <211> 60
   <212> DNA
   <213> Corynebacterium glutamicum R
<400> 229
   ggcttctgag attagtcggt gtgatccggg aaactaaatg gaaaactaaa atgaaaggca 60
<210> 230
   <211> 138
   <212> DNA
   <213> Corynebacterium glutamicum R
<400> 230
   gtgagagaag cctccacaga tagaaatcac aaataaataa cagacccact ctagccgttg 60
   cgggttctta ttgaggtcta tagtggggtg ctacacacta caaaccgtga tttgtttcgt 120
   gattggagct gagcccac 138
<210> 231
   <211> 117
   <212> DNA
   <213> Corynebacterium glutamicum R
<400> 231
   actttaagga gttcaccaag gaggaaaatt atcaccgtct taaaaacggc taaggctttt 60
   ctaaattcgc cgctgcgcac catcacgtgg cgcagcgttg tggcgaataa actccgc 117
<210> 232
   <211> 100
   <212> DNA
   <213> Corynebacterium glutamicum R
<400> 232
   gcaagggcaa cgggaatact acagtattta acggcaacgg gattcgagtt attgtcgata 60
   atgtgagcgg aaatgtgatt actgttacga aaggctaatt 100
<210> 233
   <211> 133
   <212> DNA
   <213> Corynebacterium glutamicum R
<400> 233
   aaaatagccc caccaaaaag gcggggcatg cctccacaaa gcgcggagaa gaaaaatcaa 60
   cggtccaaaa cagtgaaata cccccgctag tgcgaggaat gaacacacta tagcacgaaa 120
   agagaagaag tat 133
<210> 234
   <211> 127
   <212> DNA
   <213> Corynebacterium glutamicum R
<400> 234
   acccactgct tgaaacactt aaagcaccgg cagccttcat ggttaccggt gctttcctgc 60
   tcttttacaa agcctccacc tatgattggc taagatcctc ttccccgcac tgaaagctca 120
   acgatcc 127
<210> 235
   <211> 162
   <212> DNA
   <213> Corynebacterium glutamicum R
<400> 235
   acccctaacc catattagaa caaggatttg tgcgcttttc ctgttctggt gtgggttttc      60
   ctcacatcta acaatcgaat aactgttcga ataaaaggtt gaaggtgtcc cacccccacg     120
   gcacaatgga tggcaagaac acatgaatcc agggggatac tc                       162
<210> 236
   <211> 84
   <212> DNA
   <213> Corynebacterium glutamicum R
<400> 236
   ggtgtccctt caactgcgtt gctttagtgc cctttagtat atagagacgt cccgctgctt      60
   tcttcggcga tctagaatgt gggc                                             84
<210> 237
   <211> 292
   <212> DNA
   <213> Corynebacterium glutamicum R
<400> 237
   gttttagata acaagaccag cacagaccac catatctacg accccaaaaa cagactccaa      60
   gctccgcggc gacgaagccg cgctcgcgcc accgaccaag cagccggtcc aggtttaaag     120
   attttgcttt tcgacgctcc cctccacctc attcaatgcg gcggagggga ctttcttacg    180
   tgttcagtat ataggaaaaa gcgtttgaat agcacccttg cgttcgaaag tgtaatcgag     240
   tatagtggtt ggtattagca cggggaacta aacgggaaag ggggaagaca cc             292
<210> 238
   <211> 100
   <212> DNA
   <213> Corynebacterium glutamicum R
<400> 238
   actacctcga cccatcacgc acactcgaga tggagcgtta cgacgccaac cgcgatgaca      60
   tcaaccaccg cgtagacgcc aatggaacgg aggaacaacc                           100
<210> 239
   <211> 100
   <212> DNA
   <213> Corynebacterium glutamicum R
<400> 239
   accttcccac cagctacgcc gtcccagtag gactgaccac cggagtgatc ggcggcctct 60
   acctgctgtg gatcctcacc cgacaaaagg ctgtttaact 100
<210> 240
   <211> 100
   <212> DNA
   <213> Corynebacterium glutamicum R
<400> 240
   tcttgcatgc cgtgcaaagt ctgcctgacc tggatgatct tgatcagctc aacatcgaag 60
   tcgacataag caaccaggcc gcgacgaaag cggggctgtt 100
<210> 241
   <211> 231
   <212> DNA
   <213> Corynebacterium glutamicum R
<400> 241
   aggacctgct ttcttgaatg gaagacccat ccagattgga agtatgatgt cagatgtcta 60
   attgggggca tagacatgcc tcccccactt cacccaccag gacgggttgt gtgcgggatt 120
   ttcttatcat acaaaactta tgacacattg ttgtgtgaat cacaaagttt tttaaatttc 180
   gaaaaaccac aagcttgtgc gggaaacttc tcgcataact agggtgaggg t 231
<210> 242
   <211> 68
   <212> DNA
   <213> Corynebacterium glutamicum R
<400> 242
   gttgttgatg catcaactta tttcttggct atacttgagg ccacgtttcc tcagctcagg 60
   agaagtcc 68
<210> 243
   <211> 186
   <212> DNA
   <213> Corynebacterium glutamicum R
<400> 243
   caacttctgg atgtcttctt ggttgctcat gacccacagt gtaaatgatt aatcgatcac 60
   taaaaatgat catttaaacc cgactctatt gggatgcccg attctctgca cggtctccga 120
   tttagaatat aaaggcctta taggctgtat ttttacggag aacgtggggt tgacatccgc 180
   tggaaa 186
<210> 244
   <211> 142
   <212> DNA
   <213> Corynebacterium glutamicum R
<400> 244
   ttcaaggaca tatgaagctg tcgaacatgt gagtgctcga cagcttttcc atttcgaaaa 60
   atagccttgt attcgaaaat gtgatcgggt aaggtggttg gtattagcac ggggaactaa 120
   acgggaaagg ggggaagata cc 142
<210> 245
   <211> 647
   <212> DNA
   <213> Corynebacterium glutamicum R
<400> 245
   gtttttggga gttgtgggcc ggtgttggag gggtggttgt ttaataggcg acttcacgta 60
   tgcggacttc atgtgcgctg acttcttgcg ctcaacttct catgtgctga cttcactccg 120
   gattgctggc atttagtagt gatgtcgtgt gatgagaagt tgagtatgcg aagtcggtac 180
   cgagaagtcg acctttagga catttttaga ggagtccacg cagagactct gtctccgtaa 240
   gttcgatttc acggttacga tttcgaactg tcaatatccc gggtgcggtt tctcaattac 300
   aatttcgcac ttccaatttc tcaagagcga taccactagg aaaagtgcca ttttccgact 360
   gaaatcgccg ttgagaaatt gagcgcaagg tatcgagttt gcgaaatcgg taatgcgaaa 420
   tcgtaaccgt gaaatcgaac tttaaaccca aatgaccgaa aaagctcaaa ccaacaagca 480
   tcaagtaaca cattctcgtg cggacagcgc acggaatcca tagactggtc tgcagaagtt 540
   ttacgtgtgg gtttgcttcg gatggggctt taacaagctt cacagatgtt ggtttttcat 600
   aggcaagcta tccagaagca ggctttacag aaagtcaggg tgtggca 647
<210> 246
   <211> 104
   <212> DNA
   <213> Corynebacterium glutamicum R
<400> 246
   tggaagtttt tcaaagtgtc tgacgttgaa aacggtgagt tcacaactag ggtgaatggt 60
   gcacgtgatg ctgcactttt acgtttacta ctttgaggga aaca 104
<210> 247
   <211> 204
   <212> DNA
   <213> Corynebacterium glutamicum R
<400> 247
   cgcactcctg accccacaag cccgcccgca ccagaaagtg ccggcgggcc atctgtgccc 60
   cgggctacga gcaccacagt ctttttgcga tccagatctc actttgcggg tacttgatgc 120
   ggatcaaggt actttctccg gattgttcat aacttaatga gtatcacaga aaacacctaa 180
   tcttcatcat gaaaggacac cacc 204
<210> 248
   <211> 100
   <212> DNA
   <213> Corynebacterium glutamicum R
<400> 248
   tgtcccacac cgccgagaat ttaggaaacc gcgctctgac cggcctcgct gaaatcgaag 60
   acaccgacga ccaactcgca cacgcattgg agcgcctgac 100
<210> 249
   <211> 100
   <212> DNA
   <213> Corynebacterium glutamicum R
<400> 249
   tccgcgcctg tcgtagcact gcctacctcg gtgggttatg gcgcaggtgc tggaggaatc 60
   gcaccacttc tgaccatgct caacgcctgc gcgccgggag 100
<210> 250
   <211> 126
   <212> DNA
   <213> Corynebacterium glutamicum R
<400> 250
   ttaagcgtcg tcatccatgc ttagaggaat atgtgaataa aaagggttat cttatgctca 60
   tggttatgac ttgcgtgcga cgatatgtgt aatgcaatcg atatctaata aatcaggaaa 120
   aaggac 126
<210> 251
   <211> 1000
   <212> DNA
   <213> Corynebacterium glutamicum R
<220>
   <221> misc_feature
   <222> (405)..(905)
   <223> n
<400> 251
   gagatttttc atttatttga tccatcatga ccattaccgt ctggtcattg aaatctgcgt     60
   gccttgcaat acgatttaga gtctctcgca tagacacttg ctcgcgctct ttaaactcat    120
   tggggccgca attagtttcc tttggagtcc caaccggctt ttcttccgaa taatagaaga    180
   gatttccctc aagctgtcga agcttagata agagggcacc tagcaatcga agattttggg    240
   gacgctcttc atagaccttt gcatatagaa gatcagaacc cttcttttcc caacgccctg    300
   gatcgcggtc tgacggaatg tcgctcctaa aaaatctttc ttccttgcag caaagaaaga    360
   accaaacacg cgggcgtttc ttcggggatt ataaatcctc catanacaaa ggttgggcta    420
   tcggagaact tttatgagac gggtgaacaa atgctccggt cgaaccaatc tcatcgatat    480
   atgcaagaag caagcaagtc tcccccaaaa cgtttaggaa taagcacgag ggcctgcaaa    540
   tgcgaggccc tcggagttgc tgcccggggt attcttgcat gttactgctc aacgcctggc    600
   ttacacctcg aagtgtacaa ggagaagaaa cttcacgcaa tcaaccaatt tacccccaag    660
   ttttccccac aaatgaagaa tcttgaactg agatgctcac aatttattcg ctaatctgca    720
   caaatgctag taacgcgatg ttgagtttct ttctcaatct gactccaccg ggggtaacat   780
   cttcgaggcg gaccgtttga aactgtattt gtaagggttg gcgcagcctc cttccacttt    840
   ggcactcatt gatactgcat cgtcaaaagc gcaaaccgcc cccaccacca taaaggtgat    900
   gggggcattt ccaggggtag cactttccaa actgccttat ggacctcggc ccggtgttaa    960
   atttcgagta tttagatcac cttctactca tcgaggcgcc                          1000
<210> 252
   <211> 142
   <212> DNA
   <213> Corynebacterium glutamicum R
<400> 252
   ataagacacc ctagtggtgg tggagtgatt tttgggtatc gacgacaggt gaattcatgc     60
   acgtttgagt gtcccgtgtg tggggtaatg ttgtccaaga gagtgcaagg aaatgctgtg    120
   gcggttgaaa ggagtgcctt tc                                             142
<210> 253
   <211> 100
   <212> DNA
   <213> Corynebacterium glutamicum R
<400> 253
   gccattgcag ggaattttct ttgtctgcga tgaatccgtt gagcgtgctc gatgtgttgt 60
   agatgatcgc catcacacga tgctacctac gatggggatc 100
<210> 254
   <211> 100
   <212> DNA
   <213> Corynebacterium glutamicum R
<400> 254
   aacagaagcc acttcccgta cctgcgatta ttccgttttt gtctatcgca gccttggtgg 60
   cctctgcggc tgtcttggtt ctgattattg ttcagtagct 100
<210> 255
   <211> 100
   <212> DNA
   <213> Corynebacterium glutamicum R
<400> 255
   cggtgtacga catgatcaag gctgtggaca agatggccgt gattgatggc attcgtgtgc 60
   tgtcgaaaac tggcggtaaa tctggggatt ggtccgtgca 100
<210> 256
   <211> 178
   <212> DNA
   <213> Corynebacterium glutamicum R
<400> 256
   aatgtgacta atcacaccct cagatttcaa cttgctgggg gtgtttttgg cgtgttagca 60
   gattttagct tgactctggt ctagggtaga gaccagactt atagctaagt tgcatgctta 120
   gaattaacac gcatgttgtt ttgaataaaa catgggtgct gaataatcgg agaaacta 178
<210> 257
   <211> 100
   <212> DNA
   <213> Corynebacterium glutamicum R
<400> 257
   agcgaatcgg attcagtgat ttgcttgcgg gcgcagaaat gatttttcaa ttagacacac 60
   ttagacacac gtaactaaaa cctcagggaa gtgactgata 100
<210> 258
   <211> 100
   <212> DNA
   <213> Corynebacterium glutamicum R
<400> 258
   gcgccaggca gctttgcggt ggcgtttatt gatgcgcttt atgacgtgga tgcccaggct 60
   gtggcctcgt tggttgatgt gcgagaggcc tgaaaagtac 100
<210> 259
   <211> 100
   <212> DNA
   <213> Corynebacterium glutamicum R
<400> 259
   acgcgttgct ggatatcacc ctggccgtcg atgacaacgc cgaatgcatc gacgccggat 60
   gcgccgtacc tgggtgtgtc actggacagg agagtgcgta 100
<210> 260
   <211> 156
   <212> DNA
   <213> Corynebacterium glutamicum R
<400> 260
   gaggttttcg ctttcctata caggttatat aagagcttaa aacaagactc ccgacatatt 60
   agacctgtac gggttgaagc gaaacacttc ccttagagtc aaagtgactg taagatagct 120
   caacagttaa agtcagattg accctaaggt ggtttc 156
<210> 261
   <211> 106
   <212> DNA
   <213> Corynebacterium glutamicum R
<400> 261
   aataaataga tacagaacac attccattac cgagaattat caaatctcat acattagtct 60
   cgtaggggca atctcatttt ccatgcagta tttttaagga gtccct 106
<210> 262
   <211> 208
   <212> DNA
   <213> Corynebacterium glutamicum R
<400> 262
   acctttctgt aaaaagcccc gcttcttcct catggaggag gcggggcttt ttgggccaat 60
   atgggagatg ggggagttgg atttggtctg attcgacact tttaaggact gagatttgaa 120
   gatggagacc aaggctcaaa gggaatccat gccgtcttgg tttaatgctg caccctgcta 180
   atgaaaatca ttactattag gtgtcatg 208
<210> 263
   <211> 61
   <212> DNA
   <213> Corynebacterium glutamicum R
<400> 263
   agtcgaagca gcgctaaacc cctcacagag attgttctgt gaggggtttt agtttttact 60
   g 61
<210> 264
   <211> 100
   <212> DNA
   <213> Corynebacterium glutamicum R
<400> 264
   aggcaaagac tggaccagca accccaattg gttcggatat gcccatgaca ccgctgcaat 60
   ttacagcttc cgccccaacc acaggtgaaa aggaataacc 100
<210> 265
   <211> 187
   <212> DNA
   <213> Corynebacterium glutamicum R
<400> 265
   aatcgcaacc ctagttgagg gggaggattt agtgcatcat ctaaataaaa gtcagctaat 60
   aggtgaactt tggtgagacc aaaggtgaac tgccaggtcg accaaattac tcgccaagca 120
   gactccgaaa aacacgggta attcatatgg cttgtatcta atccatactg aacagaggac 180
   ctctcca 187
<210> 266
   <211> 100
   <212> DNA
   <213> Corynebacterium glutamicum R
<400> 266
   gtgccttccg tcgactacgg ttaaacaaaa agctttttgt ccatttcact ggattcaccg 60
   aaagaatgaa tccacactcg atcaccaaag gtagcgatga 100
<210> 267
   <211> 100
   <212> DNA
   <213> Corynebacterium glutamicum R
<400> 267
   gcaacatccc attcaccacc tgggaaggct ggtacaaact cgacgcagca gagcgcgcac 60
   tcggtgaagc cgaaggccgc gagcgtaaga agattgttga 100
<210> 268
   <211> 100
   <212> DNA
   <213> Corynebacterium glutamicum R
<400> 268
   gaatctgatc tttcccgcac cgttgaactg ctgaaggcta agcctgttgt taaggcaatc 60
   aacagtgtga tccgcctcga aagggactaa ttttactgac 100
<210> 269
   <211> 130
   <212> DNA
   <213> Corynebacterium glutamicum R
<400> 269
   ttcaaactcc tttgaaggtt tgtgatccca gacacttcgt cgaggcgtga taccaatgaa 60
   aactcataac gttgaaaatg tcaactatta gttttgaaaa cttacatcgt tcgcttgacg 120
   cacagaatgc 130
<210> 270
   <211> 100
   <212> DNA
   <213> Corynebacterium glutamicum R
<400> 270
   ctcagggctg gctgcactac tgcggttaag agtaaactct tggctaaaaa tcttctcacg 60
   ttaactagtg tgccagctgg actcgtctaa ggtggggacc 100
<210> 271
   <211> 68
   <212> DNA
   <213> Corynebacterium glutamicum R
<400> 271
   agctctccta aaagaatcgt tgtattggaa tgaccctatt gtaaccgtgc aacgatagta 60
   tctaagtt 68
<210> 272
   <211> 100
   <212> DNA
   <213> Corynebacterium glutamicum R
<400> 272
   taaccatcct cgtctcccta tccggccgtg gcgacaagga cgttgaccac gtgcgccgca 60
   ccctcgaaga aaatccagaa ctgatcctga aggacaaccg 100
<210> 273
   <211> 100
   <212> DNA
   <213> Corynebacterium glutamicum R
<400> 273
   aagttgcgga aacctttgac gttttgcaga tgacggcggc gattatgggc gatgtggcgc 60
   cacttaacac cattcggggg cttgcgtgag cagcgtaagc 100
<210> 274
   <211> 100
   <212> DNA
   <213> Corynebacterium glutamicum R
<400> 274
   gggtggtggt cgcgttgatt gtgtcgctga atctcgcctt gatcggactg ctggtcaccg 60
   gtcggggctg accggctgtg gtcttcggta gcgtgagtcc 100
<210> 275
   <211> 291
   <212> DNA
   <213> Corynebacterium glutamicum R
<400> 275
   gatttctcct taaatttaga gtgtgagaca cccataggct aggagagaat gggggagagt 60
   ggcgctttga ggtgcctgcc gttgtgaata actattttga ggtacgcgtt acctgtggat 120
   aactctcttt gacctggaag attggagaga tttaggggag gattgggcgt aactctagtg 180
   cgatcagctt ttgaataagc gtttgaatat cacccttgcg ttcgaaaatg tgatcgggta 240
   aggtggttgg tattagcacg gggaactaaa cgggaaaggg gggaagatac c 291
<210> 276
   <211> 100
   <212> DNA
   <213> Corynebacterium glutamicum R
<400> 276
   aaccgcgccc aaaatcccgt cgcgatggcc aaatccatgt tccacgccgt tgaagccgga 60
   agattagccg cccaagcagg ccgaatcccg caacgccaac 100
<210> 277
   <211> 100
   <212> DNA
   <213> Corynebacterium glutamicum R
<400> 277
   cttgctcttg atggccgcta cgcgcagctg tatcaacgat ggagtgctca atagttcgaa 60
   tccgccacaa actccggaga tttggggtag aaacgaagac 100
<210> 278
   <211> 100
   <212> DNA
   <213> Corynebacterium glutamicum R
<400> 278
   tcggaacttg gcatcgacca cgtcatcttc aacatccagc gccgaccagc aagcgaagta 60
   ctgacccaga tttacgaaga agtgctcccc cacctctaaa 100
<210> 279
   <211> 70
   <212> DNA
   <213> Corynebacterium glutamicum R
<400> 279
   gctttaaggc cctccggggc cttttttgct tttcgacgcc tacctccttc ggaggcgtat 60
   tctgtcgttc 70
<210> 280
   <211> 100
   <212> DNA
   <213> Corynebacterium glutamicum R
<400> 280
   gcacgtccac gcgccggact tgcgcgccta tttacaagca cgcttatcga cgtaattaca 60
   ctcggtggaa accgtcgcgg aaaaatggag gatcctcgcc 100
<210> 281
   <211> 341
   <212> DNA
   <213> Corynebacterium glutamicum R
<400> 281
   gtttcgtggc attttaatca gttggggctt tccagaattt ctggagggcc ccatcttttg 60
   tgcatttttc taaggggctt caatagcata tttacctttg aggagcgtga cagttttctc 120
   tcgttttcgg tcagtgcgcg gttgcggggg tgaaaactag acttatgggt ctatttgatc 180
   ataattcccc tgaactgcta ttatggtgta aaaatagaca gatctgttta atctttatag 240
   acagcttcgg ttaatttggt cacactaatg caataaattc ctgtctacag cgttacagtt 300
   aatgaattca attcaaccgc taaacgcaag gagtgctacc c 341
<210> 282
   <211> 98
   <212> DNA
   <213> Corynebacterium glutamicum R
<400> 282
   cgctgctcct gttaactctt ggctttctgc tctgtctcag ccatactgga ccttccagta 60
   caggggaagg tcaagcgcgg ccacggaggg agacagca 98
<210> 283
   <211> 418
   <212> DNA
   <213> Corynebacterium glutamicum R
<400> 283
   tttcgatgga atgtggcgac cctcacgatc gcatgtcatg acaataacac attgtcctga 60
   caaagcaata catttccgaa aattttctaa aacgattcac gaaaatgcgc aaaaccccag 120
   gacaacgccc cggttttcat tcaaagattt tgcttgtcga cgaaaacccc ctcgctttgg 180
   aaggtacggg gttaacaaag ttgcacgtta tgacctgcaa actctgccct tttcactaaa 240
   tttcgctact cattccccaa tacaagtgat aatgtcagat caataaaagc cctggatgac 300
   acaaaagtcc tgcataaaca cggattcacc aagaccacca cccgcaattc agttacattg 360
   ttcaaatgtc ctaacacatt tacatgagct tgttgggcgg gcaacgaaag gagacatc 418
<210> 284
   <211> 100
   <212> DNA
   <213> Corynebacterium glutamicum R
<400> 284
   cctatccaca cggcatgggc ggagtaggcc tggcacagca gacgctggat aaaggcgacg 60
   ccaactaaac attaggacgc caaacattag gattggaggc 100
<210> 285
   <211> 278
   <212> DNA
   <213> Corynebacterium glutamicum R
<400> 285
   gggttgtttt cccgcactca acgcagtttt tgagtgcggg ttttttcatt cttttttact 60
   tttcaaacac gtataaacat tgctattcaa acaaatgggc acccctcaaa actcgcacat 120
   acccagcaat tatcagaggt tctaataatc cccttagcct ctgctcgaat tcttgaaaac 180
   ttcaataaac aggtgtccac catagggaac ataatctaca gctgcagcca aggtttttcc 240
   taggctactg ccatattccc tctatttagg agactccc 278
<210> 286
   <211> 77
   <212> DNA
   <213> Corynebacterium glutamicum R
<400> 286
   catgcgccaa cagcaaatat tagtaaaatg ttagaaatag ctgtttttga ttcactttgc 60
   gcatgtaggc tgtgacc 77
<210> 287
   <211> 100
   <212> DNA
   <213> Corynebacterium glutamicum R
<400> 287
   atgtgggtga gataaccgac cgtgatgtcg ccctagcaaa agtcatcgac gcccacgcca 60
   agaccttgaa catttcggca gaggcttaag gttaaagatt 100
<210> 288
   <211> 128
   <212> DNA
   <213> Corynebacterium glutamicum R
<400> 288
   agtgtaaaaa gccgtccgaa ccaaaggtcc acacctctgc acgagtagaa gctcacccaa 60
   gttttcaaag tgccgttgat tcttgacaac cacccgccgc tccttagagc agatttgaaa 120
   agcgcatc 128
<210> 289
   <211> 100
   <212> DNA
   <213> Corynebacterium glutamicum R
<400> 289
   gccttactcc cgaggaagat cagcagctca ttaggatcaa cgcccttatc agcaaagtta 60
   cccgtcagat tcgcattgtc actgagggag cagcaaaaca 100
<210> 290
   <211> 100
   <212> DNA
   <213> Corynebacterium glutamicum R
<400> 290
   atggtcgtgc cgaatttccg gtttggatgg gctttgggat gtaatgggag cggatcggcg 60
   tgatcgtgcc ggtttttctt gcgttggtag tcttggggct 100
<210> 291
   <211> 228
   <212> DNA
   <213> Corynebacterium glutamicum R
<400> 291
   attgagtcat cttggcagag catgcacaat tctgcagggc atagattggt tttgctcgat 60
   ttacaatgtg attttttcaa caaaaataac acatggtctg accacatttt cggacataat 120
   cgggcataat taaaggtgta acaaaggaat ccgggcacaa gctcttgctg attttctgag 180
   ctgctttgtg ggttgtccgg ttagggaaat caggaagtgg gatcgaaa 228
<210> 292
   <211> 183
   <212> DNA
   <213> Corynebacterium glutamicum R
<400> 292
   ttcaagcccc ccgtttcaat tgaaagtttg aactcggctt ttgatgcgaa ttttccccca 60
   attcgcaaac ttttcaatcg accttccggg aactcttcgt agccctttta cgacagttac 120
   ataattgcag gttagagggt tcacaggcag ttccaatctg ccccaaatca cactcgtccc 180
   att 183
<210> 293
   <211> 100
   <212> DNA
   <213> Corynebacterium glutamicum R
<400> 293
   atcaggtgtt gcagcgttat ttgactgact tggggttgac tcctcagggt cgtaagaact 60
   tgggtttgga ttctggggat gatgaacaag acgggtggtg 100
<210> 294
   <211> 100
   <212> DNA
   <213> Corynebacterium glutamicum R
<400> 294
   agatctagcc aacgaacttg atttcatcga gctggaagag acagaggcag agcagcaatt 60
   gaagtggctt ggccttatgc agtttttggg aagtagataa 100
<210> 295
   <211> 100
   <212> DNA
   <213> Corynebacterium glutamicum R

<400> 295
   cttgcaaaca ggcgtggtgg tggcgttcat tggctcacca attttccttt atttactgct 60
   cagcatgcgc aagcgacgcg gattggggct gtaaaaactc 100
<210> 296
   <211> 251
   <212> DNA
   <213> Corynebacterium glutamicum R
<400> 296
   aatttagaaa atccgccgtt ccacacaagg aacggcggat tttttgatga ctaagtgagt 60
   ttggatgcgg aagatttggg ctatttccat aaatcgaacc aggtgaccga gagttagaag 120
   cctggagagg acggttcgct tggaatcttt ccccttgttc gagggtcctg ccttttatct 180
   gggtggtgga gttatgaaaa acttgcgtat tagaccaaga gccgcttttt tcggggagtc 240
   tttggtctcc a 251
<210> 297
   <211> 126
   <212> DNA
   <213> Corynebacterium glutamicum R
<400> 297
   atctacctct ttttcttaat ctcttacatc tttacaggaa accccttgac ggcatcaatg 60
   ggtggtatct agtatcgact agaacgttat agtagaacgt tctagtaaaa cttggaagga 120
   tgaaaa 126
<210> 298
   <211> 100
   <212> DNA
   <213> Corynebacterium glutamicum R
<400> 298
   agaacctgcg cctgctcgcc agctggacgg tcaccggtac tgataaaaca ggcactgaca 60
   gggccttcca cccggcggac gatgtcccta gtctggagga 100
<210> 299
   <211> 238
   <212> DNA
   <213> Corynebacterium glutamicum R
<400> 299
   aacctgccat cttagtgctt aacaccactt gaatgggagg agttcgcgat agttcacagc 60
   atttaattca caaaaccgca gatagtagca ctctcccacc ctcaataggg ctcaacctgt 120
   gcactgtaac ccatcgcccg tcaatcaagc tgtcaaaaaa aatacaagtt aggtcacaaa 180
   atgatttcag tcgtgagaac catcacatat aaaacatctc atgatctaac atttcttc 238
<210> 300
   <211> 100
   <212> DNA
   <213> Corynebacterium glutamicum R
<400> 300
   accaaccgcc acgcggtatc aataatttcc gctaggcggc cagtggggga tcgggaaaaa 60
   tcactggtct ttagactcca caaaatttgc aagtgcttta 100
<210> 301
   <211> 483
   <212> DNA
   <213> Corynebacterium glutamicum R
<400> 301
   cccataccag ccccaaccag acaggcctga caagattcga gaatttccac agcttttggc 60
   acgtgtgtct ggtttttgct cccgataacc ggacaggtgt gccaaaatca tagacttttc 120
   gccaatcttg tcacgcctgt ctggcccgaa tcagcattcg aacgccatga ccaaaccaca 180
   ctgcccacgg ctcaggtttt cccggttcat gggcagcgtg gtctggtttc gacggcttga 240
   aaccagacac aaatgcccag agttgtcgaa tttccagttc gtgggcactg tggtttggtc 300
   gagcttaaaa ttggagccgg aactcaaaaa acttgccctt tcagaatcgc gtctaaggca 360
   cgatcgcaat gtgatctata caaacacccg tttgagaatc tcaacctctt aaaacagcgc 420
   agactattga tttggcaact accctatata tttgagtgtt tattgtcgaa aaaggggttt 480
   caa 483
<210> 302
   <211> 800
   <212> DNA
   <213> Corynebacterium glutamicum R
<400> 302
   gatttcctcg ttcccatctc ggctgcatgc gcgcacgatt ttaaagtgtg tatctgggcc 60
   atttagtgat gttggtggtt cccattgagg gcaccatgta catcctaggc gtttgctgga 120
   gcaaaatacc tgtgaaaaat ctgatcctga aaattttaaa aactgcgttc acatcaaccg    180
   gcttgtcata cctaacggtt aatagtttta aactacggat gccctaaaga gcaacttctt    240
   gctttgagac ttaactcacc gcgggcattt atgctggtaa atgctcacgt tcaattatgg    300
   cagcgtcgca cagatatgac cacaaagaat taaaattgtt tgaaaatttc tttaggttta    360
   tcaattttgt accgccaaaa cgcgcggtaa ctgcatgaag tctcttcttt aattaaattc    420
   atccgaccag cgaatgagac cagttgactg actggttata taacctggcc acacttgagc    480
   gccctcaaat ggaattggaa cgacttttat aggcgctaca tcggagaaat ttgacccgtt    540
   tttcacaatc tcctagagct cccgaggcta gctagaacaa cgcacattcg ggcactttcc    600
   gaccttttcg atagccgtat atgccatgga cccactcctc aattgggcga ccgttagact    660
   tgaaacacca aggctagaga ttttaactaa agagtctcct catggattcc catttggagc    720
   cttatttaca caggccacct ggcttgtttc ccaccgcgat gtgccacaat aacgccataa    780
   cagaaaggca tactgacaca                                                800
<210> 303
   <211> 1000
   <212> DNA
   <213> Corynebacterium glutamicum R
<400> 303
   ttagcgttca acccgtcaac aaatgttgcg tcaacagtca acttcacgta acattaagac     60
   atgccacgaa aggagaaaca catgcaagac aagcttttgg gacgccaaca agcggcccac    120
   attctcggga tttccctgtc caaactcgac gaactcaggc attccggcca catcaaatac    180
   ggccacatag gaagccgaat cttcatccca caatccgagg tcaacgcatt catcgaagaa    240
   gtcatgcggg catgaaaata ccccgacaca ggaaacattt ggactgccat ccaaggtgca    300
   tgttaacggc cagggtatgc gcacgaaact aaggagaatc aatgcagttt gattataaca    360
   caccaagcga tgatgaaaca ttgccagaac gcaaagagcg actgcgttcg ccccacacta    420
   tccaaaggct tgtcattgaa gcagcaatgc tgaaaaggct ttacgcgctc cacaatgact    480
   tcaaagccga agttgccgaa gcgctcaacc ctggcgattc cattaaggca aaaaatgcgc    540
   aggcgctaga cattgggaca gtcaccatgt cgtcgccaaa taacaaggct gtccctactg    600
   atgagtccat tttggtggcg gaagcgcagg aacgtggcat ggagctggtg gaccgcctgc    660
   caaacaacga cacccctgaa gctgttgcaa ttatagacta cctgctggag cacgcagcgc    720
   acctactgcc agcccccacg gtctccaaag atgatttaga gactatcgct aaggacgtgc    780
   tggaagcgtg gcaggagaca ggccggaaac ctttgggctg ggaaatcaaa caagcatcca    840
   ccccatccat atcagttcgg cctggcacgt caaaggtcgc taaagcggca attgaccaca    900
   tcgtcggtga agttcaccaa ctcctcccgg agactcccca actcgaaaag aagaaagaag    960
   cctaaccatg actactgttg ataagtacga aaattctgga                         1000
<210> 304
   <211> 100
   <212> DNA
   <213> Corynebacterium glutamicum R
<400> 304
   aaacgtattc cttgatctgc gcatcaaggt gctgaagaac tggcagtccg atccaaaggc     60
   tttgaaccgc ctgggctttt agctttaagg gggtgagttc                          100
<210> 305
   <211> 100
   <212> DNA
   <213> Corynebacterium glutamicum R
<400> 305
   ttcatcatgg cactgcccgg ctccacgggt gcggcgcgcg atgccacggc tgtcctcgac     60
   ccgctcattg atcacatcac tggaactctg caaggccacc                          100
<210> 306
   <211> 99
   <212> DNA
   <213> Corynebacterium glutamicum R
<400> 306
   gtttgctcct taaaacacca atacttctcc tccatctttc cctcaaaagc aattctgcgc     60
   acgagatacg gcaaaattca cggagtaggg tggcttaac                            99
<210> 307
   <211> 100
   <212> DNA
   <213> Corynebacterium glutamicum R
<400> 307
   ctggagattt ccgatttcca gcgcgcccgc atcgacgcga atgctcagga attgcaggcc     60
   gagcgcgagg cagtgcgcga cttgctctaa tctttaacgc                          100
<210> 308
   <211> 763
   <212> DNA
   <213> Corynebacterium glutamicum R
<400> 308
   tccaattggg cacacctcct tagcaaccac gctatgcgag agttgcagct cgacgagagc     60
   aaagatggca ggattgatca ccaggatcga tctttaaata aggactattt cttacctgga    120
   agtaacattt tggccgttga ggataatacc tttggtgcat tttgagccaa aaaattcttg    180
   gcctctggat taatagttgc caccacagca ctcgcaacca aaactgagcc tataataaat    240
   gcttctttat ttatctgcag tgattgcaga ttaaatcttt tgttgttgac gtcatgctcc    300
   ataagcttgt cgtcaaggcg ttctagccga tcgaaagcat ctcggcgact ttcttctgat    360
   gaattagggt cgctaataac ttgcgtaagt atgcttttcg tcttatcaaa actgtcataa    420
   agcctatctg agctgtattt attgctagat agggcctgtt ggaaagaatc ggccattgct    480
   tgagacactt tcaccatagt gttatgagac acgccctaag ccaccgtggc ctccgctgca    540
   atcatcaggg gatcatcagg ggatgacggt gagtattcca cctaccaaca accgcagtga    600
   gggcctccag tgtccaggac gcgagatgtt ttcctgaccg cgtgtgggaa ggctggtttt    660
   atgaagtcgt cggcattttt cgcttggatg acctgggggt ggcacttctt accggtactg    720
   gcaggccctc ctttccgggt gggcgctgat ctagcatgga gac                      763
<210> 309
   <211> 100
   <212> DNA
   <213> Corynebacterium glutamicum R
<400> 309
   tggatcgtcc tcgccttcac attcgtcggc cttggccttg ctctcctcgc gatgaagcaa 60
   tggcgattcc gcgtcagcta ctgggtataa ggagcaccac 100
<210> 310
   <211> 100
   <212> DNA
   <213> Corynebacterium glutamicum R
<400> 310
   cgaaaccatg ctcggcggta gacagctggg ctaagcggtt ggctaattga gcaacggaca 60
   tggacaccca ccttagttcg gcgggttaag ctgtgtaacc 100
<210> 311
   <211> 100
   <212> DNA
   <213> Corynebacterium glutamicum R
<400> 311
   ccacctttga acgtgagggg atcttggagc gtgatgagta tgactcgcta gtgattggtg 60
   actttggttc tgctgtgctc aaccacgtta ggagtgggat 100
<210> 312
   <211> 52
   <212> DNA
   <213> Corynebacterium glutamicum R
<400> 312
   gatttaagtg aatatcgcga tgaaacagca ggtggggaaa gattttcaac cc 52
<210> 313
   <211> 100
   <212> DNA
   <213> Corynebacterium glutamicum R
<400> 313
   ccgccatctt ttcttcttgg atgttgtggg cgttgggctt aagtctcgca atcattctga 60
   tcactttgct gtggatcgtc cgacgcaaga aaggccctca 100
<210> 314
   <211> 100
   <212> DNA
   <213> Corynebacterium glutamicum R
<400> 314
   ggcgggaaat tacaatcagg catcggcgtt gtcgttgatg tttgcgatta tcggtatcgt 60
   ggcgctcgcg ttgacggtgc gcagccagaa ggagttttag 100
<210> 315
   <211> 100
   <212> DNA
   <213> Corynebacterium glutamicum R
<400> 315
   ctccaccctg gcgaatagaa caaacaaggt tcttcgcatg aaggactacc acccagaact 60
   gaaggtctaa aagcttttcc cgcccggttc aatagcgtta 100
<210> 316
   <211> 52
   <212> DNA
   <213> Corynebacterium glutamicum R
<400> 316
   attgtaggta gtctcgtggg cacaactgaa atcttattga aaaggagtgt cc 52
<210> 317
   <211> 100
   <212> DNA
   <213> Corynebacterium glutamicum R
<400> 317
   gtcacctcac caccgaggac ttccagaacg aaggcgcaga ccacatcatc gattctgtag 60
   cgatcatccc agcgttgatc ctcaactagg cataaaactc 100
<210> 318
   <211> 100
   <212> DNA
   <213> Corynebacterium glutamicum R
<400> 318
   tgggcggtgc acttcttgct ctgtctgatc cggaggctga gtgggaggaa atccgcgtta 60
   aatcacggcc tctgctgaat ttatttgggg ttgaattccc 100
<210> 319
   <211> 100
   <212> DNA
   <213> Corynebacterium glutamicum R
<400> 319
   gctggctatc ctcaattccg ctcgggtcgc agtgccacgc ggagcgatta gtgattttga     60
   tacgcaagaa aaagtttctt agcagggtaa cctaaatgtc                         100
<210> 320
   <211> 82
   <212> DNA
   <213> Corynebacterium glutamicum R
<400> 320
   ctctcgaata ggccatttct tacttcatcg acaatactgg cttagtagaa aatgctgtcc     60
   agaactgttg aaggagttga aa                                              82
<210> 321
   <211> 100
   <212> DNA
   <213> Corynebacterium glutamicum R
<400> 321
   ctgccggctg ggtaagaaaa ggtcgtggcg gcgtttatga agtccccact gagcggatca     60
   tcccgctact aaccatcatt tctgccagtg agaatcacta                         100
<210> 322
   <211> 766
   <212> DNA
   <213> Corynebacterium glutamicum R
<400> 322
   actcccgggt tttgcttggt tttccggata ccgtcctatt tgaccgacgt ctggggtggg 60
   gtggatcttt cggccacctg gtcgaatttt cctcaacccc actacccaga aagtacaaag 120
   agcacaggag ccgttttata aggatcaaat ttctcagaga ggtataattg aagcatttca 180
   gcctcttata tacgatcctg agaggaccct gattctagag ttccagatag taattgcagg 240
   ctgactgtca tatctgacca aaaccgacct cgccaatccg agtcttggcc tccaacgtat 300
   atttcggctc cgaatatgtc tgaacatccc aataatatcg acaatacgat atatttttta 360
   tgcatgcctc tcgaaaataa ttcgaatctt caaaagattg aaggttacgt ctcaaaaatc 420
   accaaaaaaa cggcaacaaa gtcactaacg attccttaag ttctggggcg tgcgacacca 480
   aaatggaacg ctggaagagc tagtaaagaa ccgcgtctag ttaaaagccc atcaaaactc 540
   aattcatttg cgacttcagt aataggtgac gtcactaaat caattaacct aaaagggcgt 600
   caatcacctg tttgaagtag tgattcctag atctataaat aactggaaag aacgcagagc 660
   tgaaaaaaga cgcacagaag ctacacacat agctacatcg tcagcaccaa agccggaaca 720
   agaactacag caaccgacga cactcaacga gcgcgacctc caagac 766
<210> 323
   <211> 100
   <212> DNA
   <213> Corynebacterium glutamicum R
<400> 323
   gcaagtggaa ccacgatggg aacggtaact gctgccgctg cggtattact cgtagtttca 60
   gaaattaggt gtcgatgcag caatacggaa ctttgccaat 100
<210> 324
   <211> 53
   <212> DNA
   <213> Corynebacterium glutamicum R
<400> 324
   cggattgaac ccaagacggt tttcttcgcc ggggagctcg accccggaaa ccg 53
<210> 325
   <211> 65
   <212> DNA
   <213> Corynebacterium glutamicum R
<400> 325
   aaattcgctt ttcgacgcct ccctcaatgc gacgcttaaa gcatctacgg acctttgagg 60
   tcacc 65
<210> 326
   <211> 100
   <212> DNA
   <213> Corynebacterium glutamicum R
<400> 326
   gcctgtggca cgggagctcc cagcggcaag gtgagtctga cctcgtggaa cgttggcgaa 60
   cgccccgctg cgatgttccc accaaggaag gactaggcgg 100
<210> 327
   <211> 100
   <212> DNA
   <213> Corynebacterium glutamicum R
<400> 327
   gctgaggttg agacccagct gaacaccatc tacacccgcg acatcgaacc acttatttaa 60
   tccgagcact tcagctacac ctatttaagg aggctgtgac 100
<210> 328
   <211> 438
   <212> DNA
   <213> Corynebacterium glutamicum R
<400> 328
   tggcatggcc agctaagaaa ttcgaaccga cttcattgat ccttggctcg actcgctcag 60
   cgacgaagtt tatgacgcag tacttgccgc cattgaatac cttgccgagc aggggccgac 120
   ctgcggacgc ccattcgtta ataccatctc acaatccaga cataagaaca tgaaagagct 180
   gcggccccgc gaaaaatcac gcgctaagaa catcaggatc gttttcggct tcgatcccca 240
   ccgacatgca attttcttgg tcgccggaga caagaccagt caatgggata agtggtacag 300
   caagcacata ccagagcaga tgaaagattt ggctaccacc tcaaacaact caagaaaggc 360
   aactaacagc tacctactca gggagaaccc gttaatcgaa acaggtacca ctcacccaca 420
   atctttaagg agaacccc 438
<210> 329
   <211> 100
   <212> DNA
   <213> Corynebacterium glutamicum R
<400> 329
   gacggcgcca actctgcatt cttcgcctca gcgtgcgtgg cagtgtttgc attgatcgtg 60
   ggcttctttg taaagaggcc agcccactaa gctaggtcgc 100
<210> 330
   <211> 65
   <212> DNA
   <213> Corynebacterium glutamicum R
<400> 330
   gttaatagcc cgcacccacc gcccgcgcgt ttaccccagt tctactggga ttagaatggc 60
   taacc 65
<210> 331
   <211> 70
   <212> DNA
   <213> Corynebacterium glutamicum R
<400> 331
   taaaactgcc ctggttgcaa caattggcaa caagtagata tcgctcagtg tgacgggtga 60
   tcataaagtt 70
<210> 332
   <211> 100
   <212> DNA
   <213> Corynebacterium glutamicum R
<400> 332
   cacaaattga aatgatgctc agggaacaat tgcgcaaggc gggtcggttg cctaaaaata 60
   ttgggaacat tccgaaacca ggccgaccca aggagaatcc 100
<210> 333
   <211> 226
   <212> DNA
   <213> Corynebacterium glutamicum R
<400> 333
   atcttctaac tgctttcttt aaagcacccg cacatgtctg ttgaggtttc acctgtggag     60
   acaatctctg ccttcacgga ttcgaactga cacagttgaa ggcatgtcgg gtgctttgcg    120
   tattctttgc cagtgtgatt taggcgacac cattaattta ttgggtatcc accaattacc    180
   gctgtgagca ctgcaaatta cgtattcgaa aagccatgtc caccac                   226
<210> 334
   <211> 343
   <212> DNA
   <213> Corynebacterium glutamicum R
<400> 334
   gtgctgaact atacagctgt tgctgaacta acttcaacat ggggtggcgt cgttgccaga     60
   ggtcgtctgc actttcaggc tgaggccttc actgcgcaag aaggaagcag ctgcctagtt    120
   ggcggtgttc ccgacgtgat ttaaaggtga gggctatcgc cggcagcttc cggcccaggc    180
   cagcggcgac ccacgtaccg atcaaggtgt cccacttcct aggaagtggg acacccttaa    240
   tgaaaccaat tgaaaatgca tagtcccaag agaaaactgt tccacaagct attccaacga    300
   actgtctcac gaaccctgct acagaataga cttccggtac gct                      343
<210> 335
   <211> 100
   <212> DNA
   <213> Corynebacterium glutamicum R
<400> 335
   cacttgctga agacgcccac atcgaagacc ttgcagatgt aaacgcaaac gcctaactgt     60
   ttttcgagct aaacccatcc ttgaaaggat cttttccacc                          100
<210> 336
   <211> 517
   <212> DNA
   <213> Corynebacterium glutamicum R
<400> 336
   gtcccgaaac tcttgagccg tcggctggcg acgcccctgg gtgatttgtt ctgcattcac 60
   tggaaactta tctcgctttc atatccctcg agcgggagtc ggtgatcggc cactctctaa 120
   gcaatgccgg ctttaaaata aagcaactta tatgtttctc accacatctg gccgacgacc 180
   gcgaagtatg ttgtcgatca cagctaaacg tgtgaatgtg aagttaccta actcacattg 240
   caatgcgata gcgatttgga aaactcgctc cccccaatac cttaacttga acttaaaagt 300
   agtgttttac ctgcatttat aaaagttccc gatctacccc ctctttgccc cgaaataccc 360
   cttttgcaaa gattgcaaac acaacagtgc aatagttaac gggcttcaca tatcaccatt 420
   ctgtctggtt ttaggctatg ttcgggtcgt ttaggcaaaa atagttttgt gggatgaaac 480
   gcataatccc tcagttttgc gcaatcgata gcctgaa 517
<210> 337
   <211> 180
   <212> DNA
   <213> Corynebacterium glutamicum R
<400> 337
   aagcgattct gcacattttt taacatcccc aaggcgtgat ttcgattttc ggaatcacgc 60
   ctttcctatt tccgcgttaa aataccaggt caacacacac aggaaccgtt cagaaacctt 120
   ccagattact cagtttttaa tttcactttt ttgagaagta ttacttttat attagttctc 180
<210> 338
   <211> 441
   <212> DNA
   <213> Corynebacterium glutamicum R
<400> 338
   gatctcgcac cttcctgcat taaaactcta aaagtttaat aataagacta gtataaatcc 60
   cttacgcttt tacgcagggg tcattcaccc agtcagattt tggtcccacg ggttttcacc 120
   atcctagcaa ctagaacccc tcaaaccctg atcccctcag aaactgacag ataacaccta 180
   ctgctttaac cccgtcctga cctgcaaaaa caagttatct acaagaattc accggagatt 240
   cacctcaaca cataaccgca ggtcagaacg ggcgtactgg caggagttcg cctgcatatg 300
   gccatttagt cagctttgct tcactaacaa gtcacaattt gttcatcatt gcggtcgaca 360
   ctgttgtctc gggcgaacac cccacatcag agaacgcgat caaagctata aaaagtagct 420
   gacaataggg agtatttgaa g 441
<210> 339
   <211> 100
   <212> DNA
   <213> Corynebacterium glutamicum R
<400> 339
   ttgactatct cgaattaggt gagggctggg agccagaaat tttgtgctct gatgctgttg 60
   aaattttaga aaatctcatc aaggaactaa ggggaattcc 100
<210> 340
   <211> 100
   <212> DNA
   <213> Corynebacterium glutamicum R
<400> 340
   ctcgcagaga tcgccagcgg ctggctagaa attgaagcca agcgccgggc gcggaaggcg 60
   ctggctgagg ccttcggcgc ggaggtccag ccactgccgc 100
<210> 341
   <211> 100
   <212> DNA
   <213> Corynebacterium glutamicum R
<400> 341
   tttttctgca gtgaattccg aggttgatca gttgattgag tatatgaccg ttcatgcgga 60
   gagctatcgc agttagtccg ctcgcacttc ggaggccttg 100
<210> 342
   <211> 100
   <212> DNA
   <213> Corynebacterium glutamicum R
<400> 342
   ccgacaatcc agtggaactt ccctatcgct gtgatgcatt tttactttca cgttccggta 60
   ccctggcagg cagatcttcc aatctttagg agccctcgcc 100
<210> 343
   <211> 54
   <212> DNA
   <213> Corynebacterium glutamicum R
<400> 343
   tggtaatttc tttcgctaat agtcaaatga tcatttgagt gttagtgttt tctc 54
<210> 344
   <211> 111
   <212> DNA
   <213> Corynebacterium glutamicum R
<400> 344
   aacgacaatc ctagaagcct tcggtcaata aatttcagca aattcccgat ctccacgacg 60
   cgtcatctca attccaccta ggcttggacg caggttagaa aggagccttc g 111
<210> 345
   <211> 272
   <212> DNA
   <213> Corynebacterium glutamicum R
<400> 345
   aagcgagatt ttgccagact tgtccagggt ttctgaaatt tatatataca agtttgggaa 60
   aagttaaccc aatcttcact tttgagctga aaagtatcca aaactccacc gaacagacat 120
   tgacttattg gttttatgaa gtttttgaac gcaaaaggaa tttaataaga aaaagcccct 180
   tcgattcaag aattttcatg tagagtaatg aaacaaatat ctacaagttt taaaattgaa 240
   gtccaaaatc tagacaagtg tccaggaatc tc 272
<210> 346
   <211> 100
   <212> DNA
   <213> Corynebacterium glutamicum R
<400> 346
   gcgggagtgg acgggccgcc agccatcggg gcgacaatca cgggagtttt caacgtgtca 60
   aggatgctca tgtcaccatc ctaggcgcgc ctgccatagg 100
<210> 347
   <211> 100
   <212> DNA
   <213> Corynebacterium glutamicum R
<400> 347
   ttgagcgatc acagcaccga gatctgtgct gaagaattca atgattgggt tgatgattgc 60
   gttgaagtcc atgtctaaca gggtagtaca aaagccaaaa 100
<210> 348
   <211> 100
   <212> DNA
   <213> Corynebacterium glutamicum R
<400> 348
   gcgtttagct ccaagaaagt cagtgctgtg catggctcgg tgctgctcat gcttttcggt 60
   gtttacatga tgagcatgtt cgcctgattt aggtagcctg 100
<210> 349
   <211> 100
   <212> DNA
   <213> Corynebacterium glutamicum R
<400> 349
   agcatcacga caagccttga tcgacgaagc gctcaaccag caggagcaga agaaccacgg 60
   cctgccggtc tactctggcc caacgtcggc ggtggaataa 100
<210> 350
   <211> 174
   <212> DNA
   <213> Corynebacterium glutamicum R
<400> 350
   acttcgttcg aaactctagg cccgcattag aacaaggatt tgtgcgcttt tcctgttctg     60
   gtgtgggttt tcctcacatc taacaatcga ataactgttc gaataaaagg ttgaaggtgt    120
   cccaccccca cggcacaatg gatggcaaga acacatgaat ccagggggat actc          174
<210> 351
   <211> 256
   <212> DNA
   <213> Corynebacterium glutamicum R
<400> 351
   tcacgggtgt ctgagattcg caagcaacgg ccgagttaga accactacgt aggcgtgtcc     60
   caccatcacc gcgtcgaaaa gcgccctctc ttaacccccg caagggggta acttttacgc    120
   gcacgcgtgc aacgcgctag ttttgacggc agtccccacg ggcgcccgag cacgggctga    180
   gatcgcgctg attgctgcgc gagcaccgtt tgaacctgtc cggttagcac cggcgaagga    240
   agagaggaat ggtgca                                                    256
<210> 352
   <211> 100
   <212> DNA
   <213> Corynebacterium glutamicum R
<400> 352
   ccggcgacca gggcgcggga gatgaatgaa acgtcaaaag gcactatgag ggcgtcaata     60
   aaaaacttca tttgaaaatg ataaccgtta tcattaagga                          100
<210> 353
   <211> 100
   <212> DNA
   <213> Corynebacterium glutamicum R
<400> 353
   tcctcggcac ccgctacccc gtcggagttg tcaccggcgc attcggcgcc ccattcctta     60
   tctatttact cattcgttcc aaccgcgcgg gagtaaccct                          100
<210> 354
   <211> 100
   <212> DNA
   <213> Corynebacterium glutamicum R
<400> 354
   tgcagatttt acgtcgcctg cgatcggggg atccggcgca acgtcaagcc gtggcctttg 60
   actctgtctc tggtaaagaa gcagttgcgc gctagaaacc 100
<210> 355
   <211> 170
   <212> DNA
   <213> Corynebacterium glutamicum R
<400> 355
   ttactgaaag ttctgagcta aacacccgcc gcgcttcaat gtggcgggtg tctagtttta 60
   gggaacccca tacgaatcgc atcaatctaa tgaaagtcca cgctcagtat ggtttgctta 120
   ttaaatctat ccccatttag aacatttgat gcccaacagg agtccgcgca 170
<210> 356
   <211> 100
   <212> DNA
   <213> Corynebacterium glutamicum R
<400> 356
   ccatcatcgg cgcgcttgga tggtttggaa tgttcgcagt ttgtctgtgg agcacgctgt 60
   aaaaggacgt taaaaaccag ccgttgctga ggagaaactc 100
<210> 357
   <211> 100
   <212> DNA
   <213> Corynebacterium glutamicum R
<400> 357
   ccggccgcga tgccaccaga gctctgaaag ttctccttgg tgtctatgaa tcagcaacca 60
   cccaccagcc ggtctctttg atctaacgga agcttttaaa 100
<210> 358
   <211> 100
   <212> DNA
   <213> Corynebacterium glutamicum R
<400> 358
   catcaaagtg accgccggcg gcgtcgaatg gtccgttgca ggaaacgcgg aagcagttag 60
   tgagatctcc gaaactttaa gcgcactaga ctaacaacac 100
<210> 359
   <211> 155
   <212> DNA
   <213> Corynebacterium glutamicum R
<400> 359
   acgcccacat ccctctctct tttccaggac aatttcctgg taggagagat ttgtcgttgt 60
   ttgatctagg tcaaggaatt aacccggaaa ggaccgtatc tttaaaggtg caagcacagg 120
   aacatgacga taaaagatga aaggacctgg ttacg 155
<210> 360
   <211> 319
   <212> DNA
   <213> Corynebacterium glutamicum R
<400> 360
   ttcttaggtt taaaatcgct ggacaatggg atattcaatt gtttggctcg agcttatgta 60
   ctagagaatc ccgcatcaat cccgtctcaa tcttgctttg attttgcttt gattgggcgg 120
   gattgcttat tgaaaagacg tattcgagat tgatcgaatg cgtcttttgt caacccttag 180
   tgggctcagc ctgtggacaa ccctggatgg ggactgctta tccacaggtg aggactggag 240
   atgtttgaag cccgcgataa aaccacgttg aatctgaaag cacagcttaa tcagactttt 300
   aaacaatcgt gggggacag 319
<210> 361
   <211> 100
   <212> DNA
   <213> Corynebacterium glutamicum R
<400> 361
   tacaacgagt acaacgcttt cgaccagcaa gtattcacct attccgctga cagctacaag 60
   cccaccttct aacccgccta tatataagga gtgaatcacc 100
<210> 362
   <211> 100
   <212> DNA
   <213> Corynebacterium glutamicum R
<400> 362
   tctatcgcgc aggggattaa gcagctgcga gcaggcaaaa ccaccattgt ggtgagttct 60
   tcgcccgcgt tttacaactt ggcggatcgg gtgatttcac 100
<210> 363
   <211> 100
   <212> DNA
   <213> Corynebacterium glutamicum R
<400> 363
   tccgcagtgg aaaaactcac tcgcccaggc tgcgaaaacg cccgcgacac cgtggaaggg 60
   gagacaccag cgacttttgc gacatcataa atggtggctt 100
<210> 364
   <211> 100
   <212> DNA
   <213> Corynebacterium glutamicum R
<400> 364
   aggggagccc tgtggacgct gtggcacaca gatcgtccgg gaaaacttca tgaaccgtgg 60
   atctcactac tgcccaaact gccagaagcg gcgctagctg 100
<210> 365
   <211> 135
   <212> DNA
   <213> Corynebacterium glutamicum R
<400> 365
   ttctccttaa tttttgggca ttgaccttgg agtttacgcg gatgtgtgag ggaaatctgc 60
   tcgatctcgc caatggtatt accctccgtt gtcaggtagg tagaccgggc tacctacctg 120
   tcaaggaggt gcgac 135
<210> 366
   <211> 403
   <212> DNA
   <213> Corynebacterium glutamicum R
<400> 366
   gtcaatctcc ttggatcgtg gtaggaccgt gagaagacac cgcctcaagg tcgatgccat 60
   acattttcta gcacgggtgc ctgacggccc agaaatcaaa aaccctgctc acagccttat 120
   aacagggcga aaagggggtg actccggtgg gctgggtcac caccgggaca ccaccccaca 180
   gccgtgggcg atgtccttct acccgccccg ggtatgcggt gcaggcagtg aaatccctgg 240
   tggcgcctgc tgaactgacc aggggaggcg atgccgccgg cccggggtgg ggcacagggg 300
   tgacggcggt cgaagggtga tgtttgaaga tttgatgaag gtttccccgc cgggcactga 360
   gcgagggtgg tattctcccc tggccggagc gatactgggg tct 403
<210> 367
   <211> 100
   <212> DNA
   <213> Corynebacterium glutamicum R
<400> 367
   atctatgaga cccctcaaaa caccgagaat ttcctcgatg cattcaccaa ggcagttgaa 60
   gatctcaccg ctgccgctaa ccaggtttag aattatttaa 100
<210> 368
   <211> 51
   <212> DNA
   <213> Corynebacterium glutamicum R
<400> 368
   ggtgaggtga gttatttaaa gactgcataa tattttgggg agtgaactgg t 51
<210> 369
   <211> 498
   <212> DNA
   <213> Corynebacterium glutamicum R
<400> 369
   gaaacaggta cctcaaaggc cccgcaggtt tgatacttgc ggggcctttg gcgtttctag 60
   gtgatctggt tgggtcagac gggcaaaaga gaccaaagcg ctcgaagatc cgagggccct 120
   tggcctaaaa atgcactttt ccagagagcg aaactgtcat tcgagaccaa aaaatcgacc 180
   cgccggggga cgcttggtct cgattgacat tccgctacca agcaccgaaa aattgccgcc 240
   aggatcaaaa gaaagtggcc ttccagaatc gtctctgagg gcctcaaacc cacaacgcca 300
   tacaaacacc taacccagaa tctcgacccg ttaaaaagcc tttgatcgac tagtagcgtt 360
   gaggtggttt cgtcgatgaa tactgttgtt actgcgtgag ctggtgatga gccagaatca 420
   ggtgactgga atgcctgtgg ttcacgttta ggggatgatt cacaatgaag aagccaactc 480
   tatatttaaa tctcgaat 498
<210> 370
   <211> 227
   <212> DNA
   <213> Corynebacterium glutamicum R
<400> 370
   cgatatttca gcaaaccgat gctattcgat aagccagaca gcgctctgat ctttcatccg 60
   agagatcaga gcgctgtctt attccaaaga aatggcctgc agcggggagt gctccccgct 120
   tttgagatac ttccattttt cccgctttac ggtgaagttc atcagctaca aagcacatgc 180
   cagatagctg aaaaggaaac cactcccctc ctcggaaagc aggaacc 227
<210> 371
   <211> 96
   <212> DNA
   <213> Corynebacterium glutamicum R

<400> 371
   aatttctcat ggtatcacta gaaatttacc gtgaactaat gttagagttt gtttcacgct 60
   aaaaagcgta aattaacatg agaaagaaga tttgtt 96
<210> 372
   <211> 100
   <212> DNA
   <213> Corynebacterium glutamicum R
<400> 372
   aacttaaaaa cccctcacaa aagtggcgag ctccccgatt gggactcgcc tcttttcgta 60
   ttctcactaa gatcacccga gcgaccttgc ccaccacccc 100
<210> 373
   <211> 100
   <212> DNA
   <213> Corynebacterium glutamicum R
<400> 373
   tggggaattg gggctgatca gggtgacgtg atcgaggggg aggtggtggg cgttgagtga 60
   tcaagagttg cctcctgttc ctgaggatca gatgttgggg 100
<210> 374
   <211> 100
   <212> DNA
   <213> Corynebacterium glutamicum R
<400> 374
   ctgcagagat ggtgggcagc acccctggtg ctgtacgagt tgcccaacac cgggcgctca 60
   cgacacttcg aagcacactt gagcagcagg agaacaagta 100
<210> 375
   <211> 100
   <212> DNA
   <213> Corynebacterium glutamicum R
<400> 375
   cacctctacc aagctcaggt cgatgcgtcg atcgcgcgcc tagctgaaaa actcaatacc 60
   accatcatca atgacacctt tctgaaggag aactccgatc 100
<210> 376
   <211> 100
   <212> DNA
   <213> Corynebacterium glutamicum R
<400> 376
   ggcagaacgc taagggtcaa aacctcacta ccaccgacgc taccgcagca atccgccacg 60
   acctatccgc aatcctcgca gcactacagg agaagaaata 100
<210> 377
   <211> 135
   <212> DNA
   <213> Corynebacterium glutamicum R
<400> 377
   gtcgcacctc cttgacaggt aggtagcccg gtctacctac ctgacaacgg agggtaatac      60
   cattggcgag atcgagcaga tttccctcac acatccgcgt aaactccaag gtcaatgccc     120
   aaaaattaag gagaa                                                      135
<210> 378
   <211> 100
   <212> DNA
   <213> Corynebacterium glutamicum R
<400> 378
   gacgcgatca ggcgctccta cgcacccagg caattaggcc tgagcttatc gacgcatccc      60
   tcctcgattc caccgacctc aaagtattgg gactggacaa                           100
<210> 379
   <211> 100
   <212> DNA
   <213> Corynebacterium glutamicum R
<400> 379
   gtggcaatca acgccgcggt cgtacccaga tcccagtggt cacgcgccat ttgtgacaac      60
   gattccgtag aagttctcac cgcaattcag ggaggttaaa                           100
<210> 380
   <211> 79
   <212> DNA
   <213> Corynebacterium glutamicum R
<400> 380
   tcacgggatc ggccgctccc ggggtgggct gacgaccaag ctgcacctgg tctgcgacgg      60
   ccgcggcaga ccactgggc                                                   79
<210> 381
   <211> 262
   <212> DNA
   <213> Corynebacterium glutamicum R
<400> 381
   ctggctctga agctgggtgc tcgtgtgaca tgtcagatgg ggtattcgct ggaaggtctc      60
   actcgtatgg tcagccctcg atgtctttac ggtctgcgct tcggcaaatg gtaattgatc     120
   gatctgctgc taaagagatt ttcggtagag ggtcttttgg aagagctcag ccgtatttcg     180
   gtcagtcagc aactaactac gcttatcttc cgtgtacgat agaccgtagt taacataagg     240
   aatggaatag gagaattgcg gc                                              262
<210> 382
   <211> 100
   <212> DNA
   <213> Corynebacterium glutamicum R
<400> 382
   gccaaatact cagtcgtgcg gtattccatg tgggagagct caggatggct gtgcaaatgc      60
   ctgcgccact taatgacctc gtcatggtgg cggtccatcc                           100
<210> 383
   <211> 375
   <212> DNA
   <213> Corynebacterium glutamicum R
<400> 383
   ttatcctcct ggcctcctca gcacttcaaa cccacatatt ttcacaatag tgactaaaag      60
   gtaccgggag atgtgttctt tagatcctgg tccgattaca aatcggacac gattgacttg     120
   tctaccctac cagtatttac ccaagaaccg catggcaacc cctttttgta aggcgcatca     180
   taggtgcgaa aagctcctcc cctaccccgc ccaatagtta ccttaagtgt tggataataa     240
   aaatactggg tctgtgccac attaaagata tggcgatagt cttaaacaat acgtcgttac     300
   actggccgat ttgatacctt tcaaaacttt tactcttcat aggagtgcca ggggaactta     360
   gaggagcatt aaata                                                      375
<210> 384
   <211> 396
   <212> DNA
   <213> Corynebacterium glutamicum R
<400> 384
   ttcagggctt ttaagatgca gaggcaagga aacaatgtag tttccttgcc tattttagcg      60
   ttttcagcgg gtggtttccg tggaccagac cacgatgccc acggaaacca taaactggat     120
   tcgtgggcat cgtggtctgg tttcgagcgg gaaaaaccag acccagatgc ccaccgatcg     180
   cagatttgag gatcgtgagc acgcctgtct ggtttgcctc gataagggtg atttcatggc     240
   tgaggatcca aaaagttcga cctcccagaa tcgcttctaa gggcctaaag ttgctctccc     300
   taggccgata cccatgtgga aatctcgacg tcttaaatgg acgatgagag ctaaaaccac     360
   gaacagctgg gattttccac gataggattg ggtctc                               396
<210> 385
   <211> 100
   <212> DNA
   <213> Corynebacterium glutamicum R
<400> 385
   gaatgacctc aatcgctggc atctctggct tttcctgcat gacagatgat tgtaactgtg 60
   atgaaataag gccagatgaa gtaactgtgt cgattaagtt 100
<210> 386
   <211> 100
   <212> DNA
   <213> Corynebacterium glutamicum R
<400> 386
   gggctgaagg gctgggcgga acaataatta ttgaatctac aatcggatcg ggaactggaa 60
   tttccgcccg ttttccctat ccacaaaagg accaagataa 100
<210> 387
   <211> 78
   <212> DNA
   <213> Corynebacterium glutamicum R
<400> 387
   ggtgggtcct tttgtgaaat tcgatccaag cgggctttga gtaacatgtt accggttact 60
   gtggtgaatt gtgcgata 78
<210> 388
   <211> 100
   <212> DNA
   <213> Corynebacterium glutamicum R
<400> 388
   ggatcgacga agtgcttctt ctctacgttt gccatgccat acattctttc aaactctggg 60
   aacaaaagtc acctcaacaa ggcttaaact taacaacatc 100
<210> 389
   <211> 100
   <212> DNA
   <213> Corynebacterium glutamicum R
<400> 389
   tcaccacgtt ttcggccatg atgcttaacg acgtctcctt ctatttcttc accgctgtgg 60
   gctgcattct cgcctggtta gctggggatc ggttggcgcg 100
<210> 390
   <211> 73
   <212> DNA
   <213> Corynebacterium glutamicum R
<400> 390
   tttcatccta tgaatcttgg tgtggttcat gcgtttttat gcaatatcaa tcaaaagttg 60
   gtacgatgct cgt 73
<210> 391
   <211> 67
   <212> DNA
   <213> Corynebacterium glutamicum R
<400> 391
   agtcaactcc ttggttcaag tgtcagtatt tttctagtct atcgccccac aggtaggctc 60
   aggacac 67
<210> 392
   <211> 81
   <212> DNA
   <213> Corynebacterium glutamicum R
<400> 392
   gaaaacactt taaatattct aagaaacagt tcaagttttg ctgtagttta gatatgacaa 60
   ggcaatgaca caaggagaca a 81
<210> 393
   <211> 80
   <212> DNA
   <213> Corynebacterium glutamicum R
<400> 393
   ccccaattaa ttcgaaacaa ggaagcttcg gcttcctttt tcggcatttc actagcattt 60
   agaatttaag gaattagtta 80
<210> 394
   <211> 211
   <212> DNA
   <213> Corynebacterium glutamicum R
<400> 394
   aacttggatt tctcaagcac gctcgctgct tcttcaattt tgattgaaga ggcagcgagt 60
   tttttatcga ttttgctaaa acacgctgac aaacgagtaa gataattaga tacataaaag 120
   ctgttttagt agtttacttc tgattgtttg tcgtttacgt agacgttttc tccatgccta 180
   atccggttta aaaatgagag agcaggtaat c 211
<210> 395
   <211> 56
   <212> DNA
   <213> Corynebacterium glutamicum R
<400> 395
   ttagcccctt ggtttaactc ttcaattgct tttaactaac tgaaaggcac gcaaat 56
<210> 396
   <211> 498
   <212> DNA
   <213> Corynebacterium glutamicum R
<400> 396
   aaatttaaaa ttccataaat ttagacaatc gacaacgggg ggaaattcct tagatcgaat      60
   gggattggca tagaaagtcg cttgaaaacg cttagtggtt gaaacttgat gtaattgcgt     120
   gacttaaaaa cctatcttta cctgcagcta tggatgcgat ttcgtctttt cagatggttg     180
   tgtgtaaagt tcttaatcga accaaggagc acagcgcaca ggggttcagc acagagactt     240
   agtaagtcac ttactgaatt tgttctgaga tcacagtgtg ttagtattcg atgggtcaaa     300
   ttaaatatcg gggcgtggcg cagtttggta gcgcacctgc tttgggagca gggggtcgca     360
   ggttcaaatc ctgtcgcccc gacttgagga cgagctcggt ttcggacgac gcgagaaatc     420
   gcattaatcg tcggaaaccg ggcttgtttt tgtaatatct gaaactttcc ctttcccgat     480
   catccaggag atttactc                                                   498
<210> 397
   <211> 275
   <212> DNA
   <213> Corynebacterium glutamicum R
<400> 397
   gggagttcca cttcctcaaa gcgccttttg gctaatgtga ccccaaccag tatactgccg      60
   gtttttggat taggtttggc catcgtgcta gcatatttag gtctcggcga gggtcaagta     120
   cttttagtgc tcaaccgtta tcgacgcgat ctagacttct aaagtgcact tttgtgcgct     180
   gcctgcgaag actcgaccaa gacattcgag tcggtcgcgg gcatttttta ttttcgcggc    240
   cgagtgtcca ccttcatcca tgaggagaaa tcact                                275
<210> 398
   <211> 362
   <212> DNA
   <213> Corynebacterium glutamicum R
<400> 398
   gggacaatca atcctcgctt ccttcgctgg tgttaaccag acaggttcga acggtcccgc      60
   gcagcaacag cgcgcaatct cagcccaatg cattgggcac ccgtggggta agtcgccacc 120
   agtcaatccc gacaaccaag atttgtcaac ggttagtgca cccaagtgag gggagcagac 180
   cccagtttga aaaccaaggc acacatgtga aagaatattt cagttgactg tgcaaacccc 240
   gactcacgtt gggcaaacgt gacagcctcg aaaattgaaa cggaaacgaa ccggccgagc 300
   accccaaacc tggggaagtg ccgccagggt cctctttccc tactagtaaa aggattgttt 360
   at 362
<210> 399
   <211> 100
   <212> DNA
   <213> Corynebacterium glutamicum R
<400> 399
   gacgccaact ccatctgtgt tcatggggat aatccgcaag ctctcgcatt ggtggagaaa 60
   atcgtgacca cccttgcagc tcatgaggtt caggtttccc 100
<210> 400
   <211> 298
   <212> DNA
   <213> Corynebacterium glutamicum R
<400> 400
   accgcgattt ggtatttcgg ccgatcctgc cctaaagtaa tagaccgaag tttgaacgat 60
   cggccatgcg ccagatcgag ctcaaggttc accgaagacc gtaggtcatc cgcacagcgg 120
   atcgaaggtt tcctacccct aggaacggcc cacgcaggag acacttgaac gccttagatt 180
   ccttatgtgg aatgtatagc gccctgtgct cttgcacggg gcttttctca ttggtttatt 240
   gaccgtgtga aagctccgcg gatcagtaga ttacacataa gaggaaggag gcgaagta 298
<210> 401
   <211> 332
   <212> DNA
   <213> Corynebacterium glutamicum R
<400> 401
   tccgtttttg cccctaaaca ctctcaggtt tctcagtgaa aattgaggat tggtgtcgca 60
   tgacgctcca tttgcgcagg cagtaggtag taaatggtta gatactttta aaaaggagcc 120
   tgccacccca aataggggta aaggactggc atgtaattta caggcgcctg caaggaatag 180
   tcccaagaag tcgaaaacta gtcacattag tcacaaaaat cacttaaata acatcttgct 240
   tgatttttca tcacaaaagg cttgcacgac tataaagccc cggttaaggt gatcagagaa 300
   attcaacctc tctgaaatga aggatccagg aa 332
<210> 402
   <211> 81
   <212> DNA
   <213> Corynebacterium glutamicum R
<400> 402
   ggggaaactc tgatcgggtt gatggaaaag aatcaacctt tcactttcat ccacaattaa      60
   gttcttaata ggagaattac t                                                81
<210> 403
   <211> 113
   <212> DNA
   <213> Corynebacterium glutamicum R
<400> 403
   agtaccggtt ggacggtaat gtgcaattcg gatatatcaa tagccccgcg gtttcatgtt      60
   gaatccatgt aatcgaaaaa cacatcgggg cccacacgag gaggattatt aaa            113
<210> 404
   <211> 61
   <212> DNA
   <213> Corynebacterium glutamicum R
<400> 404
   caccccatct aaaaggggct ctctggcaga tatgctctcc ggcagaagag cctcttaatc      60
   c                                                                      61
<210> 405
   <211> 100
   <212> DNA
   <213> Corynebacterium glutamicum R
<400> 405
   tacgtgcgcc atccaccact aacttgaggt tgccaatcgt gctcatattc aactctcctt      60
   aattggtcgc tttcttctaa gaccccacat tagtaaaagc                           100
<210> 406
   <211> 331
   <212> DNA
   <213> Corynebacterium glutamicum R
<400> 406
   tgcttttcta acccctgaac tcatgggggt cgatgttacc ggctcgattg agaactcccg 60
   cattggctag aaaaacctga ttacttcacg cctctttaag taaaaaatcc tgtgtctttc 120
   taacgaaaag acccaaaaag acacgctaaa tcagcctcct atgcaattag tagagcattc 180
   acatacaccg tgccaagacc taatttccac gaccgaaact tcactaaatc cgcaggtaga 240
   agctttgatg atctacatca caaatttaca atgtgtggtg agttattcat attacccaag 300
   gacttgaacc ttaaaaggag ccctaaaaat c 331
<210> 407
   <211> 61
   <212> DNA
   <213> Corynebacterium glutamicum R
<400> 407
   gttaaaacct tcgcctaaaa cccctccacc tttcaagaca agattttaag gaagtaccac 60
   c 61
<210> 408
   <211> 422
   <212> DNA
   <213> Corynebacterium glutamicum R
<400> 408
   aagcttacgc agccgtaagt tttgagtgca gaaaattttc catgtcaagt taaactcgtt 60
   aatgaagatg gaaaataagt tgtttctaag attaaattaa ggaaagttac atttccgcag 120
   gtcggcgtta ccccctaagt ctaccccttt ccattgtgcc caaaggaaat acatatagac 180
   tttcaggtat tagattgcct ttatgaaact atagggaatc taaatccatt gatcgagact 240
   tgcagtcgcc tgactccatt tcgcttgagc cagacctcta atggttccga tctttgaatg 300
   cactacttgc tggcagtcat ctgaaaaaac gacgttggtt cgtagtcgct ggaaatttga 360
   taattcctcc gtccccttca actagggggt ggaaacccga ctatttccga aggacttttc 420
   tc 422
<210> 409
   <211> 100
   <212> DNA
   <213> Corynebacterium glutamicum R
<400> 409
   cctccgtcat cgccgacgta tcccgcggcc tcggtgaagc catggtgggc atcaacgtat 60
   ccgacgtccc agcaccacac cgactcgccg agcgcggctg 100
<210> 410
   <211> 100
   <212> DNA
   <213> Corynebacterium glutamicum R
<400> 410
   catgttcctg ggcatcgcct tcgttgaggc cctggcactg atcggccttg ttgctggctt 60
   cctgttctaa tcagctaact taaccgaaag ctggtaaacc 100
<210> 411
   <211> 100
   <212> DNA
   <213> Corynebacterium glutamicum R
<400> 411
   ttcttggaga tcagctctcc gataacgtga agcgctctgg cacgattgat cgcttcctcg 60
   ctgacctcga taccgtggca ccgaacggaa agtaggcgac 100
<210> 412
   <211> 181
   <212> DNA
   <213> Corynebacterium glutamicum R
<400> 412
   gtaagcacaa tatcggacag ctccctcacc ggcactatta cagacaacaa atgtgcgtac 60
   ttagccacct ttcaaaaggt tgtgacgtga gacatttcct ccaatacctc tccagtgata 120
   ttctgtcggt cagcctaacc taagttaatc cgttgttgtt cgagaaagag agaaactttt 180
   c 181
<210> 413
   <211> 100
   <212> DNA
   <213> Corynebacterium glutamicum R
<400> 413
   aactgctggg caccactgag attcccatgt gggatgtgga ccggtggcaa ccatcgcttc 60
   tcaagcccgg tgattcagtt cgatttgtac aggtgaagaa 100
<210> 414
   <211> 173
   <212> DNA
   <213> Corynebacterium glutamicum R
<400> 414
   aagcagcttt ggtgaaggtt tttggagaag taccaccaag ctgttaagct tccaaggtgg 60
   ttttgcgact gcggtccgca gcagttgcga aaagtacaca catcatccgg gtttatcttg 120
   gatgaatgag tgaaaatttt tcatgcggac tgaaataact tttaggagac acc 173
<210> 415
   <211> 100
   <212> DNA
   <213> Corynebacterium glutamicum R
<400> 415
   ctgctgcttc cgcaaagaag ctgcacgttt tctccggctc cgagcaccca tacgctgctc     60
   agaagcctga ggcctacgag atcaagaagg tggcccagta                          100
<210> 416
   <211> 576
   <212> DNA
   <213> Corynebacterium glutamicum R
<400> 416
   ccacaccaac ctacagcgat ttcgtggtga acctagcgaa acacctagcg accctcggga     60
   ttgggcagtg ggcagataac ggcaaatatg tgtccactac accgccaccg attttttggg   120
   gagtcatccc aggcgacacc aatgtgggct atgcgattgg tttgcaggtg taccacgacg    180
   acaccacccg cgacaactac acccgcgata ttcgtgtcca aatcctggca cgcggtgacc    240
   gtcacccagg gtcccctgcg aaaatcttgg accgcatttt caccgccatg cacgacgaat    300
   ccaactggac actaaacaac caacagtccg tcctattgtc acgtcaggac acccggccag    360
   cagcatcccg agatgcaaac aaaaactggt cgcagccagc gaattgggtg tttacactaa    420
   acccgtagga gccaaccaat ggttgcaaaa ccaagcaaga agtcactggt ctcaacactc    480
   gcccgcaata acgcactcca agtccgcaat cgtggtgaaa ctgactgggt ttacgtccgt    540
   ggcctgaccc agtgtgcacc gcagttcacc ggcgaa                              576
<210> 417
   <211> 231
   <212> DNA
   <213> Corynebacterium glutamicum R
<400> 417
   tattcctccg tgactaggct agatgacgga tccatcatat aactcgcggg agtttttggc     60
   ctgggctaga catattccat attcaatttt tagttacagc gcaacaggtg taaggtattg    120
   gaggttcatt gccaaatcat ggtgatgcat acttaccgaa cccaactcgg gcacgatctg    180
   gcatccgctg gaaccgacct gaagtttcaa tcctgaggga atcgagagta a             231
<210> 418
   <211> 366
   <212> DNA
   <213> Corynebacterium glutamicum R
<400> 418
   gaactacccc cgtttaccct caaaataagt cctgtgacac acataacacc ccctaatcgt      60
   acccgctcac acgctatttt cgaggtgtgg ttcgccttcg gaaacgaatg cccccgcccc     120
   acttggataa aagacgaatt cacctgttag tctataacgc gggttgaacc gagaaacccc     180
   tcaaggcagc agacattagc cgcaggggtt ttgcggagca cgtcccctgt gatcgttgcg     240
   ctgatgtgcg acggagtccg tagcgattac agcgagtttt tcagacgtcc atcgcaccgt     300
   gcacaacaac atttcaggtg cacggcccga acacgggaga gaacgctgag cgttacaaca     360
   ctgtcc                                                                366
<210> 419
   <211> 937
   <212> DNA
   <213> Corynebacterium glutamicum R
<400> 419
   acgcacgtac ataatccccc ggccaacagc ttacttgcgc caggggattc cctattttta      60
   tctctgaact gcaaaatctt gagtcaatgt cttcttagcc aatttaaggg aagaatcttc     120
   ctagctgggt atatgttata ggcacaagca ccggaggctc ctcagatcga ttctggaggg     180
   gtacttttca tcggctcagt cttggttttc atcgcataaa tagcgagacc aaatcaggtg     240
   gacgaaaact tctaagctgg cagtctattg gtcatcttgt acggcatcga acaccacaga     300
   ccacccccta tgaccaagaa tctcccagtt agcatccctt cggccatatg gtctgatttt     360
   ggatttcgcc atccccaaaa caaccccgaa acgcacgacc tcagtgctca gtctgcgcaa     420
   tcttccccaa aacctgcccc accaaaagaa ccctcacaca aaaaaatgac ccccaaggat     480
   tcccttggag gtcattttta aagttctggt gaacttatgc cacggttgcg gagccgccag     540
   cggcttcgat cttttccttg gcagagccgg agaacttgtt agcggtgacg ttcagcttga     600
   cgctgatgtc gccgttgcca agaaccttaa ccagttcgtt cttgcggaca agtcctgctg     660
   caacgatgtc agcaatgctg acgtcgccgc cctgtgggaa cttctctgcg agatctgcaa     720
   tgttaactac ctggtagtca accttgttag ggttcttgaa gcccttcagc ttaggaagac     780
   gcatctgcag tggcatctgg ccaccttcga atgctgcaga aacctgcttg cgtgccttgg    840
   tacccttggt accgcgacct gcagtcttac ccttggatgc ttcgcctcga ccaacgcggg    900
   tcttagcttt gtttgagccc gctgctgggc gcaaatc                              937
<210> 420
   <211> 253
   <212> DNA
   <213> Corynebacterium glutamicum R
<400> 420
   cactcctgga cttttctcag acacgtatgt ttcactcatg gttgactagt gtaatcctct 60
   cctagaattg cacctacttt ggcataggtt ttatttccaa caagctcttt gcagaacaat 120
   gctatagatt gttttctagc caatactgtg gttgtggcac tacactttcc acgagttgag 180
   gatagctttc gctatccaat cttctatctt gtttccatag taaatccgca tatttattgc 240
   caggagaaaa gtt 253
<210> 421
   <211> 100
   <212> DNA
   <213> Corynebacterium glutamicum R
<400> 421
   taccacggcc gcgttgcagc tcttgctgac gccgctcgtg aaggtggtct gaaattctaa 60
   tgatgaccat ttctaagaac atcaacggaa ggattgcgta 100
<210> 422
   <211> 100
   <212> DNA
   <213> Corynebacterium glutamicum R
<400> 422
   aaccaccacc atgcgtgcag aacgcactgg taaccctttc ttgctggcac tgtagggcta 60
   agttccgcac tacttctttg aataggtatc gttaataatc 100
<210> 423
   <211> 100
   <212> DNA
   <213> Corynebacterium glutamicum R
<400> 423
   tgtccgttac aagatcgtcc gtggcgcact ggatacccag ggtgttaagg accgcaagca 60
   ggctcgttcc cgctacggcg caaagagggg ataattaaaa 100
<210> 424
   <211> 354
   <212> DNA
   <213> Corynebacterium glutamicum R
<400> 424
   gggctagttg tgaaagctgg ctttgatggc tggcgaggtt gctgcacttt taaaaaggca      60
   aaaaacagcg aaaacacacc ccaggttttt cccgtaaccc cgctaggcta tacaatttcg     120
   gtctaaccca gtttttcaaa gaaggtcact agcttttccg ctggtcacct tctttttggt     180
   ttttcaatgc agagatagta cactttactc tttgtgtgtg gagtcaaacc tcccctttaa     240
   ggggtgcgct tggacagcag gacaaattcg ggtcaccacc ggccgccgaa tttagcttcc     300
   ttccgaacat attcctggct ggcagttcta gaccgactaa ttcaaggagt catt           354
<210> 425
   <211> 213
   <212> DNA
   <213> Corynebacterium glutamicum R
<400> 425
   aattttattt cttggtcagt taccgctaag attgttcagg ttgtctgcgc tcgaatgggc      60
   gtggcaacgt ctaacagact ttcctgcgta ttgccgccaa cgaccccggt cgtccggagg     120
   agtcacgcgc aggttaaaca agaagggctg aaccggctca ccagcacacg gtgagtgact     180
   gtactgccca gtgacctagt gaggaaaatt cac                                  213
<210> 426
   <211> 121
   <212> DNA
   <213> Corynebacterium glutamicum R
<400> 426
   gaggagggac ctttctagga ggaagtgtct ggccgattgg ctaacaatct ctagttaaat      60
   cccgcctcat gaaaccacga tgaaaccaac atgagagttt tttcatccac gttttctggc     120
   t                                                                     121
<210> 427
   <211> 225
   <212> DNA
   <213> Corynebacterium glutamicum R
<400> 427
   gacctacagg ttctgacaat ttaaatctcc ctacatctgc acaacggatg tagggagttt      60
   ttccttatat atgccctcca caaatcccct atcgtgtgag atgtgtttca taggtgcccc     120
   caacgttgcc tgttgactgc aaattttccg aaagagtcca tagactactt ctttaagtca     180
   tcagatttaa gtagtcagca ttaagtcgcc aatgaaagga catac                     225
<210> 428
   <211> 902
   <212> DNA
   <213> Corynebacterium glutamicum R
<400> 428
   ggcattgctc tggctttcgg aactagtatc ctcaacccgc tcttttcaac tgttctcacg     60
   attgcctttt ggctttccaa atctctatta gaagcagcct tcggaactac accaggaaaa    120
   atgctcacac acctgcacgt caaccacgac atacatggtg agctcctaag catctactac    180
   tcctcagcaa gaaacgcgtg gatcttgttg gagatcatcc cacctgtcgg cctagtgcta    240
   ttcagctttg tagccatttt cctcgtcgct agttgcattc acgcttgcaa cttcgtgggt    300
   ttacatgaca aatttgcaca tgtaaatgtc ttgcggaaaa acgaaaaaca ccctctcaac    360
   tgctttaaat gcaactgaga gagtgttatt attttgaggg aatgacggga atcgaacccg    420
   cgtcatcagc ttggaaggct gaggtaatag ccattatacg acattcccac ttgtgaactt    480
   ttgcaagaac tcttgtttgg ttcgaagaaa agcatagcgc atattgttgg tttattaaaa    540
   atcggcaggc tggcacccgc ttttcgaaca agatttttgg ccagaccaca ttcttaagaa    600
   tccctttcga cacatcgcta ctttttatcc ggcatctatc cagcaaatgg tcagttaacc    660
   tgcagcaagt gtattattgt acatttcctg caattccccc ttatgaaacc cgctaaacct    720
   taaagaaaca taagagaatt ttagcaagtg tgcccgtctg gccccataaa atatacaagc    780
   acttgtctat aagtccagcg tacatgggat aattttagca agatacctga aatgttctta    840
   ggttgtaaaa accttagaat aatttcgaca aaaactacct ccttggcgta acctagctaa    900
   ac                                                                   902
<210> 429
   <211> 100
   <212> DNA
   <213> Corynebacterium glutamicum R
<400> 429
   tccacgtcac agggcctcct gaccgaccgt caggctaccg agaagggcgt aggcggagaa     60
   gttctcgcct acgtctggta atagggagga ttgactaaat                          100
<210> 430
   <211> 559
   <212> DNA
   <213> Corynebacterium glutamicum R
<400> 430
   gcccctgatt cgcgcattat aaatgccctc ccgcacgctt tgcgggaggg cttttgcgtg     60
   ccaaaagata tcgcttttcg acgtctcccc accccttcgg aggggcgtaa ggtgacatgc    120
   acggcaacct tccgttaacc tgtaagacat ctcaaatctt gttaaaaacg accaattgca    180
   caccattttt tcgaccaccc cacttagtca attgtgtaaa ttacccccac attagggtat    240
   gtatgacgat tgcttattca aagtttcgct ttaatcgagc cgttattaag tagtgttaca    300
   ccttcatggg gtttagctgg aaagttttct ccttgttcac ttaaatagct aacatttctg    360
   cagttcaaga tatatttacg ggaaaaatcg tcaaataatt ctttgctgag tttggttgaa    420
   aagcaagctg ggaaattttt cccattcgcc ttgacggggt gccaaaatgt gattgaataa    480
   ttgagtgacg tcctgagggc gtcagcaaac tggcaaccat caccagcaaa atttgctggt    540
   cctctcaagg agatttctc                                                 559
<210> 431
   <211> 362
   <212> DNA
   <213> Corynebacterium glutamicum R
<400> 431
   agatcgttta gatccgaagg aaaacgtcga aaagcaattt gcttttcgac gccccacccc     60
   gcgcgtttta gcgtgtcagt aggcgcgtag ggtaagtggg gtagcggctt gttagatatc    120
   ttgaaatcgg ctttcaacag cattgatttc gatgtattta gctggccgtt accctgcgaa    180
   tgtccacagg gtagctggta gtttgaaaat caacgccgtt gcccttagga ttcagtaact    240
   ggcacatttt gtaatgcgct agatctgtgt gctcagtctt ccaggctgct gatcacagtg    300
   aaagcaaaac caattcgtgg ctgcgaaagt cgtagccacc acgaagtcca ggaggacata    360
   ca                                                                   362
<210> 432
   <211> 872
   <212> DNA
   <213> Corynebacterium glutamicum R
<400> 432
   tttctaggac ttcatctctg aaactccccg ctgtagggac ctgaatcgaa aggtctccgc 60
   atcggggagt ttttctctat tcagacgagg ctgaagataa gggagagggc tctttaacac 120
   acgaggagtg gcgtagagcc tgtagttgcc ttatatgtag cttgtggcgg cgtgaagcaa 180
   cgtgcaggcg cgtggaaagc gtagagtttt cttctcctta tatataagga gtgttcttcg 240
   tcgtgagcat tacgccctag gtcgcctggg gtcggtgtca acttcgctca aattccgctc 300
   aaaatccagc tcggtgtggc ttaagaattg ttgtcataac tccaaatctc aaagcataag 360
   ccgtcaatgg tgattaatgt cacatggtga gatcattgct gaaactggtg ccgatttttc 420
   ggctctgtga aaacgatttg actactggaa gtttcctgaa attgcaggtc atctagcttt 480
   ctcagggttc taggggagaa cccttagtgg ttggggtctg agtggaggac ttgcgtctcg 540
   gtcaaattaa tccgcgataa cggttcgata acgaccaatt ttttcgcttg ggctagacaa 600
   gtgttgttgc ggtttcgtaa ccttattgag acattgcggg acggacaccg aatttccgcc 660
   agcattacag aaacaaatag acgcttaatc gcaagcatag tttagagaaa ttctttaaat 720
   cgtgcggcga gccggggaac caaacgtgtt cctggggtga gtttcccaca agggttctct 780
   cgcagagaga gaaggagtgg ggataggggc cttccgctcc gaacccgaca gctaactcgg 840
   tcagcaaaca ggaagaattt ggagtttcat ca 872
<210> 433
   <211> 100
   <212> DNA
   <213> Corynebacterium glutamicum R
<400> 433
   ggtgaaaccg gtagtgaaac cagcggtgaa tccggtagtg aaaccagcgg tgaacgctaa 60
   aattttccga acacacccga ggggtctaga cttgcctatc 100
<210> 434
   <211> 177
   <212> DNA
   <213> Corynebacterium glutamicum R
<400> 434
   ctcagaaata agaattcccc aggcagaagt attgggatca cgccacgcaa gatcgcgggc 60
   atttgctcta aaccaggaga gcaaggctga ttgaaaagtg gtaaatgaca tgtcgaagat 120
   gattgtactg cccagtgcat taggtcatac gtcacaccga gtggaataat aaagctt 177
<210> 435
   <211> 200
   <212> DNA
   <213> Corynebacterium glutamicum R
<400> 435
   gggcgctcct tctcctgcaa attctcttgc gattcttctt gcgatatcag gactccaacc 60
   ttatatcgac cgtcgtctca cgcacgaacc atacgcgcgg ttccaaaatt ttcgaggatt 120
   tccttgcccc gggtctcgtt tatttttgaa cacgctagaa ttcaagggca gtaactaatt 180
   tcaacccggg agaaatacct 200
<210> 436
   <211> 351
   <212> DNA
   <213> Corynebacterium glutamicum R
<400> 436
   ggggagacct ccgggtggga aaacgtttgt tgactgacga ttaatcttaa atcttttggc 60
   ttgatttctt gtgctgtgcg ccgaagttca caggctagct atacagaaag tgtcaaatta 120
   gacaggtttt cgctacagaa agcgtaatgc tcatcataaa tttatgcagg ggtgacgggg 180
   ttagctaaga cctgcgcgta tgcaacgaaa atatgtgtct taagtcgcaa aggtttggct 240
   tggttaacta gctgtgttaa ctttcatctt taggtaacct aacctcacta aaactctggg 300
   aatactctgg cagttttggt ggattatttt tatagacttt caaaggacga c 351
<210> 437
   <211> 80
   <212> DNA
   <213> Corynebacterium glutamicum R
<400> 437
   ctagacattc ggacaatagt aagatgtgag ctgaaactca gtttccatcc ttattcaacc 60
   tggaaggtag ataccgcacc 80
<210> 438
   <211> 77
   <212> DNA
   <213> Corynebacterium glutamicum R
<400> 438
   gtcgccacca ggcgcaccag ttttacccca aactttttcg agcgtgctaa acgctcaaca 60
   acaggaagga tgccacc 77
<210> 439
   <211> 100
   <212> DNA
   <213> Corynebacterium glutamicum R

<400> 439
   gcggaaccgc aatcttgatt cttgtatctg ttgcactgac cacagtgaag cagattgaga     60
   gccagctcct gcaaagcaac tacgaaggac ttctaaaata                           100
<210> 440
   <211> 164
   <212> DNA
   <213> Corynebacterium glutamicum R
<400> 440
   acggacagac tactacttgg cgtgcgtaaa actcaggttg aataaaataa tgcaggtgag     60
   ccggtgattt taagacgctt ttcgacgcca ctttcaacca tttccgaacc gccaagaata    120
   ctggaatagc ttggatcaag ttttgcagga taaactgtgc aacc                     164
<210> 441
   <211> 56
   <212> DNA
   <213> Corynebacterium glutamicum R
<400> 441
   agcttttctt ttctaaaaca ttcacaaaca ctcaaaaacc acgaaaggca gggatc 56
<210> 442
   <211> 128
   <212> DNA
   <213> Corynebacterium glutamicum R
<400> 442
   aagcctattg tagggggcat ctgtttagct tcatatgacc cgaacaccac acatcacaaa     60
   ttgaatcggt atcctttggg gtattagttt ccgttttaac gacacgactt gcgaggagtc    120
   ttaaaata                                                             128
<210> 443
   <211> 135
   <212> DNA
   <213> Corynebacterium glutamicum R
<400> 443
   tccgagcagt tgtttcgcaa gaaagcccac gatttcaaca tcgtgggctt tcttgttccc     60
   cctatttagt gtggattggt ctttctgtag accacgtgca cgacaagaca atcctaaaca    120
   cgtatccttg aatgc                                                     135
<210> 444
   <211> 100
   <212> DNA
   <213> Corynebacterium glutamicum R
<400> 444
   ccacaccgtg atccgtcccg ataccccaga ggtacgtggc atgatcctgg cagttcgcca 60
   cctgatcgtc gtcgaagaag tggcggggga gtaggtaaca 100
<210> 445
   <211> 100
   <212> DNA
   <213> Corynebacterium glutamicum R
<400> 445
   atccccatcg tcatcttcta cttctccacg cagaggcaca tcatcgaagg tgtggctgcc 60
   ggtgccgtga agggttaaaa ggtttcgata ggttaaaacc 100
<210> 446
   <211> 397
   <212> DNA
   <213> Corynebacterium glutamicum R
<400> 446
   ctgattcata tgtataacga taggtcgatt gttggtgtgg tgtgcgcgag tcgactgaaa 60
   tgttcacgtg gtgaaacttc cgcgatacta ctcatgtttg cgaatttcac atttactaac 120
   tttgcaaatt gggggagggg gtagcgcggg ggaggaattc gcatgagaaa ggggaatatc 180
   ccgtgcttgt ttattcagct cgaggtggca ggcgtacact ctatattcac ggacaatgtg 240
   tacccacgct ttcttgtaag aaacaagaag ggtaacgccc cacgcgtcag tcaaaaatat 300
   ggccaacact tgcattcggg tgctggcgat catttatgag atgacgcctt gtgttggtgt 360
   tcggcagaga actcgcggag ataaaaggaa gttgaac 397
<210> 447
   <211> 100
   <212> DNA
   <213> Corynebacterium glutamicum R
<400> 447
   tggtcatcga ctccgacgga aacccaactc gcgttggcta ccgtttcgat gaaaacggca 60
   agaaggtccg cgtttctcgt cgcaatggga aggatatcta 100
<210> 448
   <211> 100
   <212> DNA
   <213> Corynebacterium glutamicum R
<400> 448
   gatccgccgc ctgcgtaagg acgatcctta caagggtaag ggcatccgct acgagggtga 60
   gcagatccgc cgcaaggtcg gaaagacggg taagtaagca 100
<210> 449
   <211> 98
   <212> DNA
   <213> Corynebacterium glutamicum R
<400> 449
   gaaccatcct tatttcggag ttgttcttta actgctgacg tgacgcccgg gggacgaaca 60
   cgaaggctga gcttgtcgat ggcgaaggat ttgaaaaa 98
<210> 450
   <211> 100
   <212> DNA
   <213> Corynebacterium glutamicum R
<400> 450
   ctccgtacgc tacttccact agcccattcg tttccgatcg caccgcaacc cgcgacggcg 60
   aaaacactca gagcaacgct taagaaggag tggcgaaaaa 100
<210> 451
   <211> 119
   <212> DNA
   <213> Corynebacterium glutamicum R
<400> 451
   atcaggaatc gacatttccg cgcgtctggt gtaccttgaa taggttgctg acgcaacgac 60
   cctcctgcta tgccaacgac ggcatggccg aaaaaacaat tactagacca taggaggtg 119
<210> 452
   <211> 100
   <212> DNA
   <213> Corynebacterium glutamicum R
<400> 452
   ttcgctgcca agccagccac cttggagtcc gtattcatgg acatcgcctc actccagaac 60
   acctcgctgc aaaccgccta gaatctttaa ggagcccaca 100
<210> 453
   <211> 54
   <212> DNA
   <213> Corynebacterium glutamicum R
<400> 453
   accctactta gacctgactt agtgtgggaa aatttccagg gtagaatgcg acga 54
<210> 454
   <211> 421
   <212> DNA
   <213> Corynebacterium glutamicum R
<400> 454
   acgtttgatg cacctccagt gggatttatg ccaagaacgc tcaatgaata cttaatcaag 60
   aaagttcggc gggttaactg ctaaaagtag tgtacgtgcg catgacgatt cgggcgcgaa 120
   ggcgcgtcga aatgggttta atccggcgat atttcgcctt taatccagca cggataaaaa 180
   gttctgacat ttgacgtgac atatgccata ccaacgtgca tacttattca aagtgggcgc 240
   gaattccgca agattttctc cgaacggaca gaaaagcatg acccgatcct gtttgtttcc 300
   gtaggaagta ctattgttta acgcgtacac gtgctcaaca cgacaacgct taaacggctg 360
   cacgcgtaac acggcagacc gcacaagctt taagatccac gatcaggaga ctttgacaaa 420
   t 421
<210> 455
   <211> 267
   <212> DNA
   <213> Corynebacterium glutamicum R
<400> 455
   tagctaaact gcaccactta ccccgcattt cctaggccac atataagggc tttggtgatg 60
   cggggttttg cgtgtaaggt agacaatcgc gtgtttttta agcatgctca aaatcattca 120
   tccccggtgg cccggttacg taaagatcgg caaagatgat caactaaagc gatcatctga 180
   agttgtagcg ggaccgagca tccggacggt tactagtggg gtttcatcgt cccagttgtg 240
   gccggtaaca aggaagcagg tttaacg 267
<210> 456
   <211> 88
   <212> DNA
   <213> Corynebacterium glutamicum R
<400> 456
   gtgtcatatc gtaccgtttg cataagcctg ttcgcgcttg gtgaaccttt tctagcacca 60
   aaacaaaact ctccctagta tggggtcc 88
<210> 457
   <211> 518
   <212> DNA
   <213> Corynebacterium glutamicum R
<400> 457
   gaatgaaaaa tcctcctgaa ataaaaggcg ccttaaatcg caaagaaatt ttgttggaag 60
   aaataagacg ctgcatttgt taaatctcgt gtcaacgata cggcgaagtt acatctgagg 120
   tgaaaagggc acgccaaaat tgacgaaagc tccctcaaac aacgtgcatc agctgactat 180
   tgcagcatcc tcaaaggttc ctgaaaacca gattgatttc ctaatacatg caccttgtag 240
   gaacgtaggg ggtaagggtg ggggaatttc aagggcaatc aaaaggttga tggtctgtga 300
   cgtggcatac accaattgcc tagactttag gtattccacc tgaggattcg ggcatatcgt 360
   tgcagttgaa agacatttga cgcccctaaa aacgaaaccc acgaagatat ttccaccaaa 420
   cacaagatat ggaatcggct ggcaaatagg ctattctgcg aagatagaaa tgaccgtaag 480
   gtctctggtt tttgtgtgga caggaaggca gaacacac 518
<210> 458
   <211> 66
   <212> DNA
   <213> Corynebacterium glutamicum R
<400> 458
   gattcttaaa acttttaaga atcagccaga aacattttga ttgaacagga acaaagagaa 60
   caaaac 66
<210> 459
   <211> 84
   <212> DNA
   <213> Corynebacterium glutamicum R
<400> 459
   tctaggtgag ctaatcggtc tgcgcgtttt tcaagcactt tgcgcagacc cccatccacg 60
   taatccacga gggagatcac atcc 84
<210> 460
   <211> 188
   <212> DNA
   <213> Corynebacterium glutamicum R
<400> 460
   aaaaaattgc ttttcgacgc ttccctccac ccttgaacaa tccacattta agatttatgc     60
   cccttttgtt tgcccccgtt tacaccctcc gtttaatcaa ccgactgtgt gaatgcgctt    120
   aaacggacta gggttgtgag cagcatatcc agatttttct ggataaatcc tggaatttct 180
   taaaacca 188
<210> 461
   <211> 327
   <212> DNA
   <213> Corynebacterium glutamicum R
<400> 461
   gacatctacc ttctaaaata ggcgactact taaggtggaa aaacaagctg attgttatat 60
   cgcgaatcac acctatattg ttccttatta acacgcaaca cggatttgaa ttcgggggta 120
   ggggtgtggt tagatataca ggttgtctgt gtaggtcgga ctcgcggtgc ttttccgagt 180
   cgcgtttccg agacccctcg acgaggctgt aaaaggccaa gcgttcgttg cccacattcg 240
   tggagtatgt agacatctgt agtacaaaga ccacgcgtgt ttgggacgga aatccagcac 300
   gcattaatcc aggtcaggag accagta 327
<210> 462
   <211> 96
   <212> DNA
   <213> Corynebacterium glutamicum R
<400> 462
   aagcttgctt ctcgacgcaa aaacccatcc ggcgcatccc ttcaatgtta ggggtgcgct 60
   gctatttttc tccccagttc tacgaaatga cttatt 96
<210> 463
   <211> 194
   <212> DNA
   <213> Corynebacterium glutamicum R
<400> 463
   gtaggtggtg ggagccccaa agttgcggaa aattgttcca actaagggac tatatgtagg 60
   tgtgggtaac ctaagttaat cttttgtggg cgtgaggatt tctctgagga atctagacgc 120
   agattaactt ccgcttggca gcgaccggga taacaccgcg gttgcggcca cgcaggctca 180
   caaaggacac cact 194
<210> 464
   <211> 242
   <212> DNA
   <213> Corynebacterium glutamicum R
<400> 464
   cgctttagtc tccttaactg ttggcccttt gaattacttt taggccggga catcataggc 60
   ttgcagtgta ctcccctttt tacggatctc cggcgagcga tgctggatta cgttcatatg 120
   ggaagcggat ggatgttccc cagcctactc accgtccaca gatgagtaaa cccggaaaaa 180
   cccgtattta gttattggtt ttacctgcgt gggctgaaag tcttcacttt taatccttac 240
   ag 242
<210> 465
   <211> 871
   <212> DNA
   <213> Corynebacterium glutamicum R
<400> 465
   ttctgctcct cctgtgccgc accggaagcg cggttgatga aggtgtggag agcctcaacg      60
   gagaacacaa cgtcgtcaga cttgagaacg tcgtaggtgt tcagctgatc agcggccagg     120
   atgtggacgc caggcaggtt gttagcactc ttctgggcgt tgatatcctc acggctcact     180
   acgagcagca cggacttacg ctcggtcaga cgctcgatga aagcctttgc agacttggtc     240
   gaaggggtct ggccaggcac caattcggag acgacgtgga tacgtgcatt gcgtgcacga     300
   tcagacagtg caccgtaaag tgcagccttg atcatcttct taggggtgcg ctgagagtag     360
   tcgcgtggct tagggccgtg ggagatgcca ccaccggtga agtgaggtgc gcggatcgag     420
   ccctgacgag cgcgaccggt tcccttctga cggaatggct tacggccacc gccacgtact     480
   tcgccacggg tcttggtgga gtgggtgccc tgtcgagctg ctgcaagctg tgcgttgaca     540
   acctggtgca gcagtgcgac ggagacctca cggtcaaaaa tctctgcagg gagttctaca     600
   gatccgttga tgtttccatc agcggtctga acatccagct tcagattcgt catgcgtgtg     660
   caccgccctt cactgcggtc ttaacggtaa cgatgccacc acggttacca gggattgcgc     720
   ccttgataag gatgatgttg gcatcggcgt caatcttctg aaccttgagg ttctgggtgg     780
   tgacgcggtc attacccatg cggccagcca tacgcttgcc cttgaagatg cgacctgggg     840
   tagcagctgc accaatgcca cctacgcggc g                                    871
<210> 466
   <211> 100
   <212> DNA
   <213> Corynebacterium glutamicum R
<400> 466
   cgccgaagac tgttgatgcc cttatgcgca tcgaccttcc ggccagcgtc gacgtgaaca      60
   ttcagtgatc gacggaattt ttggcagcgg agaataaata                           100
<210> 467
   <211> 243
   <212> DNA
   <213> Corynebacterium glutamicum R
<400> 467
   tcttgcactt gttgggtaaa cgcacctact acacccattt ggggcaatcc aaacggatgg     60
   gtttcaggtt atgcctgcat gatgactgcg aaagcgcagt tggaaacctc acaacaccac    120
   tggttttttc gcttgaccgc gcaaaatatg agaacatagt gagagttaaa ccaagttctg    180
   taggtgcttg ttgcagcggg cgcgaaggcg taccactgca acttgcgaat aaaggagtaa    240
   aac                                                                  243
<210> 468
   <211> 141
   <212> DNA
   <213> Corynebacterium glutamicum R
<400> 468
   gtcctgtggt gtccttattg gggttgtcct gcaagtttta gaaatcatag cgtgagatga     60
   caaggaaagg aatatttcga tccggcaaca acccccgcgt caggtgggat tatgccgaaa    120
   aaatcgctac gatagccact t                                              141
<210> 469
   <211> 422
   <212> DNA
   <213> Corynebacterium glutamicum R
<400> 469
   cagtttcact gccaattttt taagcctaac cctctgcata attttgtttg agccgtgtaa     60
   tatacacggc ttttctgctt tcaggccaac tttccctccg caaattcacg aaagtttgcg    120
   caatcgtttt cgcccacacc ttccccagca cacattccgt tcacactatt tcgggggtag    180
   ggcaaataac gattcatctc acaaatttct atcaaactat agaaagatat ttgcaaaatt    240
   ggcacaaact cccagctcgc tccgaaaaac ctagcaactt agttaatttt ccctcacaaa    300
   aatttaggac acagatctcc attcaagttt ggctgtgact catgtcgcac atagtatttc    360
   aagcacagga tcccactcgg ctgaacagct caggggaata ttcataacaa cggaggtcaa    420
   tc                                                                   422
<210> 470
   <211> 410
   <212> DNA
   <213> Corynebacterium glutamicum R
<400> 470
   agcctccttc cacatggcgt ggcctgacaa atgcggtgag attaatttgt acattgggcc 60
   aggttagcag cgctaaccgt gccccgcgtg gagcgcgcgc gggaggctga tggagggcgg 120
   cgtcgataag cgtgctagga agtttgtgtt ttagtgttca cttgctgtac tctgtccgtt 180
   tggactttgc accagaaaac tctatagagc acaacggtgt tttaaagatc catgtaaacg 240
   taaacaccta ctttgttgta ggccccccgc catgaatggc gatccgtgtg ggaaatggca 300
   gcgagcaaac cgactattga gttcggtgag cgttgagtgg ccagaaaaca cacggttaac 360
   gtccaaggtg ggaagggaac cccaacgaga aaggcatcag gtcgtctcta 410
<210> 471
   <211> 100
   <212> DNA
   <213> Corynebacterium glutamicum R
<400> 471
   aatccaaacg tgcgcttccg cggcgtgtac tttaacccgg agtcatttga tcagatgtgg 60
   cagaccaagc aagtgggacc tttcgtggtg gtgacgcaca 100
<210> 472
   <211> 100
   <212> DNA
   <213> Corynebacterium glutamicum R
<400> 472
   gacgagctgg gcattgctca gacccgtcgt cttcgtggac tgggtgaccg tcagcgtcgc 60
   gcacttctcg agcgtttcgg cttcgaggat taattcttca 100
<210> 473
   <211> 335
   <212> DNA
   <213> Corynebacterium glutamicum R
<400> 473
   tgatcctttc gacatcccag aaccgaaaag tatcccggcg aagatggcag cttctgctgt 60
   tcgcttctac caaaagtatc tttcgggtct taagatgggt tccacctgtc gttttgatcc 120
   agtttgcagc acctatgcat tgaaagccgt ctcagttcat ggggctttta agggaactat 180
   tctctccgct gcaaggttgt ccaaatgtgg gccctggcat ccgggtggat tcgacccggt 240
   gccaaaccat ggattttggt ccaccgaaac ggtgacatag aagtcctgcc aacaagtaca 300
   tagtccctat taatcccaag gagtttcgac tcaca 335
<210> 474
   <211> 61
   <212> DNA
   <213> Corynebacterium glutamicum R
<400> 474
   agacacgacg aattagacaa cattagtaat gctggaagaa acaaccgaga gcaggaagaa      60
   c                                                                      61
<210> 475
   <211> 100
   <212> DNA
   <213> Corynebacterium glutamicum R
<400> 475
   ggaaaaagga atcataaagc cacagcgcaa agccactgaa tcaataaaga agcgttaata      60
   aagtttgact tgtgcctctg acctgcgttg acttgagtaa                           100
<210> 476
   <211> 100
   <212> DNA
   <213> Corynebacterium glutamicum R
<400> 476
   cagggacacc gtcagcccct gaccgttctg aaggtaaccg gaatcaagta agccctcggg      60
   ctttctatga gtttttcctt tagaaggagg gaattaccac                           100
<210> 477
   <211> 91
   <212> DNA
   <213> Corynebacterium glutamicum R
<400> 477
   tcccctatcc taatccacat ctcggtttat taaactgtta gtgaattccg atgtataaca      60
   ctggtccgtt tcaggtgaac ccgtatggtt a                                     91
<210> 478
   <211> 117
   <212> DNA
   <213> Corynebacterium glutamicum R
<400> 478
   tttttgctta cccggctgcc ccgggtgcac aggaatcttt cgattttcca aacatccttc      60
   atggttttaa aaacctttac cgacgtcaaa tagcggtcgt cactcaagga gagttca        117
<210> 479
   <211> 100
   <212> DNA
   <213> Corynebacterium glutamicum R
<400> 479
   attcgctaaa cctgcgcagg atgagactgc cctcgcagaa agcacattcg acgaagccac 60
   cgcgtaaaca gtacgtggtg gaagcttgag aggaagacaa 100
<210> 480
   <211> 269
   <212> DNA
   <213> Corynebacterium glutamicum R
<400> 480
   aacgcgatga tcttaaggat ctgaatccgg taacacgaca gaaattggtg ccctttaggt 60
   ttcatcattt gaccttggcg tatactgttt tatcagttcc aactaccaac tccgtttcca 120
   tggaagctac ccggctttcg ccaccgcaac gtgagtccaa aattttcaca cggtgcacca 180
   cacatttagt ggtgtattga ggtgtttctg ggctgcgtga gaaaccattt tccggtggat 240
   gatggaagct agacgacgaa agggagcat 269
<210> 481
   <211> 100
   <212> DNA
   <213> Corynebacterium glutamicum R
<400> 481
   cgatcgtgtt ctgaacctga atgatggtgt cctgcgcacc aaggttctgc gactcgacaa 60
   gtaaagaact ttaaggctct agagaggtag ttgaaggatt 100
<210> 482
   <211> 126
   <212> DNA
   <213> Corynebacterium glutamicum R
<400> 482
   aagctttcac accggttagt ggctcattca aaatgtatgg ccaccaacca attttcacca 60
   aagttttatg tggcagggcc agctccggcc cgttaaacca cagaattcca tgaaagggaa 120
   tttcta 126
<210> 483
   <211> 100
   <212> DNA
   <213> Corynebacterium glutamicum R
<400> 483
   tgcaggtcgt gcaattgttg cggctcgtcg tcgcaagggt cgcgcaaagc tgaccgcgta 60
   attttttagc gtcaccacaa taaataaggt gtaagcaaca 100
<210> 484
   <211> 754
   <212> DNA
   <213> Corynebacterium glutamicum R
<400> 484
   tttttatcct tttcatgtgt tggaaacaga gtcatggtag gtcgccttat ttgtcagtgc     60
   aagggtttta ttagcttttg gagatagttt ctggctggtt gaaatagttg acatctgtgc    120
   aggttggagg cttttgtttc cgacttgggg ttatgtgaag tggggtgctg tgtggcgttt    180
   taagcggtcg actgtctgcg gcgacctgtt gtcctagttt tggcgagttg ggtgcttaga    240
   agcggtttta gagggggtcg aatttgtgac gattggggct attttgattt ctgctgggtt    300
   gctgtgggct tgctgatcaa cttctcgttc caacggggcc tcacgccctc aaacggggac    360
   gagttttcgc ataatacccc gtgattagcg caaattttcg actaaaaatg gccttttccc    420
   tctggctggg gacggatttt cgcaacttca ggcagtcacg tgtcgagcgg gacatggatg    480
   actcaatggg taaggggatt gtggtgcctg tcggatggcg atggttagga ctcaccattt    540
   ttaggatgcc acctcgaaaa aacggtgaat cggcacgctt ttaacccctc gaaagtgtgc    600
   caattcaaca tattctgcgc ccaaccttca aaaaatggtg aatcctaaca ggcttagggg    660
   gcttgcaagg cgggactcat gccgggggta tgccttgggg cagttattaa ggataacctt    720
   gccttaatta tttggattgc agtaatttaa atga                                754
<210> 485
   <211> 55
   <212> DNA
   <213> Corynebacterium glutamicum R
<400> 485
   gtcgcatggt ccaattcatg ggccgcgcta aatcaacgta caaggagtac atcta 55
<210> 486
   <211> 239
   <212> DNA
   <213> Corynebacterium glutamicum R
<400> 486
   gctgccctta actgcagttt ctgcagttag ctgaccaatg taaagccccc acgacaatgg     60
   aactttgact tttaaaattt catcgccgtg ggggcttttg ggcagccagc ccgccgtgtc    120
   ccaacgtaat cgactgaata cctgtacgat cactttttag acgggcgggt agggctactg    180
   tgccctaacc taagcttgta aagcattaat tatccataca taaggaggat cgccccgta     239
<210> 487
   <211> 347
   <212> DNA
   <213> Corynebacterium glutamicum R
<400> 487
   tactgtcaag acatgaaagc ccaatcacct ttaagatcaa cgcctgccgg cgcccttcac     60
   atttgaataa gctggcagcc tgcgtttctc caaggcgact gggcttttag tctcattaat    120
   gcagttcacc gctgtaagat agctaaatag aaacactgtt tcggcagtgt gttactaaaa    180
   aatccatgtc acttgcctcg agcgtgctgc ttgaatcgca agttagtggc aaaatgtaac    240
   aagagaatta tccgtaggtg acaaactttt taatacttgg gtatctgtca tggatacccc    300
   ggtaataaat aagtgaatta ccgtaaccaa caagttgggg taccact                  347
<210> 488
   <211> 179
   <212> DNA
   <213> Corynebacterium glutamicum R
<400> 488
   acagcaatta atctgattgc acctgctgca taaatgtgac tagtcaaaca ccgtctaatt     60
   acatgtgtgt ggtagaacaa taatgtagtt gtctgcccaa ccgagtgaca ctcccacgat    120
   ttacagtggg ggcagacatc ttttcaccaa aatttttacg aaaggcgaga ttttctccc     179
<210> 489
   <211> 100
   <212> DNA
   <213> Corynebacterium glutamicum R
<400> 489
   tggcgatacc agctccgagt agggctgcac ttgaaaaagt gagtgtttta gcgatagttg     60
   acaccttatt caccctacct ggggagtact ctgggcaaac                          100
<210> 490
   <211> 183
   <212> DNA
   <213> Corynebacterium glutamicum R
<400> 490
   aatgggacga gtgtgatttg gggcagattg gaactgcctg tgaaccctct aacctgcaat     60
   tatgtaactg tcgtaaaagg gctacgaaga gttcccggaa ggtcgattga aaagtttgcg    120
   aattggggga aaattcgcat caaaagccga gttcaaactt tcaattgaaa cggggggctt    180
   gaa                                                                  183
<210> 491
   <211> 108
   <212> DNA
   <213> Corynebacterium glutamicum R
<400> 491
   tacttggccg ttataaagac aaaaacaccc ctggtggatt cccaaaagga aattccacca 60
   ggggtgttaa gcattcgctt gaagcgaata gcaagaggtc atcagtag 108
<210> 492
   <211> 240
   <212> DNA
   <213> Corynebacterium glutamicum R
<400> 492
   gcttccacgt ctctttcttc aaattaaact gttaaaacca aaggtggccg gcaccgcaca 60
   atgcatgcgg ttccggccac cttgaggtga gtgcttccac gtcactgctt gctatctacc 120
   tatcatcgta gattcagcag cacgagagaa gaaatcttat ttgcccagtg ccttgagggt 180
   acgagcgttg atctcttcga cttcgccttc agcctcaatg ttgatgatct tgtcaccgta 240
<210> 493
   <211> 364
   <212> DNA
   <213> Corynebacterium glutamicum R
<400> 493
   cctctcgcgt gccccaacgg ctcctacgat caaaaatcct ccctgcttcc ctcaactgtt 60
   tttatatgta tttgtatgtt ttaggcccga ttgccccata attggggagg gtttcaatgt 120
   ccgaatgaag gaaattgtca caatcgggtg ttttgggtcc gatttctttt gaatatgtgg 180
   gttacctcac taaggtggaa ggaaggctaa ccagatcata gtcggactgc aatccgctat 240
   gaagtacttg gtggcgctgg gaagaagcct tcgttatggg aggtctccca gacacaatcg 300
   aatacgggcc ggatatccat ctcggctcat caccccgctt tttaccaaga aagatgagga 360
   cctc 364
<210> 494
   <211> 100
   <212> DNA
   <213> Corynebacterium glutamicum R
<400> 494
   agtaggggat catccagcga agcggtcgca gaggctgtga ggaagctcta agtcgactta 60
   agtgcgcgaa gcagaccacc attaggtaga atcacccaac 100
<210> 495
   <211> 610
   <212> DNA
   <213> Corynebacterium glutamicum R
<400> 495
   ggcaacgacc tcccagcccc ctgctcagac aaatcatcat caggaggcgt caaaccatgc 60
   gtcgcatacc aaccagggcc atgcctaacc cacgcattca caatcgcacg gttacacgca 120
   gcatgaatca aacgcttagg ctcattcgac aagtcagcat tgagatgatc cgcatgcaca 180
   gtcctacgat caaaattgtt ttcagctttc cgatacatcg ggcgcccgca atactcacac 240
   aaatcaccat cacgaagatt gaaccgaaga cgctcgcgag cccgtttgtg ttgcgctttg 300
   ttgtactgaa cggccattcc acacctccta agtcggtttc cgcagctcag ggagagagag 360
   ctggaaagat cactcagagg ggcggagtgc cgttcgaaag gactcagata aaacgacccg 420
   ccaggtttgc cgaagatgcg cggacagatg gcgttagccc atggcacggg ctatccagat 480
   tgatcaggac tctctcaaga agtgattccc aatactccca tctttatgca ggtatgctat 540
   ccacatgaca ataatcaacg ctgatagttc agttatagaa gaagtaaaaa ggcaatttgc 600
   aaacagattt 610
<210> 496
   <211> 375
   <212> DNA
   <213> Corynebacterium glutamicum R
<400> 496
   tatttaatgc tcctctaagt tcccctggca ctcctatgaa gagtaaaagt tttgaaaggt 60
   atcaaatcgg ccagtgtaac gacgtattgt ttaagactat cgccatatct ttaatgtggc 120
   acagacccag tatttttatt atccaacact taaggtaact attgggcggg gtaggggagg 180
   agcttttcgc acctatgatg cgccttacaa aaaggggttg ccatgcggtt cttgggtaaa 240
   tactggtagg gtagacaagt caatcgtgtc cgatttgtaa tcggaccagg atctaaagaa 300
   cacatctccc ggtacctttt agtcactatt gtgaaaatat gtgggtttga agtgctgagg 360
   aggccaggag gataa 375
<210> 497
   <211> 100
   <212> DNA
   <213> Corynebacterium glutamicum R
<400> 497
   tggagatcgg ttcaatctcc gacgtgaccc cacagccaca caacggctgc cgtccaccaa 60
   agcgtcgtcg cgtttaatag ggaaggaaag gtaatacaaa 100
<210> 498
   <211> 479
   <212> DNA
   <213> Corynebacterium glutamicum R
<400> 498
   tttcttttaa gtttcgatgc cccggtttcc tgattttgtg cagggaggcc ggggcattgg 60
   tgtttgcggg ttagttcggg ccatccgaaa ggaagaaacc aagggcggcc agacagacgt 120
   gccaagaatc tggatttccg ccaggttttg gcacgcccgt ctggtttagg cagtgagata 180
   ccggacacac gtgccaaaac ttcggctttt tcgccaatct tgtcacgtct gtctggtttg 240
   ccttgaaaag ggtgatttca tggccgagac tcctaaaagt ttgacctcac aggattgctt 300
   ctaagggcct aaagccggta ttcctaggcc atagcccttc ggggaattcg ggcgcttaaa 360
   tcgagaaatt aggccatcaa cttttaataa caatccaatg aataattgga ataggtcgac 420
   acctttggag cggagccggt taaaattggc agcattcacc gaaagaaaag gagaaccac 479
<210> 499
   <211> 1000
   <212> DNA
   <213> Corynebacterium glutamicum R
<400> 499
   ccctacgtat taccgcggct gctggcacgt agttagccgg tgcttcttct ccaggtaccg 60
   tcaccatagg cttcgtccct agcgaaagga gtttacaacc cgaaggccgt catcccccac 120
   gcggcgtcgc tgcatcaggc ttgcgcccat tgtgcaatat tccccactgc tgcctcccgt 180
   aggagtctgg gccgtgtctc agtcccaatg tggccgtaca ccctctcagg ccggctaccc 240
   gtcgacgcct tggtaggcca ttaccccacc aacaagctga taggccgcag gctcatccca 300
   caccgcataa agctttccac accaccccta cgatgatgtg aatattcggt attagaccca 360
   gtttcccagg cttatcccaa agtgtagggc agatcaccca cgtgttactc acccgttcgc 420
   cactcatcca ccaaatgcaa gcatctggtt tcagcgttcg acttgcatgt gttaagcacg 480
   ccgccagcgt tcgtcctgag ccaggatcaa actctccaca aaaaaacaat aaaaaagatt 540
   gtttaggccg tgaaaagccc aacacctgac aaaaacaaca acacccccac caaccattag 600
   aaaatgatca gataatgggg tgttactggc attatcaaaa aaataatgat ttaaaaaaaa 660
   caccacaacc cacataagga tcatcgtgtc tttaatgtgg gcctgatccc ttgacgggga 720
   acaagcagac ccacaaaaat catgccaaca ataagtttat tgtttacagt gatggataat 780
   attggcgatc caccacccgg catgacccac caccaaaacc ctcaaggggt taaaagtggt 840
   agtgacaaaa ataataaatg gcacactatt gagttctcaa acaacatacg cacactagga 900
   aattgaggta atatttgcac cttaatctct ttagcagctg aagattcatc gtagttcatt 960
   caaaatttga agtcaacttc gaattaatct ccgtgtcacc 1000
<210> 500
   <211> 205
   <212> DNA
   <213> Corynebacterium glutamicum R
<400> 500
   tcgcggaaac ggcccttaat tgcaagcctg tgacctgcag gtttagcttt taggggtcct      60
   gccacctttg tgtttgcgaa agtagtgcta gactattcga aaatcgcagg ctgacatcct     120
   tggtattaac caggtgtact ctcgatttct ggatactttg gtattcattt tgtcactaaa     180
   aaccacacga taacggagga acccc                                           205
<210> 501
   <211> 1000
   <212> DNA
   <213> Corynebacterium glutamicum R
<400> 501
   caccatagtc accgctgtaa tattcctcca caatctggtg cagataggag tcgcggaagt      60
   agttatggtc taagcacgtc gcaatcgcat caataaagct cactcgctgc ccattaggca     120
   tccgtgtact tcgcacctgt cggacatatg catcggtgat attcgacttg gaagaggtag     180
   gcaccctaat aagaattggt agcgtcaggt gctgattcaa atgggactgt atcgctgaat     240
   agccatccaa ggctatattt tttcgacgcg acttgagggc ccaccacact gaatctcctg     300
   ttacttcacc cgagatatat acaccacgaa aaattcgagt cagttgttgc tcgtgaacca     360
   acgattcaat ctgtcgctta gttttccctg agtcaataag ttgttggtag gtccaaattc     420
   ttcttgccat actcaggtta gactgctgga aaggcctaaa ggttcccaat tttcctgccg     480
   atgtgaattc gacaccaaga tttcctgatt ttgaccccca ttttgccaat atcttggtgc     540
   cgaaagtacg cacggaagag agtccaaacg gacaaaaacc acccttccga agaagggtgg     600
   ttttaaatgg tgcccggggg gggacttgaa cccccacgtc cttgcgaaca ctggcacctg     660
   aagccagcgc gtctgccaat tccgccaccc gggcagggtg tcttttgcga cttgttaaag     720
   atagcacgca cttggcgttg aatgccaaat cggcagctaa atagcggttt cctgaatggc     780
   aacaaagttg taaatttatg cattccggtg gtttggccta taactctggc gcggttcacc     840
   tctataatga cgctatcgtg cgctttggtt tagaccgtgt gaaggttttt ctaaatgaaa     900
   tttcagtcac agggagggac aaagccatgc ccttaggcgt tttctaggag aagacactta     960
   tggaacaccc aacttattga gaaaaggagg tcgcaaagtt                          1000
<210> 502
   <211> 100
   <212> DNA
   <213> Corynebacterium glutamicum R
<400> 502
   gtgtggccaa ccaggcacgt caggcacaga tcacccagga aatcacagag attgttggtg 60
   gcgcaggcgc gctcgccgac agcggagaaa gtgactaatt 100
<210> 503
   <211> 647
   <212> DNA
   <213> Corynebacterium glutamicum R
<400> 503
   acgcaatagt tgcattttta gttaacatcg agctgcaaca acagcaataa acactcaatt 60
   acatgacaga aatgatctgg tctcactccc gtccagtgtg agttcgagtc tcaccagggg 120
   caccactacc agcggaaact ccgacttgga gaagtgattt cggccaagat gttctagaaa 180
   tcaaatccca ctgcggttaa agcagtggga tttcttcatt tttggggtaa ctcgacaaaa 240
   atcgcccaga aacttggggg tagtttcaag atcgactagt gcaatctccc caaaccggac 300
   acacgtgaca aaactttgga tttcggcctt ctcttgtcac ccctggctgg tttcgaccac 360
   ttataaccag tctggtctgc ccgagttgat gcttcgatgc cgaaaaatca tgaggccaca 420
   gaatccaatc taacggcctg taaaacctct ccccgactat ttgcccgtgc gaagcattag 480
   agccttcaaa ttggccacaa atgcgtctga gttgagagac ccaactttct gcaccctacg 540
   cccagttttc agcacagatc accctttgcc agctaccgac ttagtttcgt aacacgtata 600
   gtgggaggcg ttttgccaga cgccaaagaa agataattgg attacct 647
<210> 504
   <211> 100
   <212> DNA
   <213> Corynebacterium glutamicum R
<400> 504
   gaagaggtcg aagaagacga cgcagaggat cctgaagaga atcctgatga ggaagaatcc 60
   gacgaagaaa ttgagccaga aactgaggct gaagaaacca 100
<210> 505
   <211> 225
   <212> DNA
   <213> Corynebacterium glutamicum R
<400> 505
   ggtataaggg cggtttgtgt tgaagccgcc tttaggggta accttggaca gtcgctgttt 60
   taggcaatgt cagtaatgcc catttgccca tttcacggcg aatgtgtgaa tgacgtgagg 120
   tcattttggc tccgcatgtc taaaacgcaa ttgtaaaacg taagtccaat cagggactca 180
   tcatccgagt acctgagccg agaatcagat aaggggtagc cctct 225
<210> 506
   <211> 100
   <212> DNA
   <213> Corynebacterium glutamicum R
<400> 506
   atgcacccgc agagcgtcgc ggccgcggcg accgcaacgc acgtccgcgt cgtggtggcc     60
   agcgtcgtca gcgtgctgag cagaagcagg agggctaaac                         100
<210> 507
   <211> 401
   <212> DNA
   <213> Corynebacterium glutamicum R
<400> 507
   tcgcacggaa atcttcatcc taatccgatt tagctaagtt atataggtat gagactatgg     60
   ctgacttggt gctttattaa tttccccaac gactagggct gaaccccacg tttaaagtca    120
   ttcacacttc ttcagcacat tttcagccct gcccagccaa agccttctgg acctttaatt    180
   atcagagcat tttccaaatc ccctgtggca aaaaatagat caataccgta aaatacgtga    240
   gaaaaaatta tataattcac agtgaatcta tggagtggaa caagcctctg acttttgttt    300
   tatttgcaag cacatgatgg acattacagc tactgctggt gcatccattg gggtggggcc    360
   cacgcagtcg ctagaaaaca ttgcaagtta aggaatacac t                        401
<210> 508
   <211> 643
   <212> DNA
   <213> Corynebacterium glutamicum R
<400> 508
   tgtgttcaac cttcttaaaa agttttgggt gggtccacga ccggcaacac caaactccgc     60
   gacgggatgc cggtcgtgtt aagacctctg ggacccgccg cggcgaagaa gaagtagatt    120
   cgcacgcgaa gtcatgtggt gaagcataca acaactttgt ggtgtgggta gtaactcggg    180
   ggggagtttt cttttaaaaa gcttttcgac gcgcagttcc ggtgctgtca tgtttcgggg    240
   gaaacattac gagaatggaa tagctcgatg gggaaaattt gcagaatgtg tctgaaatca    300
   caatagggtt cgattgcgga atcctgagaa ttgtgaaaac ttttgcggaa tctcaaccgc    360
   ttttatgtgt gtgaaaatgt ctaatcctgc ctggttgtgt gatgttttgt gggcaaatta    420
   agagaattta acccttttaa cgtggttatt tctgagaggt agataagttt ccatgttgat    480
   tagttggcaa attcttatga atcgattgtg agaactattt atgtggtcgt taccctccca    540
   tctggcaaag gtgttgatgc gaaaactcgc gaaatcactt gactttttga gtttgcagtg    600
   ggagagcgtc aaaccccaat cttctacgaa aggaattttc acg                      643
<210> 509
   <211> 464
   <212> DNA
   <213> Corynebacterium glutamicum R
<400> 509
   ggcccctaac gtacacaaat tccgcaaggc tgtcagcata ggttagcgca gcataacccg 60
   catttttggg ctttggattg gctaagtttc ccaccttttt gaaggcttga gaatggccca 120
   gtttgagacc tgtttcacat taaaggtttt ctctaaatga cgtgctagtg gcgataattt 180
   agggcaataa atggttgaat ttgagggctg atggaggtgg tcttgtggcg ttagtccgac 240
   actgaaccct gtggggcgta atggaaaata agtgactaag atcacgtttt ggggtggggg 300
   attgcgctct aaagctaggt ctaaagctag gcgacttgac caaaattgat aggctggggc 360
   gggtcttctg aagggcttcg gttggggtaa gctggcgatc tgaaatcgcg ctgcattgtg 420
   gcgtcgaaaa gcaaaaaaat ttgtagaagg gaagagcgca ccta 464
<210> 510
   <211> 98
   <212> DNA
   <213> Corynebacterium glutamicum R
<400> 510
   tttttcaaat ccttcgccat cgacaagctc agccttcgtg ttcgtccccc gggcgtcacg 60
   tcagcagtta aagaacaact ccgaaataag gatggttc 98
<210> 511
   <211> 100
   <212> DNA
   <213> Corynebacterium glutamicum R
<400> 511
   cacccgtaag aacaagcgca acaacccgga ccgtatttcc ctcatgaagt acgatccagt 60
   agtccgtaag cacgtcgaat tccgcgagga gcgataatca 100
<210> 512
   <211> 100
   <212> DNA
   <213> Corynebacterium glutamicum R
<400> 512
   ttgggttggc atcggatcct gcctgtggcc taagatcagg cagtgttgtt aaaggacgat 60
   cggtaatccg aatggatcgt cccgtagtca ggaggaacct 100
<210> 513
   <211> 100
   <212> DNA
   <213> Corynebacterium glutamicum R
<400> 513
   cggcgaaagt gactttcgaa tcgagttcga cgccgtcgat cgcggcgatt tcctgggcta 60
   gcgatgaatc caacacctca ctaacggtag tcttatcctc 100
<210> 514
   <211> 272
   <212> DNA
   <213> Corynebacterium glutamicum R
<400> 514
   tctagtcttt ggaggtttgt taggggagag gatccctctt taatgatcga ttaaggaaac 60
   cgcggtgtct agggcctcta aaagattgaa ttggtgcacc cgctatccac ccattttgtc 120
   catgtcctgc gggatatcca gggggaattg cgcatatgac tggtagaaca agtagtttaa 180
   atcatgagac atttcacata tggttcttca tccgagacat gtgttgacgc tgtctgcccc 240
   tttttgaaaa taacacttta aggagatgtg cc 272
<210> 515
   <211> 216
   <212> DNA
   <213> Corynebacterium glutamicum R
<400> 515
   agcacatcag ggaatccacc tactttgttt tcccctagaa atcccccatt tcatcactcc 60
   cgaatggggg taatgcttga tcgatcaatt gagttgcttt atcgatcagg tctgatttct 120
   gctgggaatc cccacatttt ggaacgtagc gtcgagaagc gtgcggcgaa gctttttcgg 180
   tcgcggccgt tatcttttta agaggagaaa ttttag 216
<210> 516
   <211> 171
   <212> DNA
   <213> Corynebacterium glutamicum R
<400> 516
   aaacgagtca gatcgccttt gtgtggaact actccccttc gaaatggaag tggttgcgcc 60
   aagtttttcg ttgcttgggc gttaacgatt ctatatgtac ttccctagaa atcaagtgag 120
   cattcatctc attgcagaac gttgaagcat cattgactag gatatgtaga c 171
<210> 517
   <211> 512
   <212> DNA
   <213> Corynebacterium glutamicum R
<400> 517
   gggggctaga accacagggg aggcaaggtc aaaggggccg atttttaaag tcacctaact     60
   attgtccccc gtgaatcagg ttgggcaaaa tatttgaagc aaattgtgag cagggcgcaa    120
   ctaggaaagt ggtgtgcttt cactttttgg gggctggggt tgggttaagc ttcgcgggct    180
   ctacggttgg tctgagcttt attcctgggc tttgggaggc ttgcaaacag gaggcatgca    240
   aatttggggg tagtgcaggg ccttgaaatc ccacctcaca gatagtattc aggcatttcc    300
   tgtcacgatg gtttatcctt gggacacaac atcaaagtgg ggtacatcat atgcttccgg    360
   ttgaagtgac ctatctgaaa agactggtcg aaccttgaag caatggtgtg aactgcgtta    420
   acgaattttg tcggacgtta aaatggtcgc attctgcttg ctgaagtggc acacctatgt    480
   gttctgcttg ggtatagcag tgcgggaaaa at                                  512
<210> 518
   <211> 100
   <212> DNA
   <213> Corynebacterium glutamicum R
<400> 518
   gctgtcacca aagaacgtgc gcgtgcagct ttccgaggca aagacgacta gtctttaatc     60
   caagtaagta ccggttcaga cagttaaacc agaaagacga                          100
<210> 519
   <211> 311
   <212> DNA
   <213> Corynebacterium glutamicum R
<400> 519
   gcgctctagc gtatcaacta gctgcagttg ttatgtgcac agcaggggat tgacagtctt     60
   ctggctcagt ttcggttgtt ggtcacaaat gaatcacgtg taccctttgg tgggaaagtc    120
   attgaaaacg tcgcacacct aagtcgcatg cagaaggcgc acgcctaaat tgtgcagata    180
   acctaaactg ggtgcattta gtgtggggat gaggcacgag atcatccatc gtcggttcca    240
   tagaatgggg agctgtcgaa tgctgataga tatttggtga ccagagatta gagcaaagga    300
   tcattttcat c                                                         311
<210> 520
   <211> 133
   <212> DNA
   <213> Corynebacterium glutamicum R
<400> 520
   gcgttatagt ctagagcgag caggcgagat gtgaagtacc tacacgcctt aagtgcaaat 60
   gaattcacaa ttgccagaag atgcacagga tgtaatctag atttcccaag ttcagtgggg 120
   caaaatgact tat 133
<210> 521
   <211> 133
   <212> DNA
   <213> Corynebacterium glutamicum R
<400> 521
   atgaaaaagg tcgctattaa aagcttcttg cacaagtcgc ccagatagcg agcggaccac 60
   tcggtcaact gaataacccc actaaacact tcacagcccg aacacacggg caccagaaag 120
   ggaacgacac ctc 133
<210> 522
   <211> 823
   <212> DNA
   <213> Corynebacterium glutamicum R

<400> 522
   cacgacggcg acgaacgatc atgtcatcgc tgtaacgctt aggcttgcgg gtgcggcctt 60
   ccttctgtcc ccatggggag actgggtggc gaccaccaga agtcttacct tcaccaccac 120
   cgtgtgggtg gtcgaccggg ttcataacga caccacggac ggttgggcgc cagcccttcc 180
   aacgcatacg accagcttta ccccaacgaa tgttgatctg ctcggcgttg ccgacctcac 240
   caacagtcgc gcggcagcgg atgtctacgc gtcggatctc ggaggatggc atacgcagaa 300
   ctgcgtagga gccttcctta ccaagaagct ggatggaagc gccagcggaa cgtgccagct 360
   ttgcacctgc gcctggcttc aactccacat tgtggatggt ggtaccagtt gggatgttac 420
   gcagtggcag gttgttacca accttgatgt cggctgcagc gccggactcg ataacggtgc 480
   cctgggtcag gcccttcggt gcgaggatgt aacgcttctc gccatcgaag tagtgaagca 540
   gtgcaatgtt agcggtacgg tttgggtcgt actcgatgtg agcgaccttc gccaatacgc 600
   catccttgtc attacgacgg aagtcgatga cgcggtagcg gcgcttgtgt ccaccaccgc 660
   ggtgacgggt ggtgatgtgg ccgtgagagt tacgtccgcc ggtcttgctc agtgggcgga 720
   gaagtgactt ctcaggggtc gaacgggtga tctccgtgaa catggaaacg gagcttgcgc 780
   ggcgacccgg ggttgtcggc ttgtacttac gaatagccat aat 823
<210> 523
   <211> 132
   <212> DNA
   <213> Corynebacterium glutamicum R
<400> 523
   tttggtggta aaaaccgtcg aattggggcc tatttcgccg tttctcgcgt tgtgcgtggt 60
   actacgtggg gacctaagcg tgtaagatag aaacgtctgt atcggataag tagcgaggag 120
   tgttcgttaa aa 132
<210> 524
   <211> 100
   <212> DNA
   <213> Corynebacterium glutamicum R
<400> 524
   cctacgacgt tctcaagtct gacgacgttg tgttctccgt tgaggctctc cacaccttca 60
   tcaaccgcgc ttccggtgcg gcacaggagg agcagaacta 100
<210> 525
   <211> 146
   <212> DNA
   <213> Corynebacterium glutamicum R
<400> 525
   ggtgttcatt ttagccgatc tggtcataac caggggtgtg gttttggtga ctgaagcggg 60
   acaggagcgc ctagtaaaaa gcaaggctgg aaaacagata agccctaagt gcccacatgt 120
   ctaccgatca cggtagattg ttcttt 146
<210> 526
   <211> 130
   <212> DNA
   <213> Corynebacterium glutamicum R
<400> 526
   gtcagcttgc tcacttaaag cttcgaaata agcccttaag ttctcattct tttgttagac 60
   ttgttcacta attacagaca tccaggggct tagattttta acccctgtca taagtttttg 120
   ggaaggtcac 130
<210> 527
   <211> 775
   <212> DNA
   <213> Corynebacterium glutamicum R
<400> 527
   tttcggggag cttcgggctc gcttcttggc aatgtaagcg gtgagttagc tttcgaccaa     60
   accacactgc ccacgaatcc tagaaatggg atccgtgggc attcctgtct ggtttaacgg    120
   ccttgaaacc ggacaggatt gcccaaagta gtcgaatttc cagttcttgg gcatgtgtgt    180
   ttggtcaggt ttagtcgggt cgaaaaatga actcggaacc ggtaaaaagt gacctcacag    240
   aatcgcttct aagggccgtt caaagtgttg cctatacaaa cacatgttct gactgcccga    300
   cctcttaaaa tggcgttaaa tggcgcgaaa tggaaaacgc ctcgtggcct ggatggagcg    360
   ttagtaccag accagtacga gaccagacat acgtgacaaa aaatcctgaa aagtggaatc    420
   attgtcacgc ctgtctggtt tagctctggt tcgggacggg catggaatgg aggcagcgta    480
   ccgaagcctt gacccgcggc ccgacaagcc aaaagtcctc aaaacaaacc caccccgccg    540
   gagacgtgaa taaaattcgc agctcattcc atcagcgtaa acgcagcttt ttgcatggtg    600
   agacaccttt gggggtaaat ctcacagcat gaatctctgg gttagatgac tttctgggtg    660
   ggggagggtt tagaatgttt ctagtcgcac gccaaaaccc ggcgtggaca cgtctgcagc    720
   cgacgcggtc gtgcctgttg taggcggaca ttcctagttt ttccaggagt aactt         775
<210> 528
   <211> 456
   <212> DNA
   <213> Corynebacterium glutamicum R
<400> 528
   tttgttttcc cctccttttc tgttttccac acaaccgaag cttcaagggg aaaacaaggg     60
   ctttaaaagt tatccacaga tccgaagtga tccgcgtcct ggggtgaaaa gttatccaca    120
   ggaagcggag gggcggattg aaaaattcag cgaaatgcga aaaggtggag gggaaatgct    180
   gcgagtcttg cggattcccg gcgtggcatt gaaaaaagtc taaagttgaa cttaagattg    240
   aggtcattct gaagttgtga cctgcatcag aagagttaca tacccacata tgtaaccttc    300
   tggactaaga tcacgacaga ctgaaaagaa ctgaagactc tcaaggcata gcccacgtgt    360
   gtttgtcggg ccggaagcgg ggaactttcg ggacggatct aactcattgc gggcctgtgc    420
   gcagtatcca aaaatcaaaa tgagaaggaa aacttc                              456
<210> 529
   <211> 378
   <212> DNA
   <213> Corynebacterium glutamicum R
<400> 529
   ttgagtttta cttacctttc aacgagacaa tgcggaggga acaactttag tcacattagt 60
   aacttcagtt aacttctttt cctcctgtca cattaatcgc agaatattta ctgtcaataa 120
   ggatcaaagt gaatttctgg gtttccctaa agattcacca aagatccgag acatattaga 180
   acaacgtttt cttgttttgt ttgtcgtaca tcgatgcagt tccgttacaa aattctgaaa 240
   aaaccagcaa aatctttaga gatttgcctc acaatgtgcg gaacgggcga taatgcgagt 300
   acagtgatac cgttctaaaa caatggactc gttttacaag tcctccatat ttctttatcc 360
   ggcaggagaa tgccccca 378
<210> 530
   <211> 149
   <212> DNA
   <213> Corynebacterium glutamicum R
<400> 530
   acacttattc acaaatcatt ggactgtgat aactacccaa gagtgtcaca acttggtaac 60
   gtgtgggcgg aaaaacaaga taggcatcga gaggtatcag cggtccatac cgccgatggg 120
   tacatatcaa tttttgccgg aggagaatt 149
<210> 531
   <211> 75
   <212> DNA
   <213> Corynebacterium glutamicum R
<400> 531
   ctaaacaagc tttgattaac aagtccctgt attgtgtaaa tcgcttattg cacacaattg 60
   aaaaaggaca ttgtt 75
<210> 532
   <211> 263
   <212> DNA
   <213> Corynebacterium glutamicum R
<400> 532
   agaattaagc cctttacctt cattgcgtat tgagaggtca ggggcttaat tggagccggt 60
   gacgggaatc gaacccgcgc cgcctgcttg ggaagcaggg gttctaccat tgaactacac 120
   cggcagtgta aaaggtggct cttaattaag ggccgagaat aaggctagca cgataaaaat 180
   cagcgccagc accacgcgcc ccaggaacct catcgcagca acccgtggcc caaccctgaa 240
   cgctgaacgc tacactggtt gac 263
<210> 533
   <211> 313
   <212> DNA
   <213> Corynebacterium glutamicum R
<400> 533
   gcctcggcca ctgcctccgc ctttacgagc atttccgcct gagcgggata tatctccacg 60
   aaacgtcatg aaagaaatca tggcacatta catagggaaa ccgacaggtt tgggtgtaac 120
   aaaatggacg caatcgaaaa ctgactgcac ctaataaacc actcaaggtt caaggcaaac 180
   cagatggcgg gtacaatgcg tgtgttgcct gtttctgggc tgcatgcccg ccctttttgg 240
   gtggttgagc ttattgttta aggctccaga aaccatcgac tgatcaaaac caagcggaag 300
   gacttccacc aac 313
<210> 534
   <211> 210
   <212> DNA
   <213> Corynebacterium glutamicum R
<400> 534
   gtgactgatc ttaaaggact ggggacacac ctaggcactt gcgggggtgc tctgaccgca 60
   tgcaatgact attaatccct atgggggatt gtgctacagg acacattggg tctaaactca 120
   ctctcaactc accaagattg ttcaacaatc tgcgattggt gtgcaagcta ccccaatcat 180
   tttgaaagcc cccacgaaag gagcgcgaca 210
<210> 535
   <211> 100
   <212> DNA
   <213> Corynebacterium glutamicum R
<400> 535
   aagccgttaa aagttgagac tcgcgctgag aatctcgcag aaaactccga agctcccgct 60
   aaggtagcta ccggaatcaa atcttaggga aggaaaacat 100
<210> 536
   <211> 565
   <212> DNA
   <213> Corynebacterium glutamicum R
<400> 536
   taattatcca atccagattt aacccacaaa gaagctttac cttctcttaa gagcttttag 60
   agcaaaataa aatgacccgg aagctcatct cacgcaacca gcaggccttt aatcagtcaa 120
   ttaaagccac gttagcaaca tatctcctgt caagaaaata acagattgaa ttcactgtcc 180
   agttatgggt ttttcggatt caccccccca tgcagcggaa atgctaactt tcttcagtgg 240
   attccaacct tttttgccat aaaccgtgca ccggagcaaa acctctcact ttaagtaact 300
   tcattttgca acctggtatt agtgtgaacg catttttatg agttatcgat tccgaaatcc 360
   ggatttgcct gttaacgttg ttgcttgagt tctcgatgta aatgaaaggt gcgccacaat 420
   ctgttcctag atgcggtgtg caacgggctg ttagcccagc tttggtttat attgtcgatt 480
   agtgggttta cgccctgcag tcccgtattt accccatata ggtaatgcca gggctaccaa 540
   ccacaagttc acgagggaaa gacgt 565
<210> 537
   <211> 496
   <212> DNA
   <213> Corynebacterium glutamicum R
<400> 537
   ggcatgacta tagcgtgatc accatcacct taaccactct tggcattgag gtgttgcaaa 60
   gttgttgatt ttcgcttttc gacgcagccc gccgccatca ggctcccggc gtggtcgggc 120
   cacatgcgcc ccgggaactt tttgggcacc tacggtgcaa cagttgcgaa aattgtgtca 180
   cctgcgcaaa gccttgcttc gattcgggga attcgggtgt ctaaactttt tgattgatac 240
   caaacggggt tagaaactgt tcggatcggt atcctgtgag gaagctcacc ttggttttag 300
   aatgttgaaa aagcctcagg tttccgcagg tagagcacac tcaattaaat gagcgtcaaa 360
   cgacaataaa gtaaggctac cctaataagt ggggttttat gcctctaaat agccagttgg 420
   gggcggtagg ggagcgtccc atgactggtt aatgcctcga tctgggacgt acagtaacaa 480
   cgacactgga ggtgcc 496
<210> 538
   <211> 200
   <212> DNA
   <213> Corynebacterium glutamicum R
<400> 538
   catttatttg gtaattgggc aacatcactg atactcttga acgctgttgt ccatctttga 60
   tgtgagcatt ccagtgacat gcggacagga ccttgggttc gttgcacttt atgccaagca 120
   gtttatttaa aactgcggga gaaacactcc tcgatgggtt tgtacacaac tttaactaga 180
   aagttcaaga ggtatttgcg 200
<210> 539
   <211> 191
   <212> DNA
   <213> Corynebacterium glutamicum R
<400> 539
   tgaaacgacc ttctagaagc atcgtttaga attgctttta agtgaataag gaacagcaca 60
   gaattaaggc ctcggattta tccctcacgc tacttctcaa gtggcgccaa ggtaagttgt 120
   actttttctg tccaaaatat tgttttttcc gtagataggt tatcgaacgg aaattacttg 180
   gcaataccgc t 191
<210> 540
   <211> 100
   <212> DNA
   <213> Corynebacterium glutamicum R
<400> 540
   tgggtggata gcgagcgact gctgggaagc gggtctcctg gggtgagaat tcccctcgcc 60
   acgagacggc ggatctgttc tgtgagctgc gtggggatgg 100
<210> 541
   <211> 100
   <212> DNA
   <213> Corynebacterium glutamicum R
<400> 541
   agggcgccag gggcatccag ccattaaagc ttttcgacga gccctcgccc atgtggccaa 60
   agaatcttat ttggaggctc gtctagtaga gtgagttctt 100
<210> 542
   <211> 111
   <212> DNA
   <213> Corynebacterium glutamicum R
<400> 542
   aaagttttac ctttaaaaac tacccgcacg cagcacgaac ctgttcagtg atgcaaatca 60
   ccgctaaaat attgtggacg ttacccccgc ctaccgctac gatttcaaaa c 111
<210> 543
   <211> 488
   <212> DNA
   <213> Corynebacterium glutamicum R
<400> 543
   aagaccctaa aaacgcctct ggagaaaaat cccggatgcc gttaggcatg cgggattttc      60
   gggcttttac ccccttcgct cacacccgca accgggcgtc gaaaagcgtg ctctttgaaa     120
   ttgtgacaag gataacaacg gtaacgaatt gatataggtg agcgtggttt aagttgcatc     180
   attgggggtg caaagttttg taaacacgca attcaaatca caaatctgta accctgacgg     240
   gtagggttgg ccacgttcac ttcactgtca ttggcatgaa gcttttgggg tccttgtggc     300
   cttgaaagtg tgcaggattt ttgaattctc tttggagttt tcggcgcgta tgtcagataa     360
   aaaataactg ctggctacaa tggcacgtga agaacagtat gataaatgga aattccagtc     420
   atgagagatt cttgtggctg agtcccggcc ctgcctgggg ccaccgttaa atcgaaggga     480
   atccgcaa                                                              488
<210> 544
   <211> 100
   <212> DNA
   <213> Corynebacterium glutamicum R
<400> 544
   ctgcacgtgc actgcacgag cagttccagc tgggcggcga agacgaagcc gtcgtttatg      60
   caggcaccgg acgctaaagt tttaaaggag tagttttaca                          100
<210> 545
   <211> 100
   <212> DNA
   <213> Corynebacterium glutamicum R
<400> 545
   ttgacgccga tgccaacatc atccttatca agggcgcaat ccctggtaac cgtggtggca      60
   tcgttaccgt taagaccgca gtgaagggcg gtgcacacgc                          100
<210> 546
   <211> 1000
   <212> DNA
   <213> Corynebacterium glutamicum R
<400> 546
   aatttctaaa gtggctctgt gccgagacga aacactacaa aaaatacttg tagggctata      60
   cacttgatcg catcaataaa tcgattttga ttgatggcct tctgtatcct tatttggcta     120
   cccttagtga ggctttgacc gttgtcggca aggtctcata agggtactga taatttcgga     180
   ttacactttt atttttcaat tagccattgc gcgatggcgt ctagaatgaa tcaaaagctg     240
   cgccttgtct tgatgcgatc aaagattaag atgagcaata tttggatcgt caaagattga     300
   gagcaaattt atgcacccgg atgaatcgag agacccgcat cagattgagc agctgcggat     360
   tgcggaggtc cctgtggccc ctaaagatgc atctttaacg ctcgaggaat taactgatga 420
   aacacggaat gatggaaata aggcacaaga ctggcaggga gaactgtcta acttaaatcg 480
   acgtatttct aaagccgatc aagcactttg cgaggaaaag aaaatttccc agaatttgca 540
   agaaaagctc aaagtagaaa agcaacaaaa tgctgatttg agaaaacagc tggacaaggt 600
   gcaaagccag cttaaatcgc tccgagcatc gacaacgatg aaggctggcc gacttgttgc 660
   cactccttat catttttcta ttaatagtgc ggcaaagatt aaaagattct tcacggatca 720
   agatctggtg cgaaagattc cggccaaacc aaaaaagatc gttacaccag tgtcgaaacc 780
   atctgtggaa cgcctattaa atgacgcgtg gtataaccgc ggttcaattc aagactccta 840
   tcaaattctg agtagtctag aagatgcgac ttctgagcta tccgaaaaag gactgattct 900
   taaacagcgc gttgaggcgg cttatcgtct ttttaaacag ggggttgaga ttccggttcg 960
   cacgcaaggg tgtgcttata aacctgaatc tgggcgtgtc 1000
<210> 547
   <211> 100
   <212> DNA
   <213> Corynebacterium glutamicum R
<400> 547
   acggcacttt tgtcggtggt acgcgcattg atcagcctga gcagattgcg gtgggcacgg 60
   atatccgtat tggtcgtaca acagtgaggc ttgttccctg 100
<210> 548
   <211> 246
   <212> DNA
   <213> Corynebacterium glutamicum R
<400> 548
   gcctacgata aagtcaattt gatattttct ccaaggtatt aaattcccac ctagagacga 60
   ttaaattcgt agctcattca actacaaaac ttgcccaccg aaagtatcac cccaaaattg 120
   attatttttg actaacataa aagcgcctca gataatttaa tggtgaacat tttacaaaaa 180
   cgcctatttt caggcgtact cttaagcgcc cttgcttttc cagtagttgc aacaccagct 240
   aatgct 246
<210> 549
   <211> 65
   <212> DNA
   <213> Corynebacterium glutamicum R
<400> 549
   agtttttagc gtacctaccc tgcgggaaat gaccttaaat gacccgtggg ataggattgc 60
   ctatt 65
<210> 550
   <211> 1000
   <212> DNA
   <213> Corynebacterium glutamicum R
<400> 550
   atccacacct tgacgccgat gcgtccgaaa gtggtgtgag cctcgtaggt gccgtaatcg 60
   atttctgcgc gaagggtgtg cagtggaacg cgaccttcgt ggtagcgctc ggtgcgggac 120
   atctcggcac cgccgagacg accggagcac acgaccttga tgcccttaac ctgtggctga 180
   cgcattgcag actggatagc cttgcgcatt gcgcggcgga atgccacgcg gttggtcagc 240
   tgctcagcga tggactgtgc caccagctta gcgtttgcat cgacgttctt gacctcgagg 300
   atgttgaggg caacctgctt accggtgagc ttctcgagct cacggcggat gcggtcagcc 360
   tcagcgccac gacgaccaat gacgatgcct gggcgagcgg tgtggatgtc tacgcgaacg 420
   cggtcgcggg tgcgctcgat gacgacgtcg gcgatgccgg cacggtcgag gcccttggac 480
   aggaattcgc gaatcttgat gtcttctgcg acgtagtcag cgtaagactt gtcggcgtac 540
   caatgggact tccagtcgga agtgatgccc aaacggaggc cgtgtggatg gatcttctgg 600
   cccactagtt ggctccttcc ttctggctct caacgaccac ggtgatgtgg ctgctgcgct 660
   tacggatctg gaatgcgcgt ccctgagcac gtggctggaa gcgcttcatg gttggacctt 720
   cgttggcgta agcctcggag ataaccaggg tgcgtggatc caggccgaag ttgttctcag 780
   cgttagctgc tgcagatgca acaacctttg ctacaggctc agatgctgcc tgtggcgcat 840
   acttcaggat tgacagtgct tcgacgacgg tcttgccgcg aacaagatca atgacgcgac 900
   gtgccttcat tgggctgacg cggacgaagc gagcagtcgc gcttgcggag gtgatgttgt 960
   cactcatcgc ttatcgacgt cccttcttgt cgtccttgac 1000
<210> 551
   <211> 100
   <212> DNA
   <213> Corynebacterium glutamicum R
<400> 551
   ttccacgccc agggaccgtc cacacttcgg tgcctgtgaa agggtgaatc tgtttgatcg 60
   tgccgtcagc catggtgcga atgggggtga cagaaaaaga 100
<210> 552
   <211> 100
   <212> DNA
   <213> Corynebacterium glutamicum R
<400> 552
   gtgttatttt cttggcggac ggtcgtatcg tgaaccagtt gtttgatccc accatcgagg 60
   aaatcttggc cacgatgaac ggaattgagg atattgccta 100
<210> 553
   <211> 266
   <212> DNA
   <213> Corynebacterium glutamicum R
<400> 553
   cccaaaaagc gttagatgaa acttcccacc cgaatccaca agaactcggg tgccctcaat 60
   ttcacatacc cctaagcgaa acactatgcg agctttccgc cagaaggcca agcacccttt 120
   cgattaaccc cgacaaactt ttaaggcaag cctaaattag gtaaacctta aacagtcgcc 180
   attgaagaaa ttgaagaatt ttaaaaacaa acaaccttca acgcgctaac aagcatcttc 240
   ccagtctcgt taccggagtt tctcac 266
<210> 554
   <211> 56
   <212> DNA
   <213> Corynebacterium glutamicum R
<400> 554
   ggcagcgagc ctacactaaa tgactgtcca agcaactgaa gggaggcgtg tgaacc 56
<210> 555
   <211> 100
   <212> DNA
   <213> Corynebacterium glutamicum R
<400> 555
   attgatttct gttctggcga tccaggcttg gatatttcac aaatggttca tgaaacccaa 60
   cgtgcacgac tatttatgag ttaagaagga gaaaagaaaa 100
<210> 556
   <211> 100
   <212> DNA
   <213> Corynebacterium glutamicum R
<400> 556
   tttggtgcag atcacgcacc ttcgggcgaa caccctcaaa gatatttggg aataggggtc 60
   atcgtgcagg cccccacacc caacgcgtaa cataacggtc 100
<210> 557
   <211> 100
   <212> DNA
   <213> Corynebacterium glutamicum R
<400> 557
   gcaccactgt tggtggcgga cgacccgtgt acacaggacc agccattgtg gatgccacca      60
   acgttgatgt cattgctgaa gccgttgggg agggtctgcg                            100
<210> 558
   <211> 100
   <212> DNA
   <213> Corynebacterium glutamicum R
<400> 558
   cccactggcc gcttcggcga atgaatcccc gctgtctatt ccttggggca tcaacgagta      60
   cttcgctaaa atcgctgagc ctctgaagta aaactgcttg                            100
<210> 559
   <211> 100
   <212> DNA
   <213> Corynebacterium glutamicum R
<400> 559
   ccggaacatg ccgtcgaaaa gctaaccggc ccatctattg atggcctgga gctgttcctg      60
   tccgccgttg gcaccatcgc ggcttaagag gagtcaaaat                            100
<210> 560
   <211> 187
   <212> DNA
   <213> Corynebacterium glutamicum R
<400> 560
   ggcttccggg gcgatctaaa cccgcattga ataggcgatt aggtgttttg gggcaacgta      60
   tgtaaacttg tcccttgctg ttgtcgtaaa tattcgttat cgccccgtca gctggcatgc     120
   tcgcgccccg gtcacccggt gcggcgcgtc ttctaactga atgtgggcgg ctaggagaaa     180
   gtaagtt                                                                187
<210> 561
   <211> 100
   <212> DNA
   <213> Corynebacterium glutamicum R
<400> 561
   ggctaccaag cgcgcttatg tgactcttcg cgaaggcagc gactccatcg acatcttcaa      60
   cggctccgtc gcttaagacg tcgatagaaa aggacacatt                            100
<210> 562
   <211> 100
   <212> DNA
   <213> Corynebacterium glutamicum R
<400> 562
   cactgccaca gctgccaatc accgttgatg aagagggcta cctcatcgcc gctggtaact 60
   tcattgagcc agtcggccct gcattctggg agcgtaagtc 100
<210> 563
   <211> 177
   <212> DNA
   <213> Corynebacterium glutamicum R
<400> 563
   atagcaattt gttttatacg gaccagccgc gaatccgaga atctccatgg ataaacagtg 60
   tagtgcgcaa ggggcgctcc cccatcattc cctcaaggtg tgaagatacg gttaggatag 120
   aaaagaattt tttgacgttg gacattctca aaatcaagta gcaagggatc aaactct 177
<210> 564
   <211> 148
   <212> DNA
   <213> Corynebacterium glutamicum R
<400> 564
   tacggctcca aggacgtttg ttttctgggt tagttacccc aaaaagcgta tacagagacc 60
   aatgattttt cattaaaaag gcagggattt gttataagta tgggtcgtat tctgtgcgac 120
   gggtgtacct cggctagaat ttctcccc 148
<210> 565
   <211> 138
   <212> DNA
   <213> Corynebacterium glutamicum R
<400> 565
   gtcctctacc ctagtatcgc cgcctccatc tgatgtctgg ggtgtctgta attttcacgt 60
   ctcctggcgt ttttaagacg aattacaaca attcgttcac gattgagcca aattgggcgt 120
   cttgcttagg tttcgggg 138
<210> 566
   <211> 100
   <212> DNA
   <213> Corynebacterium glutamicum R
<400> 566
   agccgaacca tcatcgcgcc tttcgaaatt ccagtatcag taatcctggg catcatcggc 60
   gcagtggtct tcgtgatcat gattgtgagg caacgtggcc 100
<210> 567
   <211> 124
   <212> DNA
   <213> Corynebacterium glutamicum R
<400> 567
   atttagcgga tgattctcgt tcaacttcgg ccgaagccac ttcgtctgtc ataatgacag     60
   ggatggtttc ggccgttttt gcatgaaacc aaaaaatacg attttcaagg agcatgtaca    120
   gcac                                                                 124
<210> 568
   <211> 725
   <212> DNA
   <213> Corynebacterium glutamicum R
<400> 568
   gtcttcgggc aactttctgc gcttggaagt aaaagggcca gggatcgtta acgatctgac     60
   ccaacaacta taaccctgaa gctgtcagtt cctagcaccc tagattcttc acgcagtctc    120
   ccaaacgatg aaaaacgccc aaaactggcg acaccgaact attgaaaacg cggagattag    180
   ttgaccagtc accaatttgg gggtggttca aagttttgca aagttttcaa tttctaggtt    240
   gttaatatcc cctgaggttg cgttataggg tggcgaattg catggggaaa gctactcggc    300
   acccatcctt gtcgtgtgca tcacaaactt tgctaaactg tgtaccagtc cacttattgt    360
   gggattttta atgccttaaa ggccagcatt ttcaccctct agcggggttg aatgctggcc    420
   ttgagggtgc agaactaaat agcagcacat cggcacaatt gatctgagtt ctattggcgt    480
   gaccgtggct actgattacg gtggctgtgg gtggtcggga atgatgtaac caacgtgatt    540
   gtgggggaat tggctctcac ttcggatatg gctaaaccgc atttatcggt atagcgtgtt    600
   aaccggacca gattgggaaa gaaatgtgtc gagtaacaaa aactgacatg cgcttggcgc    660
   atcccagttg gtaagaataa acgggactac ttccgtaatc cggaagagtt tttttccgaa    720
   caaat                                                                725
<210> 569
   <211> 100
   <212> DNA
   <213> Corynebacterium glutamicum R
<400> 569
   acaagaccct cgatgctgcg gctgcgttgg accaagcgcc cgctgtcgag gatggacgtt     60
   ttatggttcc gcagattctg ggtgagggcg actaataatt                          100
<210> 570
   <211> 100
   <212> DNA
   <213> Corynebacterium glutamicum R
<400> 570
   cctctgacca gaccgcaatc accgcgggtg gacagaccat tgatttccag aacggcacca      60
   tccgtcaggt caatggccga attgaggagt ctcgctaata                           100
<210> 571
   <211> 572
   <212> DNA
   <213> Corynebacterium glutamicum R
<400> 571
   cgcttttcga cgagcccccc accttcgccg ccaccagagt gccggcggtc aatgtgggtg      60
   ggctatttca ctgcggggca ctgaaagtaa aaacgcaact ttcttacaga acagggttgt     120
   ctttcagacg actatgtggt taactacttg ggttgcttta acacggcgtg aattaaccat     180
   gccagttggt aaggcaaaca tgacaccttc aattggagtc gaggcgcatg aatatgcact     240
   tcaacttcag ggggtatcca ctgaagccgg gtgactggtg aaggcggaac cggagaaggg     300
   gcatggcaaa taaacagcgg cagttacgtt agggcctaga tcacgcattt tggtcccttc     360
   cgatttccct gacttcattg ttgggttcat cgtggcgcgt tttatttgta cagcgcccgt     420
   gatccaatgt cagaagcatt tgacaggtca ggttaaacac tggcgttgcg cccgagcccc     480
   aagcccggac aacgttatag agaaagaatg aagcgaattc ccaccgcttt tccaaaatgg     540
   aagatgtggg acgagcgagg aagaggataa gc                                   572
<210> 572
   <211> 100
   <212> DNA
   <213> Corynebacterium glutamicum R
<400> 572
   cctgtggata attggttgtt ggctgatggt gatgtcatta cggtgggtca ttccaatatc      60
   gaagttcgta ttgttagtcc ctagagggag aggttgatca                           100
<210> 573
   <211> 213
   <212> DNA
   <213> Corynebacterium glutamicum R
<400> 573
   aaggcttcgc tcctaaaact ctttaagagt caggccaact agcccgcccg gttttctcgt      60
   gaaaatcagg cggtctttgg cttttctaaa gtgtttctaa agccaccttc gacctcaatt     120
   aagcagcaat ccgagccggg ggagcgctgt cagaaatgga ccgtgccacc ccatgacaac     180
   atgcttgcac aatgatgact agaataatga ccc                                  213
<210> 574
   <211> 100
   <212> DNA
   <213> Corynebacterium glutamicum R
<400> 574
   ccatcgtgtt tattactcac aaccctgagc ttgctgatga atctgatcgg gtggtcacca     60
   tggttgacgg gcgcatcatt gggtctgagg tgaaacactc                          100
<210> 575
   <211> 100
   <212> DNA
   <213> Corynebacterium glutamicum R
<400> 575
   atgattccgt caccgaagct gacctaaaga aaattgctga aaccctcctc gcaaacaccg     60
   tcatcgaaga cttcgatgtg gtgggagttg aggtcgcgaa                          100
<210> 576
   <211> 128
   <212> DNA
   <213> Corynebacterium glutamicum R
<400> 576
   tttgttttga cgacgcagta acgcaatcgg ggattgtggt cgattcttta agcaagggta     60
   atgtcgaaat atctaggcaa cccgactttt atgtccctgc ttgagttgaa aactgctgtc    120
   gatcaaag                                                             128
<210> 577
   <211> 370
   <212> DNA
   <213> Corynebacterium glutamicum R
<400> 577
   gcgctccgtc catcaaacaa tctgaccctt gtggaaagtg gggcaattgg ggcagttgta     60
   aaaggcatat cctgaaacta tccacagcac ctgcgggagg gtttgggatt gcaggtcaaa    120
   gtatgtaaac ttgtcggcag cagtgtgtcc gaattgtgtt tcatcatgcc tgaaagcatg    180
   gcgaagggta attttaacta ctgactacgc gcggtggcgt tgaatgcaca ttcaactccg    240
   gagcaacgag gtcccaaaga actcatttga gttttagggt gttgacctgg gaaaccaccg    300
   ctagggtgca ggataaaaac ctgcacaaat ttttaaaacc gaaaccctaa gacttagaaa    360
   ggggcttaac                                                           370
<210> 578
   <211> 100
   <212> DNA
   <213> Corynebacterium glutamicum R
<400> 578
   tgagctaccc ggacgaagct actgctctcg aggctctgcg ccgcgctggc cagaagcttc 60
   catgcaaggt ccgtatcgtc aagagggagg atcagctcta 100
<210> 579
   <211> 508
   <212> DNA
   <213> Corynebacterium glutamicum R
<400> 579
   gtaaagttct aaagctttac ttatagcact ttcggtaggt gtcgagaaaa ttcccaaatt 60
   gcggaaaatt cacatacttt gtgccacaaa gtgataaaca tcacaaaatt ttataactca 120
   gccattcatg caggccagaa catgcaaaac cttgcacgct ctggcctttt gttatgtaac 180
   tgttgtgaaa gatgtatcaa aagttaccaa ggtgacttaa tgttcagata tcgactcatg 240
   ggttgttttc caaaacttga atcaatttga gtaacagtag ttatcaagcg ttaaaccttg 300
   aaacttccac tctttttact gatgagcatt tgggcaaacg gggaagcttg ctggagatgc 360
   aataaatcgg tgaatggttg atgcaataaa gaggagagtg gcccatagtt tccgctacgg 420
   aacgcggagt ctttacgttt caattcttgg caagtgtcta gcgtcaaggc ttgatatgtg 480
   aagggattcg gattgaacag gagaacat 508
<210> 580
   <211> 100
   <212> DNA
   <213> Corynebacterium glutamicum R
<400> 580
   gcgatgctcg aacgcgcaga acgctcctgg gtagacaaag ccactgcata cgatttgcgc 60
   tggtcagatc actcaccact gaacgtgatc tactcctaaa 100
<210> 581
   <211> 54
   <212> DNA
   <213> Corynebacterium glutamicum R
<400> 581
   actttgtcac cctagaccgt ctaaccttta ggtgtgagat taggtgtatt agat 54
<210> 582
   <211> 159
   <212> DNA
   <213> Corynebacterium glutamicum R
<400> 582
   tcgttatgtc attgtgatgc tcccgtgaac ataaacggga cttactggct ttacttaagt 60
   aacagctatg taaaagacca ggtcaggttc gggtcggttt aggtacgaaa cccatttttc 120
   ggtttgcttt ccaggtttcc ccaagtaaag gtgagtttt 159
<210> 583
   <211> 100
   <212> DNA
   <213> Corynebacterium glutamicum R
<400> 583
   cgtggacgat gccatggttg gccacaagct gggcgaattc gcccctacca agaccttcaa 60
   gggtcacgtc aaggacgaca agaagggacg tcgataagcg 100
<210> 584
   <211> 100
   <212> DNA
   <213> Corynebacterium glutamicum R
<400> 584
   gctgtgggct actcaattcc acccagaaaa atcaggtgac gcaggcgcaa agctactgcg 60
   aaactggatc aactacatct aacagatagg atcaatattc 100
<210> 585
   <211> 439
   <212> DNA
   <213> Corynebacterium glutamicum R
<400> 585
   cagccactat ccttaatttg cagtcaggta gattccagtt gatagtcgaa atctaattaa 60
   acctatagct gtcttaacgt tcgaatctct agaaatgtta acaggtggca tgaaagttac 120
   ccaccgaagc aagatttagc cttgagagtt tcagactttc gcatctagac ctgtgctgca 180
   ctctctaaac gcgcaccgaa acacctcata gtctgatggt tacgtgctag aaccaacatg 240
   tccaatcatg taagttccag ctcttcagga ataattttta gcaaaatctc agctttttcc 300
   tcagttgcta gtgcgcaaaa ttgagggtaa gaatacgaca ttcatatcca agtagtaaag 360
   tccgtagaac acggtagaga tcactactta ttcatgaaag tatcgctgcc cagatctcac 420
   aaacattgag gattccaaa 439
<210> 586
   <211> 100
   <212> DNA
   <213> Corynebacterium glutamicum R
<400> 586
   cgcgaatggc ttcctcttta gccccgattg cgtggggtgc gtgggcctca aactcgagga 60
   gggttaaatc atctgcggaa agcatgctta gaatgttgcc 100
<210> 587
   <211> 985
   <212> DNA
   <213> Corynebacterium glutamicum R
<400> 587
   ttggttagag cagcagcgat ttttagtaag gccaataaca tgttttggct taaacctgtg 60
   tcgtgtcaga tggtggcgaa gtagagttcg caaagctagc gaacatgaat tcgtgttcag 120
   gaactaaaca gggatcaaac agagaacaga gaacagatca cgctgcccaa aaatcgcact 180
   tttaaggttt gtgggcgtct gtgtgtggtt tgccgctgta aagtatcacc acgttatgcg 240
   ccctggtgtg atcaagcgtt cgttctgggt cgaaacccca aaagtcacaa ttccccagaa 300
   gcgggtcaaa cccatttagc ttattgctta catatcgagg gtttagaaaa gtgatttgtc 360
   ggatcagtcg gtttctgcca agtaaataga actttataaa ttttgtggct ctcaaatctt 420
   aggccacggc ttccgatttg aaccggaggt tcaaaaggct tatatagaca agattctgca 480
   tcgtctcacg agcccctcat tgcctgacac ggtcaatcgt gtgggaggta ccaatccgtg 540
   agatttctgc caacgagcga ttcattggcc ccgctgcaga gctggcagaa cacggacata 600
   acccaaataa tctgaggtct gccgtttgca gcagcattag cgtttgatgt ggaaggtgat 660
   gcagaggctg ttgatctgca agcgcgtctt tcccaagcac gggggaaccc tgaagcatcg 720
   gatgctctag ttgctgagct gactggtgtt actgctaatc atccgttggt cagtgcttgt 780
   ctgaagtttc cgctcaatcc taagcttctc aagatttcgt aaaaaagctg ccaactaccg 840
   taaaaccgca ctactagagg agtgcgtttt tcgttcctga acacattgcg tgctgcaact 900
   taattatggt cctcccagct cagtgtgctg tgtggattgt ttattctcgt ccattaagtg 960
   atcgagaaaa agttgttgta aagtc 985
<210> 588
   <211> 100
   <212> DNA
   <213> Corynebacterium glutamicum R
<400> 588
   accacggttg tctccattgt cactgtgctg gtcatcgtct acatcatcgc caaccttctc 60
   gtggacttga tctacgccgt tctcgatccg aggatccgct 100
<210> 589
   <211> 200
   <212> DNA
   <213> Corynebacterium glutamicum R
<400> 589
   cgcaaagctc acacccacga gctaaaaatt catatagtta agacaacatt tttggctgta      60
   aaagacagcc gtaaaaacct cttgctcatg tcaattgttc ttatcggaat gtggctaggg     120
   cgattgttat gcaaaagttg ttaggttttt tgcggggttg tttaaccccc aaatgaggga     180
   agaaggtaac cttgaactct                                                 200
<210> 590
   <211> 192
   <212> DNA
   <213> Corynebacterium glutamicum R
<400> 590
   ctgcagggcc tcctggggca cttgatctga cctggttacg tggggtgcac gtatcgaggc      60
   gcattataaa tgtatcggcg cttgggctgt atcaacccgc acgtcatcta ttgttgcact     120
   tttgtgcccg gaattttgga ttctggacac ccaaaagggg gtttcgtacc aaactcgtga     180
   catactaggc gg                                                         192
<210> 591
   <211> 172
   <212> DNA
   <213> Corynebacterium glutamicum R
<400> 591
   tgcgctactc ctgattggtg tatctgtgat caactaaaaa atttaacgcg tcatgaaaac      60
   ccgttactgg gagtccatat tactgaacac ttgcgcgcga ggtgcaccag tccccgaatt     120
   caccccatat gcaaggtatc tacatcaaag agcaccggct attgtggagt gc             172
<210> 592
   <211> 100
   <212> DNA
   <213> Corynebacterium glutamicum R
<400> 592
   cccgttacgg tggcaccgag atcaagttcg gtggcgtgga gtacttgctc ctctccgctc      60
   gtgacatcct cgcaatcgtc gagaagtagg ggataagttc                           100
<210> 593
   <211> 100
   <212> DNA
   <213> Corynebacterium glutamicum R
<400> 593
   tcgaaggctg ggtgcaaaag aagcgccctg gaaccgctgc agcacaagcc gcagaagccg 60
   cccaaaacgt ccacaaccag gaaggctaag caggatcctc 100
<210> 594
   <211> 100
   <212> DNA
   <213> Corynebacterium glutamicum R
<400> 594
   ggtggatgcc taagtggctg gatcgaattc tgccaagttt ggacattgaa ggcaccgccc 60
   tggagaagga atgggaggag aagcaggctg cacgttagac 100
<210> 595
   <211> 290
   <212> DNA
   <213> Corynebacterium glutamicum R
<400> 595
   ggttgttcct tatttctaat caggtgctgt ctgagcaatg ctcggcagcg cgtgatggaa 60
   ttttgtgtgc ggcttggaag tgacgggtca caaggacagc tcgtgtagac cctgcctgga 120
   gccttgacaa actccaccaa acaactgcga cgtgtgtcag attactgcag gcttgtggtc 180
   aaacctagtt ctttggggcg gagcatcata ccttttaatg tcaggatcgt gcagtgaaga 240
   attcaggatg aattactcgc tggaatattg gtggggatag agttgttgtt 290

## Description

### Technical Field

The present invention relates to a method of inductively-expressing the promoter function which functions in an aerobic Coryneform bacterium, and a DNA sequence having the promoter function. More particularly, the present invention relates to a method of inductively-enhancing expression of the function of a gene promoter which functions in a Coryneform bacterium, for the purpose of producing a useful substance such as various organic acids and ethanol at a high efficiency, and a DNA sequence having the promoter function.

### Background Art

A Coryneform bacterium is an industrially important aerobic Gram-positive bacterium which has previously been used for producing useful organic compounds such as various amino acids, lactic acid, succinic acid and the like. Particularly, since a Coryneform bacterium has a peculiar metabolism function that a metabolism pathway for producing a substance is not deteriorated even under a condition that cell division is suppressed by a method of restricting oxygen supply or the like, a nutrient source such as saccharides and the like which is given to a Coryneform bacterium is not consumed for proliferation, and is effectively directed to an objective product. Thus, a raw material nutrient source is effectively utilized, and the technique of producing an objective substance can be easily controlled because of suppression of cell division, and this is why a Coryneform bacterium is industrially paid attention.
In order to highly exert such characteristic function of a Coryneform bacterium, it becomes necessary to effectively and highly express various protein genes necessary for producing an objective product. For doing this, a technique which can enhance the promoter function associated with these protein genes becomes important.
As to a DNA fragment having the promoter function in a Coryneform bacterium, some DNA fragments are known.

For example, a DNA fragment having a stronger promoter function than the tac promoter derived from Escherichia coli is found out on a chromosome of a Coryneform bacterium, and a DNA sequence thereof is known. And, as a method of controlling expression of the promoter function, a method of changing a carbon source composition of saccharides, ethanol and the like which are added to media has been proposed (see Patent Literature 1).
In addition, a promoter DNA sequence associated with a specified enzyme protein (aspartase) gene expressed in a Coryneform bacterium has been found out (see Patent Literature 2). However, as a method of expressing the promoter function, there is only stated that "when incorporated into a plasmid vector together with a gene encoding a protein, and is introduced into a host Coryneform bacterium, an action of potentiating an expression intensity of the gene is possessed", and nothing is referred to a method of enhancing, and a method of controlling expression of the promoter function.

In addition, a promoter or promoters of exogenous and endogenous genes involved in production of L-glutamic acid and L-lysine which functions in a Coryneform bacterium is found out (see Patent Literature 3), but nothing is referred to a method of enhancing expression of those functions.

A technique of using a Coryneform bacterium in which the function of a promoter of a dapA gene (dihydrodipicolinic acid synthase gene) has been enhanced by a mutagenesis method, in production of L-lysine has been proposed (see Patent Literature 4). However, nothing is referred to the function enhancement under an anaerobic condition.

Regarding a method of inductively-expressing the promoter function, a recombinant DNA sequence containing a pfl (pyruvate formate lyase gene) promoter which is induced by pyruvic acid and suppressed by oxygen (Patent Literature 5), and a promoter responsive to a stress such as an oxidative stress (addition of peroxidated lipid), an osmotic stress and a glucose starvation stress of a 2-deoxyglucose-6-phosphate dephosphorylase gene of yeast Saccharomyces cerevisiae (Patent Literature 6) are also known. Patent Literature 6 refers to chemical inducing methods such as a phosphoric acid-deficient inducing method, a copper addition inducing method and the like, a heat shock inducing method, and the like as methods of inducing various gene promoters in addition to the aforementioned ones.

As described above, DNA sequences of various promoters, and methods of inductively-expressing the promoter function with various drugs or stresses are known, but a method of controlling the promoter function which functions in a Coryneform bacterium, which is inductively-enhanced in a reaction medium under an anaerobic condition, and a DNA fragment having the promoter function of the present invention are not known.
Patent Literature 1: Japanese Patent Application Laid-Open (JP-A) No. 7-95891
Patent Literature 2: JP-A No. 7-31478
Patent Literature 3: International Publication WO No. 95/23224
Patent Literature 4: JP-A No. 2001-61485
Patent Literature 5: JP-A No. 3-80088
Patent Literature 6: JP-A No. 2000-78977

### Disclosure of the Invention

### Problems to be Solved by the Invention

An aerobic Coryneform bacterium (including a recombinant) has previously been used in producing a useful organic compound under the aerobic condition (various amino acids) or the anaerobic condition (lactic acid, succinic acid, ethanol or the like).

The present disclosure relates to a method of inductively-expressing the function of a gene promoter function in a Coryneform bacterium, involved in exertion of the function, in order to highly and effectively exert the function of a Coryneform bacterium under an anaerobic condition for producing a useful organic compound under the anaerobic condition, more particularly, provides a method of enhancing the promoter function associated with gene for the purpose of effectively and highly expressing various protein genes necessary for producing an objective substance. Also, the present invention is to provide a DNA fragment having the promoter function which enhances those functions.

By using the technique of the present invention, it becomes possible to effectively perform production of a useful substance under an anaerobic condition.

### Means for Solving the Problems

The present inventors thought that, in order to highly exert a substance production function of a Coryneform bacterium under an anaerobic condition, a technique of expressing and inducing a gene promoter function in a Coryneform bacterium associated therewith is important, and intensively studied, which resulted in the present invention.

Gene promoters are roughly classified into a constitutive promoter and an inducible promoter and, when a useful substance is produced under an anaerobic condition, since finding a technique of controlling expression of the promoter function which is induced under the anaerobic condition rather than enhancement of the function of a constitutive promoter can effectively express a target gene, a technique of producing a substance highly effectively is obtained.

That is, by inductively-enhancing expression of the function of a protein gene promoter necessary for producing an objective substance, -a metabolism pathway which is specialized (concentrated) in an objective production substance is generated in a Coryneform bacterium. Specifically, productivity of an objective substance is improved.

The present inventors found out that an extent of expression of various gene promoters can be quantitatively known, for example, by measuring an amount of a produced mRNA using a DNA chip, and a DNA fragment having the promoter function of the present invention can be obtained by comparing a production amount under an aerobic condition and a production amount under an anaerobic condition. The present inventors further studied, resulting in completion of the present invention.

That is, the present invention relates to:
(1) a DNA fragment comprising the nucleotide sequence of SEQ ID NO: 32 of the Sequence Listing, which has a promoter site and which inductively-enhances expression of a protein involved in production of a useful substance in an aerobic Coryneform bacterium under an anaerobic condition.
(2) the DNA fragment according to (1), wherein the enhancement of expression means that the expression amount of an mRNA under an anaerobic condition is increased by at least 50% relative to an expression amount of the mRNA under an aerobic condition.
(3) the DNA fragment according to (1) or (2), wherein the protein involved in production of a useful substance is an enzyme involved in metabolism in a Coryneform bacterium.
(4) the DNA fragment according to (3), wherein the enzyme is at least one enzyme or coenzyme involved in a glycolysis pathway, a reductive tricarboxylic acid pathway, an anaplerotic pathway, an amino acid synthesis pathway, a purine synthesis pathway, a pyrimidine synthesis pathway, a cholesterol synthesis pathway, a fatty acid synthesis pathway, and a pathway derived from these pathways.
(5) the DNA fragment according to (4), wherein the useful substance is an organic acid, an amino acid, an alcohol, a steroid, a nucleic acid, a fatty acid or a physiologically active substance.
(6) the DNA fragment according to (5), wherein the organic acid is at least one organic acid selected from pyruvic acid, citric acid, isocitric acid, aconitic acid, 2-oxoglutaric acid, succinic acid, fumaric acid, malic acid, oxaloacetic acid, itaconic acid, lactic acid, acetic acid, gluconic acid, 2-ketogluconic acid, 5-ketogluconic acid, D-araboascorbic acid, kojic acid, tetradecane-1,14-dicarboxylic acid, cuminic acid and inosinic acid.
(7) the DNA fragment according to (5), wherein the amino acid is at least one amino acid selected from aspartic acid, threonine, glutamic acid, proline, glycine, alanine, cysteine, valine, isoleucine, leucine, tyrosine, phenylalanine, histidine, lysine, arginine, serine, asparagine, glutamine, hydroxylysine, cystine, methionine, tryptophan, β-alanine, γ-aminobutyric acid (GABA), homocysteine, ornithine, 5-hydroxytryptophan, 3,4-dihydroxyphenylalanine (dopa), triiodotyronine, 4-hydroxyproline and thyroxine.
(8) the DNA fragment according to (5), wherein the alcohol is at least one alcohol selected from methanol, ethanol and butanol.
(9) a method of inducing the promoter function of the DNA fragment having the promoter function as defined in claim 1, comprising culturing an aerobic Coryneform bacterium at an oxidation-reduction potential of a reaction medium of -200 millivolts to -500 millivolts under an anaerobic condition.

Since the DNA fragment having the promoter function of the present invention can highly express a target gene necessary for producing a useful substance at a high efficiency under an anaerobic condition, an objective useful substance can be produced at a high efficiency. That is, by enhancing expression of the function of various protein gene promoters necessary for producing an objective useful substance, a metabolism pathway which is specialized (concentrated) in an objective product is generated in a Coryneform bacterium. Specifically, productivity of an objective substance is improved.
The DNA fragment having the promoter function of the present invention, when introduced into a plasmid or on a chromosome where it can autonomously-replicate in a Coryneform bacterium so that the fragment is situated upstream of a gene encoding a protein (for example, enzyme or the like), which produces an objective useful substance whose expression should be enhanced, so as to function, can generate a transformant of a Coryneform bacterium which can produce an objective useful substance highly and effectively under an anaerobic condition.
A Coryneform bacterium transformed using the DNA fragment having the promoter function of the present invention produces highly and at a high efficiency useful substances such as an organic acid such as lactic acid and succinic acid, an alcohol and an amino acid. A purified useful substance can be used in a broad range of application fields as a raw material for polymer synthesis or a raw material for medicaments, or in cosmetic utility and food additive utility.

### Brief Description of the Drawings

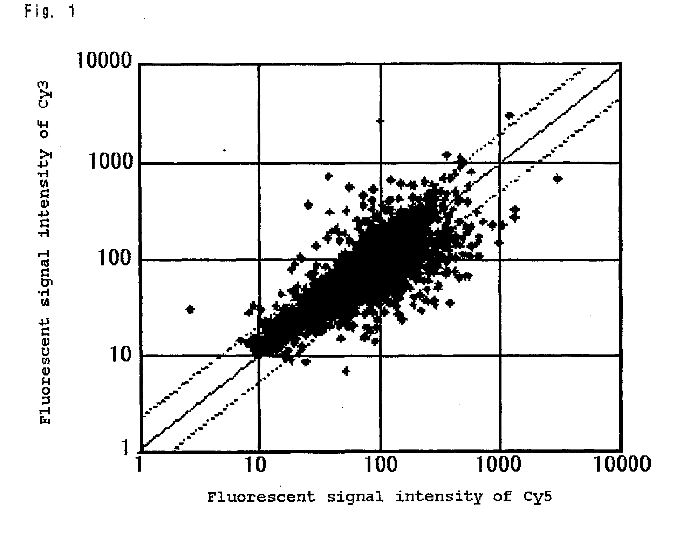

Fig.1 shows correlation of fluorescent signal intensities of Cy3 and Cy5.

### Best Mode for Carrying Out the Invention

In the present invention, the "promoter" refers to a region on a DNA to which an RNA polymerase specifically binds for initiating transcription of a gene. The "DNA fragment having the promoter function" is a DNA fragment obtained from a chromosomal DNA of an aerobic Coryneform bacterium or an artificially synthesized DNA fragment, and the DNA fragment has a function of initiating transcription of a gene, that is, an ability to transcribe a gene, and means a DNA fragment which is presumed to contain the promoter.
Regarding expression of the promoter function, a term "induce" is generally used in many cases when the expression is enhanced, but in the present invention, a term "induce" is used in order to mean that increase or decrease in the expression is induced by intracellular and extracellular factors. And, an extent thereof can be indicated by an expression amount of an mRNA.
Therefore, the "inductively-enhance" in the present invention means that, since a reaction medium is under the specified condition (anaerobic condition), expression of a DNA sequence having the induced promoter function is increased, that is, enhanced, and refers to that an extent of expression of the promoter function indicated by an expression amount of an mRNA is enhanced by at least about 50% or more, preferably about 100% or more relative to an expression amount in a reaction medium under an aerobic condition.

The Coryneform bacterium used in the present invention refers to a group of microorganisms defined in Bargeys Manual of Determinative Bacteriology, vol. 8, p. 599, 1974.

Specifically, examples include Corynebacterium bacteria, Brevibacterium bacteria, Arthrobacter bacteria, Mycobacterium bacteria and Micrococcus bacteria.

More specifically, examples of Corynebacterium bacteria include Corynebacterium glutamicum FERM P-18976, ATCC13032, ATCC13058, ATCC13059, ATCC13060, ATCC13232, ATCC13286, ATCC13287, ATCC13655, ATCC13745, ATCC13746, ATCC13761, ATCC14020 and ATCC31831.

Examples of Brevibacterium bacteria include Brevibacterium lactofermentum ATCC13869, Brevibacterium flavum MJ-233 (FERM BP-1497) and MJ-233AB-41 (FERM BP-1498), and Brevibacterium ammoniagenes ATCC6872.

Examples of Arthrobacter bacteria include Arthrobacter globiformis ATCC8010, ATCC4336, ATCC21056, ATCC31250, ATCC31738 and ATCC35698.

Examples of Micrococcus bacteria include Micrococcus freudenreichii No.239 (FERM P-13221), Micrococcus luteus No.240 (FERM P-13222), Micrococcus ureae IAM1010 and Micrococcus roseus IFO3764.

As the aerobic Coryneform bacterium used in the present invention, Corynebacterium glutamicum R (FERM P-18976), Corynebacterium glutamicum ATCC13032 and the like are particularly preferable.
In addition, the aerobic Coryneform bacterium used in the present invention may be a naturally occurring wild-type variant (for example, FERM P-18977, FERM P-18978 strain and the like), or an artificial strain utilizing biotechnology such as gene recombination (for example, FERM P-17887, FERM P-17888, FERM P-18979 and the like).

In the present invention, a Coryneform bacterium cell under an aerobic condition, which is used in the following procedure, is obtained by proliferating and culturing the aforementioned Coryneform bacterium under an aerobic condition.

Culturing of the Coryneform bacterium can be performed using a normal nutrient medium containing a carbon source, a nitrogen source and an inorganic salt. In culturing, for example, glucose, molasses and the like, as a carbon source, and, for example, ammonia, ammonium sulfate, ammonium chloride, ammonium nitrate and urea as a nitrogen source can be used alone, or by mixing them. In addition, as the inorganic salt, for example, potassium monohydrogen phosphate, potassium dihydrogen phosphate and magnesium sulfate can be used. Besides, if necessary, nutrients such as peptone, meat extract, yeast extract, corn steep liquor, casamino acid, and various vitamins such as biotin and thiamine may be appropriately added to a medium.

A culture can be obtained by culturing a bacterium using a jar fermenter while the air is bubbled, and recovering cells under an aerobic condition with a DO (dissolved oxygen concentration) of not less than 6 ppm. A culturing temperature is about 20°C to 40°C, preferably about 25°C to 35°C. A pH at culturing is in a range of about 5 to 10, preferably about 7 to 8, and a pH during culturing can be adjusted by adding an acid or an alkali. A carbon source concentration at initiation of culturing is about 1 to 20% (W/V), preferably about 2 to 5% (W/V).

Examples of a method of obtaining the Coryneform bacterium cell under an anaerobic condition include a method of washing and recovering bacterium cells which have been aerobically cultured using the jar fermenter or the like. A method of recovering and separating cultured bacterium cells from the thus obtained culture is not particularly limited and, for example, the known methods such as centrifugation and membrane separation can be used. Then, cultured bacterium cells of the Coryneform bacterium which have been recovered and separated from the thus obtained culture are subjected to the condition of production reaction of an organic compound under the reduced state (oxidation-reduction potential of the reaction solution is about -200 millivolts to -500 millivolts), like the method disclosed, for example, in JP-A No.2004-194570, and are separated and recovered. The thus obtained bacterium cells can be used in the present invention as the Coryneform bacterium cell under an anaerobic condition.

As a method of obtaining a DNA fragment having the promoter function, which is inductively-enhanced under an anaerobic condition of the present invention, a method of (a) extracting an mRNA from the Coryneform bacterium cell under an aerobic condition and from the Coryneform bacterium cell under an anaerobic condition, respectively and (b) totally analyzing a change in individual mRNA amounts in a cell using a DNA chip which can handle all genes is most effective.

A DNA fragment having the promoter function which is inductively-enhanced under an anaerobic condition of the present invention is represented by SEQ ID NO.: (32) of the Sequence Listing.

An extent of enhancement or suppression of the promoter function in the DNA fragment having the promoter function under an anaerobic condition of the present invention can be expressed by an expression amount of an mRNA as an index. For example, the "enhancement of expression" refers to that an expression amount of an mRNA in a reaction medium under an anaerobic condition, of the Coryneform bacterium is increased by at least about 50% or more, that is, increased to about 1.5-fold or more relative to an expression amount of an mRNA in a reaction medium under an aerobic condition.

As a method of obtaining a DNA fragment having the promoter function, which is inductively-enhanced under a non-aerobic condition, of the present invention, a method of (a) extracting an mRNA from the Coryneform bacterium cell under an aerobic condition and from the Coryneform bacterium cell under a non-aerobic condition, respectively and (b) totally analyzing a change in individual mRNA amounts in a cell using a DNA chip which can handle all genes, is most effective.

The DNA chip can be manufactured by amplifying an ORF (open reading frame) of each gene by PCR based on gene information obtained from, for example, entire genome analysis of a Coryneform bacterium (see C. glutamicum R strain) (Hiroshi Nonaka, Kaori Nakata, Naoko Okai, Mariko Wada, Yumiko Sato, Kos Peter, Masayuki Inui, Hideaki Yukawa "Corynebacterium glutamicum R Genome Analysis", Japan Agricultural Chemical Society, April 2003, Yokohama, Japan Agricultural Chemical Society 2003 Annual Meeting Lecture Abstract, p. 20), spotting the amplified DNA fragment on an array slide, and performing a fixation treatment, for example, by the Takara Array Slide Standard method.

A method of extracting a total RNA from the Coryneform bacterium cell can be performed, for example, by QIAGEN RNeasy Mini Kit (manufactured by Qiagen), in which, for example, a cell suspension is treated with lysozyme, and glass beads are added, followed by vibration grinding (details are described in Examples). In Addition to the aforementioned kit, commercially available RNA extraction kits, for example, MORA-EXTRACT (manufactured by Cosmo Bio), Total RNA Isolation Mini Kit (manufactured by Agilent), RNA Isolation Kit (manufactured by Stratagene), Isogen (manufactured by Nippon Gene), Trizol (manufactured by Invitrogen), QuickPick mRNA-mini kit (manufactured by BIO NOBILE) and the like can be preferably used, being not limited.

A label of a probe used in the DNA chip can be made by performing synthesis of a cDNA with a random primer using a total RNA as a template, and marker (for example, fluorescent label or radioactive isotope) labeling, which is a conventional method. In the present invention, as the total RNA, a total RNA (Cy5) extracted from the Coryneform bacterium cell under an aerobic condition, or RNA (Cy3) extracted from the Coryneform bacterium cell under an anaerobic condition is used.

Hybridization, washing and drying of the DNA chip are preferably automation-treated with, for example, Amersham Biosciences Lucidea SlidePro or the like, in order to suppress a variation in data as much as possible.

It is suitable to digitalize and normalize detected image data, for example, with Axon Instruments GenePix Pro 5.0 or the like. In an experiment, a value obtained by averaging experimental data from at least three times of experiments is preferably adopted.
A gene corresponding to a sample having the resulting data (Ratio of Meands (Cy3/Cy5); signal intensity under a non-aerobic condition/signal intensity under an aerobic condition) which is about 1.5-fold (about 50% increase) or more is extracted from genome information, and a sequence from 1bp upstream of an initiation codon of each gene to a terminus of an upstream gene of the gene (in the case of the same direction transcription; to 1bp downstream of a termination codon of an upstream gene, in the case of reverse direction transcription gene; to 1bp upstream of an initiation codon of an upstream gene) can be selected as an inducible promoter.

As a protein involved in production of a useful substance, an enzyme related to metabolism in the Coryneform bacterium is preferable. Examples of such enzyme include enzymes involved in a glycolysis pathway, a reductive tricarboxylic acid pathway, an anaplerotic pathway, an amino acid synthesis pathway, a purine synthesis pathway, a pyrimidine synthesis pathway, a cholesterol synthesis pathway or a fatty acid synthesis pathway, or a pathway derived from these pathways, and enzymes involved in a glycolysis pathway, a reductive tricarboxylic acid pathway, an anaplerotic pathway or an amino acid synthesis pathway are more preferable.

Examples of the enzyme involved in a glycolysis pathway are not limited to, but include hexokinase, glucokinase, phosphoglucoseisomerase, phosphofructosekinase, aldolase, triosephosphate isomerase, glycerin aldehyde-3-phosphate dehydrogenase, phosphoglycerate kinase, phosphoglyceromutase, enolase and pyruvate kinase.

Examples of the enzyme involved in a reductive tricarboxylic acid pathway are not limited to, but include pyruvate synthase, citrate synthase, aconitate hydratase, isocitrate dehydrogenase, 2-oxoglutamate dehydrogenase, succinyl CoA synthase, succinate dehydrogenase, fumarate hydratase, malate dehydrogenase, isocitrate lyase and malate synthase.

Examples of the enzyme involved in an anaplerotic pathway are not limited to, but include pyruvate carboxylase, phosphoenolpyruvate carboxylase and phosphoenolpyruvate carboxykinase.

Examples of the enzyme involved in an amino acid synthesis pathway include all enzymes which generate an amino acid, including amino acid synthase, and amino acid synthetase. Specifically, examples are not limited to, but include aspartate aminotransferase, asparaginase, glutamatealanineaminotransferase, phosphoglycerate dehydrokinase, phosphoserineaminotransferase, phosphoserine phosphatase, serine dehydratase, glycinehydroxymethyl transferase, glycine synthase, threonine aldolase, threonine dehydratase, threonine synthase, homoserine kinase, homoserine dehydrogenase, aspartate semialdehyde dehydrogenase, cystine reductase, histidinol dehydrogenase, phenylalanine hydroxylase, glutamine synthetase, ligase, asparagine synthase and tryptophan synthase.

Examples of the enzyme involved in a purine synthesis pathway include enzymes involved in a pentose phosphate cycle (for example, glucose-6-phosphate dehydrogenase, lactonase, 6-phosphogluconate dehydrogenase, ribulonate 3-epimerase, ribosephosphate isomerase and the like), ribosephosphate pyrophosphokinase, amidophosphoribosyl transferase, glycineamidoribotide synthase, glycineamidoribotideformyl transferase, formylglycineamidoribotide synthase, AIR(5-aminoimidazoleribotide)synthetase, 5-aminoimidazole-4-(N-succinocarboxamide)ribotide synthetase, adenylosuccinate lyase, 5-aminoimidazole-4-carboxamideribotideformyl transferase, inosinemonophosphate (IMP) cyclohydrolase, adenylosuccinate synthase, adenylosuccinate lyase, adenylate kinase, IMP dehydrogenase, GMP(guanicine 5'-phosphate)synthetase, and guanylate kinase.

Examples of the enzyme involved in a pyrimidine synthesis pathway include carbamoylphosphate synthase II, aspartate carbamoyl transferase, dihydroorotase, orotate reductase, dihydroorotate dehydrogenase, orotate phosphoribosyl transferase, OMP(orotidinemonophosphate)decarboxylase, cytidine deaminase, uridine phospholitase, deoxyuridine phosphorylase, dihydrouracil dehydrogenase, dihydropyrimidinase and thymidine phosphorylase.
Examples of the enzyme involved in a cholesterol synthesis pathway include 3-hydroxy-3-methylglutaryl CoA reductase, and lanosterol synthase.

Examples of the enzyme involved in a fatty acid synthesis pathway include fatty acid synthase, long chain fatty acid acylation coenzyme A, acetyl CoA carboxylase, and acyltransferase.

Examples of the enzyme involved in a pathway derived from the aforementioned respective pathways are not limited to, but include lactate dehydrogenase which produces lactic acid from pyruvic acid, pyruvate decarboxylase or alcohol dehydrogenase which produces an alcohol from pyruvic acid, and pyruvate oxidase which produces acetic acid from pyruvic acid, and also include malate synthase and isocitrate lyase in a glyoxylate cycle.

It is suitable that the DNA fragment having the promoter function of the present invention is introduced into a plasmid or on a chromosome where it can autonomously-replicate in the Coryneform bacterium, so as to be situated upstream of a gene encoding a protein involved in production of the useful substance. Like this, by arranging the DNA fragment having the promoter function upstream of a gene encoding a protein involved in production of a useful substance, an objective useful substance can be produced highly and effectively under an anaerobic condition.
In addition, in the present invention, in place of a gene encoding a protein involved in production of the useful substance, an expression gene which is not possessed by the Coryneform bacterium, for example, a gene encoding a useful protein produced in a plant may be arranged.

Examples of the useful substance include organic acid, amino acid, alcohol, steroid, nucleic acid, fatty acid and a physiologically active substance.

Examples of the organic acid include pyruvic acid, citric acid, isocitric acid, aconitic acid, 2-oxoglutalic acid, succinic acid, fumaric acid, malic acid, oxaloacetic acid, itaconic acid, lactic acid, acetic acid, gluconic acid, 2-ketogluconic acid, 5-ketogluconic acid, D-araboascorbic acid, kojic acid, tetradecane-1,14-dicarboxylic acid and cuminic acid. In addition, the organic acid also includes purine nucleotide such as inosinic acid, being not limiting.

Examples of the amino acid include aspartic acid, threonine, glutamic acid, proline, glycine, alanine, cysteine, valine, isoleucine, leucine, tyrosine, phenylalanine, histidine, lysine, arginine, serine, asparagine, glutamine, hydroxylysine, cystine, methionine and tryptophan. In addition, in the present invention, examples of the amino acid are not limited to, but include special amino acids such as β-alanine, γ-alanine (GABA), homocysteine, ornithine, 5-hydroxytryptophan, 3,4-dihydroxyphenylalanine (DOPA), triiodotyronine, 4-hydroxyproline, and thyroxine.

As the alcohol, any alcohol is preferable as far as it is an alcohol which is produced by alcohol fermentation, and examples are not limited to, but include methanol, ethanol, butanol and the like.

Examples of the steroid include entities having a perhydrocyclopentanophenanthrene skeleton as a fundamental structure, such as cholesterol, cholic acids (for example, taurocholic acid, glycocholic acid, and the like), sex hormones (for example, progestogen, androgen, follicle steroid and the like) and adrenal cortical hormones (for example, cortisol, corticosterone, aldosterone and the like). Also, plant saponins, digitoxin and the like are included, being not limited.

Examples of the nucleic acid include an RNA and a DNA.

Examples of the fatty acid include palmitic acid, myristic acid and stearic acid. Examples of the fatty acid also include sphingoid, prostaglandin, arachidonic acid, and eicosatetraenoic acid, being not limited.

Examples of the physiologically active substance are not limited to, but include hormones (for example, insulin, growth hormone, ACTH, oxytocin, vasopressin, thyroxine, TRH, LHRH and the like), vitamins (for example, vitamin B₁, vitamin B₂, vitamin B₆, pantothenic acid, folic acid, biotin, vitamin K, and the like), histamine, serotonin and interleukin.
The useful substance of the present invention is not limited to the aforementioned substances, but any substance can be preferably utilized as far as it is a substance which is produced by the Coryneform bacterium of the present invention.

The present invention will be explained in more detail below by way of Examples, but the present invention is not limited to them.

### Example 1

### Obtaining of Coryneform bacterium cell under aerobic condition and under anaerobic condition

### (1) Culturing of Coryneform bacterium, Corynebacterium glutamicum R(FERM P-18976) under aerobic condition:

(Preparation of culture medium); 500 mL of a medium consisting of 2 g of urea, 7 g of ammonium sulfate, 0.5 g of KH₂PO₄, 0.5 g of K₂HPO₄, 0.5 g of MgSO₄·7H₂O, 6 mg of FeSO₄·7H₂O, 4.2 mg of MnSO₄·7H₂O, 200 µg of biotin, 200 µg of thiamine hydrochloride, 2 g of yeast extract, 7 g of casamino acid, and 1000 mL of distilled water was dispensed into a flask of a volume of 1 L, this was heat-sterilized at 120°C for 10 minutes, the flask was cooled to room temperature, and the flask was used as a seed culture medium. Similarly, 1000 mL of a medium having the same composition was placed into a glass jar fermenter of a volume of 2 L, this was heat-sterilized at 120°C for 10 minutes, and this was used as a regular culture medium.

(Culturing): One seed culture medium was inoculated with a Coryneform bacterium, Corynebacterium glutamicum R (FERM P-18976) under the sterile condition, this was aerobically shaking-cultured at 33°C for 12 hours to obtain a seed culturing solution. 50 mL of this seed culturing solution was inoculated on the jar fermenter, and culturing was initiated at a temperature of 33°C at a ventilation amount of 1 vvm (Volume/Volume/Minute). A dissolved oxygen concentration (DO) started at around 7, and DO gradually began to decrease with proliferation, therefore, when a DO value reached 6, the Coryneform bacterium was recovered to obtain a Coryneform bacterium cell under an aerobic condition. On the other hand, aerobic culturing was continued as it was, and the cell was cultured overnight. 200 mL of a culturing solution was subjected to a centrifuge (5000 rotations, 15 min) to remove the supernatant. The thus obtained wet bacterium cell was used in the following reaction.

### (2) Preparation of reaction solution for anaerobic reaction

A reaction stock consisting of 7 g of ammonium sulfate, 0.5 g of KH₂PO₄, 0.5 g of K₂HPO₄, 0.5 g of MgSO₄·7H₂O, 6 mg of FeSO₄·7H₂O, 4.2 mg of MnSO₄·7H₂O, 200 µg of biotin, 200 µg of thiamine hydrochloride, and 1000 mL of distilled water was prepared, and autoclaved at 120°C for 20 minutes. 500 mL of this reaction stock was introduced into a glass reaction vessel having a volume of 1 L. This reaction vessel was provided with a pH adjusting device, a temperature maintaining device, an invessel reaction solution stirring device and a reduction potential measuring device.

### (3) Implementation of reaction:

After the culturing, the prepared Coryneform bacterium cell was suspended in 500 mL of the reaction stock in the reaction vessel. 200 mM glucose was added, a reaction temperature was maintained at 33°C, and an organic compound production reaction was initiated. An oxidation-reduction potential at a reaction was -200 mV at an early stage, but was reduced immediately after initiation of the reaction, and the reaction was continued while maintaining the potential at -400 mV. Four hours after the reaction, the bacterium cell was recovered to obtain a Coryneform bacterium cell under an anaerobic condition. The reaction medium solution thereupon was analyzed using liquid chromatography, and it was found that 186 mM (16.7 g/L)(value after 3 hours) lactic acid was produced.

### Example 2

### Selection of promoter which is inductively-promoted (example) or inductively-suppressed (reference example) under anaerobic condition

### (1) Extraction of total RNA from Coryneform bacterium cells under aerobic condition and under anaerobic condition

Extraction of an RNA was performed with QIAGEN RNeasy Mini Kit (Qiagen). Coryneform bacterium cells under the aerobic condition and under the anaerobic condition recovered in Example 1 were recovered, QIAGEN RNA protect Bacteria Reagent was immediately added at an amount which is 2-fold an amount of the culturing solution, and this was stirred well and incubated at room temperature for 5 minutes to stabilize an RNA. After centrifugation, the supernatant was removed, and the cell was suspended in a RLT Buffer (QIAGEN RNeasy Mini Kit) containing β-mercaptoethanol to a final concentration of 15 to 20 dry cell weight/L. 0.5 mg of 0.1 mm zirconia/silica beads (BioSpec Products, Inc.) and 1 mL of a cell suspension were placed into a 2 mL FastPrep Tube (Qbiogene, Inc., Ca, USA), this was ground at a speed of 6.0 for 45 seconds with FastPrep·FP120 (Qbiogene), and cooled for 1 minute in ice. This procedure was repeated three times to mechanically grind bacterium cells. After centrifugation at 15,000 rpm for 2 minutes, the supernatant was transferred to another vessel, and 99% EtOH at an amount which is 0.56-fold an amount of the supernatant was added, followed by slow mixing. A sample was applied to the RNeasy Mini column, this was centrifuged at 10,000 rpm for 15 seconds, and the waste solution was discarded. 350 µL Buffer RW1 was applied to the column, this was allowed to stand at room temperature for 5 minutes, and centrifuged at 10,000 rpm for 15 seconds for washing, and the waste solution was discarded. Using the RNase-Free DNase Set, the mixed genome DNA was degraded on the column. A DNaseI solution obtained by adding 10 µL of a DNase I stock solution to 70 µL of Buffer RDD was applied to the column, and this was incubated at room temperature for 15 minutes to degrade a DNA. 350 µL Buffer RW1 was applied to the column, this was allowed to stand at room temperature for 5 minutes, and centrifuged at 10,000 rpm for 15 seconds, and the waste solution was discarded. 500 µL Buffer RPE was applied to the column, this was centrifuged at 10,000 rpm for 15 seconds, and the waste solution was discarded. Again, 500 µL Buffer RPE was added to the column, this was centrifuged at 10,000 rpm for 2 minutes, and the waste solution was discarded. In order to completely remove Buffer RPE, this was centrifuged at 15,000 rpm for 1 minute. For elution, the column was transferred to a new 1.5 mL tube, and 60 µL RNase free water was added, followed by centrifugation at 15,000 rpm for 1 minute. In order to obtain an RNA having a higher concentration, the eluate was added to the column once more, followed by centrifugation at 15,000 rpm for 1 minute.

A concentration of an RNA was calculated by measuring an absorbance at O.D.₂₆₀ with a spectrophotometer (O.D.₂₆₀ × 40 _{E}ig/mL). In addition, each sample was thermally degenerated at 95°C for 5 minutes, and subjected to agarose gel electrophoresis to investigate the presence or the absence of mixing of a DNA, and degradation of an RNA. It was confirmed that all samples had a value of O.D.₂₆₀/O.D.₂₈₀ in a range of 1.8 to 2.1.

### (2) Manufacturing of DNA chip which can handle all genes

As a DNA chip, a Stanford manner was adopted. The presence of 3080 genes is presumed from total genome analysis of C. glutamicum R strain (see Hiroshi Nonaka, Kaori Nakata, Naoko Okai, Mariko Wada, Yumiko Sato, Kos Peter, Masayuki Inui, Hideaki Yukawa "Corynebacterium glutamicum R genome Analysis", Nippon Agricultural Chemical Society, April 2003, Yokohama, Nippon Agricultural Chemical Society 2003 Annual Meeting Lecture Abstract, p. 20). Based on the gene (ORF) information, a PCR primer of a pair of a sequence of 20 nucleotides from a third nucleotide of an initiation codon (ATG) to downstream of each gene, and a sequence of 20 nucleotides from a first nucleotide of a termination codon (TAA) to upstream was designed for all genes, and a DNA fragment including an ORF sequence of each gene was amplified by PCR. By electrophoresis on a 1% agarose gel, it was confirmed that the amplification product is a single band, and indicates an objective size. When a plurality of bands were confirmed, an experiment was repeated so that a single band was obtained, by optimizing the PCR condition such as an annealing temperature and the like. The finally obtained DNA sample was spotted on 1 × 3 inch Takara Slide with a spotter, and fixation-treated by the Takara array slide standard method. In order to obtain quantitativity, spotting of 2 points was performed as to each gene.

### (3) DNA chip analysis

Regarding a DNA chip, using a total RNA of Corynebacterium glutamicum R strain as a template, synthesis of a cDNA with a random primer (9mer), and fluorescent labeling (in Cy5, a total RNA extracted from the Coryneform bacterium cell under the aerobic condition was used and, in Cy3, an RNA extracted from the Coryneform bacterium cell under the anaerobic condition was used) were performed to make a labeled probe. In cDNA synthesis and a labeling reaction, Amersham Biosciences CyScribe cDNA Post Labeling Kit (Amersham Biosciences Corp. USA) was used, and this was according to the protocol. 3 µL random nonamer primers were added to 8 µL total RNA (30 µg), and this was heated at 70°C for 5 minutes, and allowed to stand at room temperature for 10 minutes to anneal a primer to an RNA. To this RNA was added a reaction reagent (5x CyScript buffer 4 µL, 0.1 M DTT, 2 µL, CyScribe Post-Labeling nucleotide mix, 1 µL, CyScribe Post-Labeling Amino Allyl-dUTP, 1 µL, 100 U/µL CyCcript reverse transcriptase, 1 µL), and this was incubated at 42°C for 3 hours. After the reaction solution was cooled on ice, in order to alkali-degrade an RNA, 2 µL of 2.5 M NaOH was added, and this was incubated at 37°C for 15 minutes. Then, 10 µL of 2 M HEPES Buffer was added to perform neutralization. Using CyScribe GFX Purification Kit (Amersham Biosciences), an AA-modified cDNA probe was purified. Centrifugation upon a purification procedure was performed at room temperature in all cases. The reaction solution was mixed with 500 µL Capture Buffer, and the mixture was applied to the GFX column, followed by centrifugation at 13,800×g for 30 seconds. The waste solution was discarded, 600 µL 80% EtOH was applied to the column, and this was centrifuged at 13,800×g for 30 seconds, and this procedure was repeated three times. After the column was centrifuged at 13,800×g for 10 seconds, the column was transferred to a new 1.5 mL tube, 60 µL of 0.1 M NaHCO₃ (pH 9.0) was added, and this was allowed to stand at room temperature for 5 minutes. After centrifugation at 13,800×g for 1 minute, in order to recover a solution of a high concentration, the eluate was added to the column again, followed by centrifugation at 13,800×g for 1 minute. The reaction solution after purification was added to a tube containing a Cy3 or Cy5 reactive dye, and the materials were completely dissolved, followed by incubation at room temperature for 3 hours while light was shielded.

15 µL of 4M Hydroxylamine HCl was added, and this was stirred and mixed, followed by incubation at room temperature for 15 minutes while the light was shielded. Using CyScribe GFX Purification Kit (Amersham), a CyDye-labeled cDNA was purified. The reaction solution was mixed with 500 µL of Capture Buffer, and the mixture was applied to the GFX column, followed by centrifugation at 13,800xg for 30 seconds. The waste solution was discarded, 600 µL of Wash Buffer was applied to the column, this was centrifuged at 13,800xg for 30 seconds, and this procedure was repeated three times. After the column was centrifuged at 13,800×g for 10 seconds, the column was transferred to a new 1.5 mL tube, 60 µL of Elution Buffer was added, and this was allowed to stand at room temperature for 5 minutes. After centrifugation at 13,800×g for 1 minute, in order to recover the solution of a high concentration, the eluate was added to the column once more, and this was centrifuged at 13,800×g for 1 minute to recover a purified CyDye-labeled cDNA probe. Cy3 and Cy5-labeled cDNAs were placed into one tube, 100 µL of 2× Hybridization buffer (12× SSC, 0.4% SDS, 10× Denhardt's solution, 0.2 mg/mL denatured salmon sperm DNA) was added, and this was heated at 95°C for 2 minutes, and cooled to room temperature. Steps of hybridization, washing and drying of a microarray were performed using Lucidea Slide Pro (Amersham Biosciences Corp. USA). Hybridization was performed at 60°C for 14 hours. Washing was performed with Wash 1 (2×SSC, 0.2% SDS) for 6 minutes, with Wash 2 (0.2×SSC, 0.2% SDS) for 6 minutes, with Wash 3 (0.2×SSC) two times and with isopropanol once.

### (4) Microarray data analysis

A fluorescent signal of the microarray was detected and imaged with FUJIFILM Fluorescent Image Analyzer FLA-8000 (Fuji, Tokyo, Japan). The detection condition was at 635 nm in Cy5, and at 532 nm in Cy3. The detected image data were digitalized and normalized using Axon Instruments GenePix Pro 5.0 (Axon Instruments, Inc., CA, USA). A color tone (Cy5; green, Cy3; red) was adjusted for every channel, an image was synthesized, and a spot was surrounded with a grid, followed by digitalization. As normalization, global normalization was adopted. Assuming that a sum of expression intensities of all genes is the same between cells to be compared, a fluorescent intensity ratio of all allays was corrected (normalized) so that medians of fluorescent intensity ratios of all signals of Cy5 and Cy3 became equal. For spots which seemed to be defective in reproductivity and quantitativeness (a spot having a half or less size, a spot having a stain or a flaw, a spot of a region where the background was high), values were excluded at an analysis stage. From a value calculated by GenePix Pro, Ratio of Meands (Cy3/Cy5) was used as an expression ratio.

### (5) Selection of inductively-enhancing promoter (example) and inductively-suppressing promoter (reference example) under anaerobic condition

The expression ratio obtained in the (4) was expressed with a scatter plot (see Fig.1). A central oblique line indicates that a value of Cy3/Cy5 is 1, that is, there is neither increase nor decrease in gene expression due to a difference in the culturing condition. On the other hand, two oblique lines situated while holding this have a value of Cy3/Cy5 of 2 or 0.5, indicating 2-fold increase or decrease. This experiment was performed plural times, and it was confirmed that data is obtained with better reproductivity.

A gene corresponding to a sample having this Ratio of Meands (Cy3/Cy5) of 1.5-fold (50% increase) or more or 0.5-fold (50% decrease) or less expression ratio was extracted from genome information, and a sequence from 1bp upstream of an initiation codon of each gene to a terminus of a gene upstream of the gene (in the case of the same direction transcription; to 1bp downstream of a termination codon of an upstream gene, in the case of a reverse direction transcription gene; to 1bp upstream of an initiation codon of an upstream gene) was selected as an inducible promoter. As a result, there were 394 kinds (Table 1) having Ratio of Meands (Cy3/Cy5) of a 1.5-fold (50% increase) expression ratio, and 201 kinds (Table 2) having a 0.5-fold (50% decrease) expression ratio (reference example).

**[Table 1] Inductivcely-enhancing promotor under anaerobic condition and expression ratil**

| No | Cy3/Cy5 | | No | Cy3/Cy5 | | No | Cy3/Cy5 | | No. | Cy3/Cy5 | | No. | Cy3/Cy5 | | No | Cy3/Cy5 |
|---|---|---|---|---|---|---|---|---|---|---|---|---|---|---|---|---|
| 1 | 26.64 | | 67 | 2.41 | | 133 | 1.85 | | 199 | 1.71 | | 265 | 1.62 | | 331 | 1.55 |
| 2 | 19.47 | | 68 | 2.37 | | 134 | 1.85 | | 200 | 1.71 | | 266 | 1.62 | | 332 | 1.54 |
| 3 | 15.27 | | 69 | 2.37 | | 135 | 1.84 | | 201 | 1.71 | | 267 | 1.62 | | 333 | 1.54 |
| 4 | 10.48 | | 70 | 2.28 | | 136 | 1.84 | | 202 | 1.71 | | 268 | 1.62 | | 334 | 1.54 |
| 5 | 10.39 | | 71 | 2.26 | | 137 | 1.84 | | 203 | 1.71 | | 269 | 1.61 | | 335 | 1.54 |
| 6 | 8.04 | | 72 | 2.25 | | 138 | 1.84 | | 204 | 1.70 | | 270 | 1.61 | | 336 | 1.54 |
| 7 | 6.16 | | 73 | 2.24 | | 139 | 1.83 | | 205 | 1.70 | | 271 | 1.61 | | 337 | 1.54 |
| 8 | 6.12 | | 74 | 2.23 | | 140 | 1.83 | | 206 | 1.69 | | 272 | 1.61 | | 338 | 1.54 |
| 9 | 6.00 | | 75 | 2.22 | | 141 | 1.83 | | 207 | 1.69 | | 273 | 1.61 | | 339 | 1.54 |
| 10 | 5.49 | | 76 | 2.20 | | 142 | 1.83 | | 208 | 1.69 | | 274 | 1.61 | | 340 | 1.54 |
| 11 | 5.30 | | 77 | 2.20 | | 143 | 1.82 | | 209 | 1.69 | | 275 | 1.61 | | 341 | 1.54 |
| 12 | 4.87 | | 78 | 2.20 | | 144 | 1.82 | | 210 | 1.69 | | 276 | 1.60 | | 342 | 1.54 |
| 13 | 4.86 | | 79 | 2.19 | | 145 | 1.82 | | 211 | 1.69 | | 277 | 1.60 | | 343 | 1.53 |
| 14 | 4.86 | | 80 | 2.18 | | 146 | 1.82 | | 212 | 1.69 | | 278 | 1.60 | | 344 | 1.53 |
| 15 | 4.84 | | 81 | 2.17 | | 147 | 1.82 | | 213 | 1.69 | | 279 | 1.60 | | 345 | 1.53 |
| 16 | 4.71 | | 82 | 2.17 | | 148 | 1.82 | | 214 | 1.69 | | 280 | 1.60 | | 346 | 1.53 |
| 17 | 4.68 | | 83 | 2.17 | | 149 | 1.82 | | 215 | 1.69 | | 281 | 1.60 | | 347 | 1.53 |
| 18 | 4.60 | | 84 | 2.15 | | 150 | 1.82 | | 216 | 1.68 | | 282 | 1.60 | | 348 | 1.53 |
| 19 | 4.54 | | 85 | 2.15 | | 151 | 1.82 | | 217 | 1.68 | | 283 | 1.60 | | 349 | 1.53 |
| 20 | 4.50 | | 86 | 2.15 | | 152 | 1.81 | | 218 | 1.68 | | 284 | 1.59 | | 350 | 1.53 |
| 21 | 4.42 | | 87 | 2.14 | | 153 | 1.81 | | 219 | 1.68 | | 285 | 1.59 | | 351 | 1.53 |
| 22 | 4.36 | | 88 | 2.14 | | 154 | 1.81 | | 220 | 1.68 | | 286 | 1.59 | | 352 | 1.53 |
| 23 | 4.17 | | 89 | 2.14 | | 155 | 1.80 | | 221 | 1.68 | | 287 | 1.59 | | 353 | 1.53 |
| 24 | 4.09 | | 90 | 2.13 | | 156 | 1.80 | | 222 | 1.68 | | 288 | 1.59 | | 354 | 1.53 |
| 25 | 3.92 | | 91 | 2.12 | | 157 | 1.80 | | 223 | 1.68 | | 289 | 1.59 | | 355 | 1.53 |
| 26 | 3.89 | | 92 | 2.10 | | 158 | 1.80 | | 224 | 1.68 | | 290 | 1.58 | | 356 | 1.53 |
| 27 | 3.76 | | 93 | 2.10 | | 159 | 1.79 | | 225 | 1.67 | | 291 | 1.58 | | 357 | 1.53 |
| 28 | 3.69 | | 94 | 2.10 | | 160 | 1.79 | | 226 | 1.67 | | 292 | 1.58 | | 358 | 1.52 |
| 29 | 3.59 | | 95 | 2.07 | | 161 | 1.78 | | 227 | 1.67 | | 293 | 1.58 | | 359 | 1.52 |
| 30 | 3.52 | | 96 | 2.06 | | 162 | 1.78 | | 228 | 1.67 | | 294 | 1.58 | | 360 | 1.52 |
| 31 | 3.49 | | 97 | 2.05 | | 163 | 1.78 | | 229 | 1.67 | | 295 | 1.58 | | 361 | 1.52 |
| 32 | 3.49 | | 90 | 2.05 | | 164 | 1.77 | | 230 | 1.67 | | 296 | 1.58 | | 362 | 1.52 |
| 33 | 3.37 | | 99 | 2.04 | | 165 | 1.77 | | 231 | 1.66 | | 297 | 1.58 | | 363 | 1.52 |
| 34 | 3.30 | | 100 | 2.03 | | 166 | 1.77 | | 232 | 1.66 | | 298 | 1.58 | | 364 | 1.52 |
| 35 | 3.29 | | 101 | 2.02 | | 167 | 1.77 | | 233 | 1.66 | | 299 | 1.58 | | 365 | 1.52 |
| 36 | 3.21 | | 102 | 2.02 | | 168 | 1.77 | | 234 | 1.66 | | 300 | 1.57 | | 366 | 1.52 |
| 37 | 3.10 | | 103 | 2.01 | | 169 | 1.77 | | 235 | 1.68 | | 301 | 1.57 | | 367 | 1.52 |
| 38 | 3.10 | | 104 | 1.99 | | 170 | 1.77 | | 236 | 1.68 | | 302 | 1.57 | | 368 | 1.52 |
| 39 | 3.08 | | 105 | 1.99 | | 171 | 1.77 | | 237 ' | 1.65 | | 303 | 1.57 | | 369 | 1.52 |
| 40 | 3.00 | | 106 | 1.99 | | 172 | 1.76 | | 238 | 1.65 | | 304 | 1.57 | | 370 | 1.51 |
| 41 | 3.00 | | 107 | 1.97 | | 173 | 1.76 | | 239 | 1.65 | | 305 | 1.57 | | 371 | 1.51 |
| 42 | 3.00 | | 108 | 1.97 | | 174 | 1.76 | | 240 | 1.65 | | 306 | 1.57 | | 372 | 1.51 |
| 43 | 2.98 | | 109 | 1.95 | | 175 | 1.76 | | 241 | 1.65 | | 307 | 1.57 | | 373 | 1.51 |
| 44 | 2.96 | | 110 | 1.94 | | 176 | 1.76 | | 242 | 1.65 | | 308 | 1.57 | | 374 | 1.51 |
| 45 | 2.95 | | 111 | 1.94 | | 177 | 1.76 | | 243 | 1.65 | | 309 | 1.57 | | 375 | 1.51 |
| 46 | 2.95 | | 112 | 1.93 | | 178 | 1.76 | | 244 | 1.65 | | 310 | 1.56 | | 376 | 1.51 |
| 47 | 2.93 | | 113 | 1.93 | | 179 | 1.75 | | 245 | 1.65 | | 311 | 1.56 | | 377 | 1.51 |
| 48 | 2.91 | | 114 | 1.92 | | 180 | 1.75 | | 246 | 1.65 | | 312 | 1.56 | | 378 | 1.51 |
| 49 | 2.88 | | 115 | 1.92 | | 181 | 1.75 | | 247 | 1.65 | | 313 | 1.56 | | 379 | 1.51 |
| 50 | 2.77 | | 116 | 1.92 | | 182 | 1.74 | | 248 | 1.65 | | 314 | 1.56 | | 380 | 1.51 |
| 51 | 2.74 | | 117 | 1.92 | | 183 | 1.74 | | 249 | 1.65 | | 315 | 1.56 | | 381 | 1.50 |
| 52 | 2.66 | | 118 | 1.91 | | 184 | 1.74 | | 250 | 1.65 | | 316 | 1.56 | | 382 | 1.50 |
| 53 | 2.63 | | 119 | 1.91 | | 185 | 1.73 | | 251 | 1.65 | | 317 | 1.56 | | 383 | 1.50 |
| 54 | 2.62 | | 120 | 1.89 | | 186 | 1.73 | | 252 | 1.65 | | 318 | 1.56 | | 384 | 1.50 |
| 55 | 2.57 | | 121 | 1.89 | | 187 | 1.73 | | 253 | 1.64 | | 319 | 1.55 | | 385 | 1.50 |
| 56 | 2.56 | | 122 | 1.89 | | 188 | 1.73 | | 254 | 1.64 | | 320 | 1.55 | | 386 | 1.50 |
| 57 | 2.56 | | 123 | 1.88 | | 189 | 1.73 | | 255 | 1.64 | | 321 | 1.55 | | 387 | 1.50 |
| 58 | 2.55 | | 124 | 1.88 | | 190 | 1.73 | | 256 | 1.64 | | 322 | 1.55 | | 388 | 1.50 |
| 59 | 2.54 | | 125 | 1.88 | | 191 | 1.72 | | 257 | 1.64 | | 323 | 1.55 | | 389 | 1.50 |
| 60 | 2.53 | | 126 | 1.88 | | 192 | 1.72 | | 258 | 1.64 | | 324 | 1.55 | | 390 | 1.50 |
| 61 | 2.51 | | 127 | 1.87 | | 183 | 1.72 | | 259 | 1.64 | | 325 | 1.55 | | 391 | 1.50 |
| 62 | 2.51 | | 128 | 1.87 | | 194 | 1.72 | | 260 | 1.63 | | 326 | 1.55 | | 392 | 1.50 |
| 63 | 2.46 | | 129 | 1.87 | | 195 | 1.72 | | 261 | 1.63 | | 327 | 1.55 | | 393 | 1.50 |
| 64 | 2.45 | | 130 | 1.87 | | 196 | 1.72 | | 262 | 1.63 | | 328 | 1.55 | | 394 | 1.50 |
| 65 | 2.44 | | 131 | 1.86 | | 197 | 1.71 | | 263 | 1.63 | | 329 | 1.55 | | | |
| 66 | 2.43 | | 132 | 1.85 | | 198 | 1.71 | | 264 | 1.62 | | 330 | 1.55 | | | |

**[Table 2] Expression-suppressing promotor under anaerobic condition and expression ration**

| No | Cy3/Cy5 | | No | Cy3/Cy5 | | No | Cy3/Cy5 | | No | Cy3/Cy5 |
|---|---|---|---|---|---|---|---|---|---|---|
| 395 | 0.10 | | 461 | 0.31 | | 527 | 0.42 | | 593 | 0.50 |
| 396 | 0.11 | | 462 | 0.31 | | 528 | 0.42 | | 594 | 0.50 |
| 397 | 0.12 | | 463 | 0.32 | | 529 | 0.42 | | 596 | 0.50 |
| 398 | 0.13 | | 404 | 0.32 | | 530 | 0.42 | | | |
| 399 | 0.14 | | 465 | 0.32 | | 531 | 0.42 | | | |
| 400 | 0.14 | | 466 | 0.33 | | 532 | 0.43 | | | |
| 401 | 0.14 | | 467 | 0.33 | | 533 | 0.43 | | | |
| 402 | 0.14 | | 468 | 0.33 | | 534 | 0.43 | | | |
| 403 | 0.14 | | 469 | 0.33 | | 535 | 0.43 | | | |
| 404 | 0.16 | | 470 | 0.33 | | 536 | 0.43 | | | |
| 405 | 0.16 | | 471 | 0.33 | | 537 | 0.43 | | | |
| 406 | 0.16 | | 472 | 0.33 | | 538 | 0.43 | | | |
| 407 | 0.17 | | 473 | 0.33 | | 539 | 0.43 | | | |
| 408 | 0.17 | | 474 | 0.33 | | 540 | 0.43 | | | |
| 409 | 0.17 | | 475 | 0.33 | | 541 | 0.43 | | | |
| 410 | 0.17 | | 476 | 0.33 | | 542 | 0.44 | | | |
| 411 | 0.18 | | 477 | 0.33 | | 543 | 0.44 | | | |
| 412 | 0.19 | | 478 | 0.34 | | 544 | 0.44 | | | |
| 413 | 0.19 | | 479 | 0.34 | | 545 | 0.44 | | | |
| 414 | 0.20 | | 480 | 0.34 | | 546 | 0.44 | | | |
| 415 | 0.20 | | 481 | 0.34 | | 547 | 0.44 | | | |
| 416 | 0.21 | | 482 | 0.34 | | 548 | 0.44 | | | |
| 417 | 0.21 | | 483 | 0.34 | | 549 | 0.44 | | | |
| 418 | 0.21 | | 484 | 0.34 | | 550 | 0.44 | | | |
| 419 | 0.21 | | 485 | 0.34 | | 551 | 0.44 | | | |
| 4.20 | 0.22 | | 486 | 0.35 | | 552 | 0.44 | | | |
| 4.21 | 0.22 | | 487 | 0.35 | | 553 | 0.45 | | | |
| 4.22 | 0.22 | | 488 | 0.35 | | 554 | 0.45 | | | |
| 423 | 0.22 | | 489 | 0.35 | | 555 | 0.45 | | | |
| 424 | 0.22 | | 490 | 0.35 | | 556 | 0.45 | | | |
| 425 | 0.22 | | 491 | 0.35 | | 557 | 0.46 | | | |
| 426 | 0.23 | | 492 | 0.36 | | 558 | 0.46 | | | |
| 427 | 0.23 | | 493 | 0.36 | | 559 | 0.46 | | | |
| 428 | 0.24 | | 494 | 0.36 | | 560 | 0.46 | | | |
| 429 | 0.24 | | 495 | 0.37 | | 561 | 0.48 | | | |
| 430 | 0.24 | | 496 | 0.37 | | 562 | 0.48 | | | |
| 431 | 0.24 | | 497 | 0.37 | | 563 | 0.46 | | | |
| 432 | 0.24 | | 498 | 0.37 | | 564 | 0.46 | | | |
| 433 | 0.24 | | 499 | 0.37 | | 565 | 0.46 | | | |
| 434 | 0.25 | | 500 | 0.37 | | 566 | 0.47 | | | |
| 435 | 0.26 | | 501 | 0.37 | | 567 | 0.47 | | | |
| 436 | 0.26 | | 502 | 0.37 | | 568 | 0.47 | | | |
| 437 | 0.26 | | 503 | 0.38 | | 569 | 0.47 | | | |
| 438 | 0.27 | | 504 | 0.38 | | 570 | 0.47 | | | |
| 439 | 0.27 | | 505 | 0.38 | | 571 | 0.47 | | | |
| 440 | 0.27 | | 506 | 0.38 | | 572 | 0.47 | | | |
| 441 | 0.27 | | 507 | 0.38 | | 573 | 0.47 | | | |
| 442 | 0.27 | | 508 | 0.39 | | 574 | 0.48 | | | |
| 443 | 0.28 | | 509 | 0.39 | | 575 | 0.48 | | | |
| 444 | 0.28 | | 510 | 0.39 | | 576 | 0.48 | | | |
| 445 | 0.28 | | 511 | 0.40 | | 577 | 0.48 | | | |
| 446 | 0.28 | | 512 | 0.40 | | 578 | 0.48 | | | |
| 447 | 0.20 | | 513 | 0.40 | | 579 | 0.48 | | | |
| 448 | 0.28 | | 514 | 0.40 | | 580 | 0.48 | | | |
| 449 | 0.28 | | 515 | 0.40 | | 581 | 0.48 | | | |
| 450 | 0.29 | | 516 | 0.40 | | 582 | 0.48 | | | |
| 451 | 0.30 | | 517 | 0.40 | | 583 | 0.49 | | | |
| 452 | 0.30 | | 518 | 0.41 | | 584 | 0.49 | | | |
| 453 | 0.30 | | 519 | 0.41 | | 585 | 0.49 | | | |
| 454 | 0.30 | | 520 | 0.41 | | 586 | 0.49 | | | |
| 455 | 0.31 | | 521 | 0.41 | | 587 | 0.49 | | | |
| 458 | 0.31 | | 522 | 0.41 | | 588 | 0.49 | | | |
| 457 | 0.31 | | 523 | 0.41 | | 589 | 0.49 | | | |
| 458 | 0.31 | | 524 | 0.42 | | 590 | 0.50 | | | |
| 459 | 0.31 | | 525 | 0.42 | | 591 | 0.50 | | | |
| 460 | 0.31 | | 526 | 0.42 | | 592 | 0.50 | | | |

| | | | | | | | | | | |
|---|---|---|---|---|---|---|---|---|---|---|
| No; SEQ ID NO. Cy3/Cy5; Expression ratio | | | | | | | | | | |

### Example 3 (reference example)

### Conformation of inductive enhancement and inductive suppression by real time quantitative RT-PCR analysis

In order to study enhancement or suppression data of an inducible promoter obtained by DNA chip analysis, an expression ratio thereof was analyzed by real time quantitative RT-PCR. As a sample to be analyzed, among 394 kinds of inductively-enhancing promoters which showed a 1.5-fold or more expression ratio, three kinds of SEQ ID NOS. 3, 22 and 47 (NOS of Table 1) were randomly selected and, on the other hand, among 201 kinds of inductively-suppressing promoters which showed a 0.5-fold or less expression ratio, three kinds of SEQ ID NOS. 410, 474 and 502 (NOS of Table 2) were randomly selected.

Real time quantitative RT-PCR analysis was performed with QIAGEN QuantiTect SYBR Green RT-PCR Kit (Qiagen) using a total RNA as a template. A gene-specific primer was designed using Applied Biosystems Primer Express Software v2.0 (Applied Biosystems, USA). A real time PCR experiment was performed with ABI PRISM 7000 Sequence Detection System (Applied Biosystems, USA), and a PCR reaction was performed using 96-Well Optical Reaction Plate (Applied Biosystems) and Optical Adhesive Covers (Applied Biosystems). A composition of a PCR reaction solution was adjusted with (50 µL/1 sample); Total RNA, 60 ng, 2x QuantiTect SYBR Green RT-PCR Master Mix, 25 µL, Primer Forward, 0.5 µM, Primer Reverse, 0.5 µM, QuantiTect RT Mix, 0.5 µL. The PCR reaction was performed at 50°C 30 min, 95°C 15 min, (95°C 15 sec, 57°C 20 sec, 60°C 1 min) x 40 cycles. After completion of the PCR reaction, for calculating an expression amount, a comparative C_{T} method of quantitating an expression amount ratio for every specimen, and comparing specimens by the expression ratio was used. At a center of a place where all samples were exponentially proliferating (a place where samples were proliferating at a straight proliferation curve), a Threshold line was set, and the number of cycles intersecting with this line was adopted as a C_{T} value, and a ratio of an expression amount was calculated.

As a result, expression ratios of inductively-enhancing promoters SEQ ID NOS. 3, 22 and 47 (NOS in Table 1) (results are in Table 3 which showed a 1.5-fold or more expression ratio, and inductively-suppressing promoters SEQ ID NOS. 410, 474 and 502 (NOS in Table 2 (results are in Table 4) which showed a 0.5-fold or less expression ratio were well in agreement with DNA chip analysis results, respectively.

**[Table 3]**

| Expression ratio of inductively-enhancing promoter by real time quantitative RT-PCR analysis | | |
|---|---|---|
| SEQ ID NO same as in Table 1 | Cy3 / Cy5 DNA chip | real time quantitative RT-PCR |
| 3 | 15.27 | 17.19 |
| 22 | 4.36 | 4.51 |
| 47 | 2.93 | 2.57 |

**[Table 4]**

| Expression ratio of inductively-suppressing promoter by real time quantitative RT-PCR analysis | | |
|---|---|---|
| SEQ ID NO same as in Table 2 | Cy3 / Cy5 DNA chip | real time quantitative RT-PCR |
| 410 | 0.17 | 0.59 |
| 474 | 0.33 | 0.44 |
| 502 | 0.37 | 0.41 |

### Industrial Applicability

The DNA fragment of the present invention is useful as a primer which is introduced into a transformed Coryneform bacterium producing a useful substance such as lactic acid and succinic acid highly and at a high efficiency. A Coryneform bacterium in which the DNA fragment of the present invention has been introduced can be utilized in producing an organic acid, an alcohol, an amino acid or the like. In addition, the produced organic acid can be used as a polymer synthesis raw material or a medicament raw material, or in a wide field such as cosmetic utility and food additive utility. For example, succinic acid and a derivative thereof are useful in a biodegradable plastic raw material or in utility of a green washing solvent which does not bring out environmental pollution.

## Claims

1. A DNA fragment comprising the nucleotide sequence of SEQ ID NO: 32 of the Sequence Listing, which has a promoter site and which inductively-enhances expression of a protein involved in production of a useful substance in an aerobic Coryneform bacterium under an anaerobic condition.

2. The DNA fragment according to claim 1, wherein the enhancement of expression means that the expression amount of an mRNA under an anaerobic condition is increased by at least 50% relative to the expression amount of the mRNA under an aerobic condition.

3. The DNA fragment according to claim 1 or 2, wherein the protein involved in production of a useful substance is an enzyme involved in metabolism in a Coryneform bacterium.

4. The DNA fragment according to claim 3, wherein the enzyme is at least one enzyme or coenzyme involved in a glycolysis pathway, a reductive tricarboxylic acid pathway, an anaplerotic pathway, an amino acid synthesis pathway, a purine synthesis pathway, a pyrimidine synthesis pathway, a cholesterol synthesis pathway, a fatty acid synthesis pathway, and a pathway derived from these pathways.

5. The DNA fragment according to claim 4, wherein the useful substance is an organic acid, an amino acid, an alcohol, a steroid, a nucleic acid, a fatty acid or a physiologically active substance.

6. The DNA fragment according to claim 5, wherein the organic acid is at least one organic acid selected from pyruvic acid, citric acid, isocitric acid, aconitic acid, 2-oxoglutaric acid, succinic acid, fumaric acid, malic acid, oxaloacetic acid, itaconic acid, lactic acid, acetic acid, gluconic acid, 2-ketogluconic acid, 5-ketogluconic acid, D-araboascorbic acid, kojic acid, tetradecane-1,14-dicarboxylic acid, cuminic acid and inosinic acid.

7. The DNA fragment according to claim 5, wherein the amino acid is at least one amino acid selected from aspartic acid, threonine, glutamic acid, proline, glycine, alanine, cysteine, valine, isoleucine, leucine, tyrosine, phenylalanine, histidine, lysine, arginine, serine, asparagine, glutamine, hydroxylysine, cystine, methionine, tryptophan, β-alanine, γ-aminobutyric acid (GABA), homocysteine, ornithine, 5-hydroxytryptophan, 3,4-dihydroxyphenylalanine (dopa), triiodotyronine, 4-hydroxyproline and thyroxine.

8. The DNA fragment according to claim 5, wherein the alcohol is at least one alcohol selected from methanol, ethanol and butanol.

9. A method of inducing the promoter function of the DNA fragment having the promoter function as defined in claim 1, comprising culturing an aerobic Coryneform bacterium at an oxidation-reduction potential of a reaction medium of -200 millivolts to -500 millivolts under an anaerobic condition.

## Patentansprüche

1. DNA-Fragment, umfassend die Nucleotidsequenz von SEQ ID NR.: 32 des Sequenzprotokolls, welche eine Promotorstelle aufweist und welche induktiv die Expression eines Proteins, involviert in die Produktion einer nützlichen Substanz, in einem aeroben coryneformen Bakterium unter einem anaeroben Zustand verstärkt.

2. DNA-Fragment nach Anspruch 1, wobei die Verstärkung der Expression bedeutet, dass die Expressionsmenge einer mRNA unter einem anaeroben Zustand um mindestens 50 % in Bezug auf die Expressionsmenge der mRNA unter einem aeroben Zustand erhöht wird.

3. DNA-Fragment nach Anspruch 1 oder 2, wobei das in die Produktion einer nützlichen Substanz involvierte Protein ein in den Stoffwechsel in einem coryneformen Bakterium involviertes Enzym ist.

4. DNA-Fragment nach Anspruch 3, wobei das Enzym mindestens ein Enzym oder Coenzym ist, das in einen Glycolyseweg, einen reduktiven Tricarbonsäureweg, einen anaplerotischen Stoffwechselweg, einen Aminosäuresyntheseweg, einen Purinsyntheseweg, einen Pyrimidinsyntheseweg, einen Cholesterinsyntheseweg, einen Fettsäuresyntheseweg und einen von diesen Wegen abgeleiteten Weg involviert ist.

5. DNA-Fragment nach Anspruch 4, wobei die nützliche Substanz eine organische Säure, eine Aminosäure, ein Alkohol, ein Steroid, eine Nucleinsäure, eine Fettsäure oder eine physiologisch aktive Substanz ist.

6. DNA-Fragment nach Anspruch 5, wobei die organische Säure mindestens eine organische Säure ist, ausgewählt aus Pyruvinsäure, Zitronensäure, Isozitronensäure, Aconitinsäure, 2-Oxoglutarsäure, Bernsteinsäure, Fumarsäure, Äpfelsäure, Oxalessigsäure, Itaconsäure, Milchsäure, Essigsäure, Gluconsäure, 2-Ketogluconsäure, 5-Ketogluconsäure, D-Araboascorbinsäure, Kojisäure, Tetradecan-1,14-dicarbonsäure, Cuminsäure und Inosinsäure.

7. DNA-Fragment nach Anspruch 5, wobei die Aminosäure mindestens eine Aminosäure ist, ausgewählt aus Asparaginsäure, Threonin, Glutaminsäure, Prolin, Glycin, Alanin, Cystein, Valin, Isoleucin, Leucin, Tyrosin, Phenylalanin, Histidin, Lysin, Arginin, Serin, Asparagin, Glutamin, Hydroxylysin, Zystin, Methionin, Tryptophan, β-Alanin, γ-Aminobuttersäure (GABA), Homozystein, Omithin, 5-Hydroxytryptophan, 3,4-Dihydroxyphenylalanin (dopa), Triiodthyronin, 4-Hydroxyprolin und Thyroxin.

8. DNA-Fragment nach Anspruch 5, wobei der Alkohol mindestens ein Alkohol, ausgewählt aus Methanol, Ethanol und Butanol, ist.

9. Verfahren zum Induzieren der Promotorfunktion des DNA-Fragments mit der Promotorfunktion, wie in Anspruch 1 definiert, umfassend das Kultivieren eines aeroben coryneformen Bakteriums bei einem Oxidations-Reduktions-Potential eines Reaktionsmediums von -200 Millivolt bis -500 Millivolt unter einem anaeroben Zustand.

## Revendications

1. Un fragment d'ADN comprenant la séquence de nucléotides SEQ ID NO : 32 du listage de séquence, qui présente un site de promoteur et qui améliore de manière inductive l'expression d'une protéine intervenant dans la production d'une substance utile dans une bactérie Corinéforme aérobique sous condition anaérobique.

2. Le fragment d'ADN selon la revendication 1, dans lequel l'amélioration de l'expression signifie que la quantité d'expression d'un ARNm sous condition anaérobique est augmentée d'au moins 50 % par rapport à la quantité d'expression de l'ARNm sous condition aérobique.

3. Le fragment d'ADN selon la revendication 1 ou 2, dans lequel la protéine intervenant dans la production d'une substance utile est une enzyme intervenant dans le métabolisme d'une bactérie Corinéforme.

4. Le fragment d'ADN selon la revendication 3, dans lequel l'enzyme est au moins une enzyme ou une coenzyme intervenant dans une voie de glycolyse, une voie d'acide tricarboxylique réductrice, une voie anaplérotique, une voie de synthèse d'un acide aminé, une voie de synthèse d'une purine, une voie de synthèse d'une pyrimidine, une voie de synthèse d'un cholestérol, une voie de synthèse d'un acide gras, et une voie dérivée desdites voies.

5. Le fragment d'ADN selon la revendication 4, dans lequel la substance utile est un acide organique, un acide aminé, un alcool, un stéroïde, un acide nucléique, un acide gras ou une substance active physiologique.

6. Le fragment d'ADN selon la revendication 5, dans lequel l'acide organique est au moins un acide organique choisi parmi l'acide pyruvique, l'acide citrique, l'acide isocitrique, l'acide aconitique, l'acide 2-oxoglutarique, l'acide succinique, l'acide fumarique, l'acide malique, l'acide oxaloacétique, l'acide itaconique, l'acide lactique, l'acide acétique, l'acide gluconique, l'acide cétogluconique, l'acide 5-cétogluconique, l'acide D-araboascorbique, l'acide kojique, l'acide tétradécane-1,14-dicarboxylique, l'acide cuminique et l'acide inosinique.

7. Le fragment d'ADN selon la revendication 5, dans lequel l'acide aminé est au moins un acide aminé choisi parmi l'acide aspartique, la thréonine, l'acide glutamique, la proline, la glycine, l'alanine, la cystéine, la valine, l'isoleucine, la leucine, la tyrosine, la phénylalanine, l'histidine, la lysine, l'arginine, la sérine, l'asparagine, la glutamine, l'hydroxylysine, la cystine, la méthionine, le tryptophane, la β-alanine, l'acide γ-aminobutyrique (GABA), l'homocystéine, l'ornithine, le 5-hydroxytryptophane, le 3,4-dihydroxyphénylalanine (dopa), la triiodotyronine, la 4-hydroxyproline et la thyroxine.

8. Le fragment d'ADN selon la revendication 5, dans lequel l'alcool est au moins un alcool choisi parmi le méthanol, l'éthanol et le butanol.

9. Une méthode d'induction de la fonction promoteur d'un fragment d'ADN présentant la fonction promoteur telle que définie à la revendication 1, comprenant une culture d'une bactérie Corinéforme aérobique à un potentiel d'oxydoréduction d'un milieu réactionnel de -200 millivolts à -500 millivolts sous condition anaérobique.
